# EUROPEAN PATENT APPLICATION

(11) **EP 3 214 442 A1**
(43) Date of publication of application: **06.09.2017**
(21) Application number: 17158409.7
(22) Date of filing: 25.10.2011
(51) Int. Cl.: G01N 33/53, C07K 16/28, A01K 67/027, A61K 38/20, A61P 37/02

(54) **TREATMENT OF GASTROINTESTINAL INFLAMMATION AND PSORIASIS AND ASTHMAINFLAMMATION AND PSORIASIS A**

(30) Priority: 25.10.2010 US 455780 P; 03.10.2011 US 201161626838 P; 12.10.2011 US 201161627493 P
(62) Divisional of application: 11778773.9
(71) Applicant: F. Hoffmann-La Roche AG, 4070 Basel (CH)
(72) Inventor: GONZALES, Lino, South San Francisco, CA 94080 (US); OUYANG, Wenjun, South San Francisco, CA 94080 (US); PAPPU, Rajita, South San Francisco, CA 94080 (US); RAMIREZ-CARROZI, Vladimir, South San Francisco, CA 94080 (US)
(74) Representative: Heiroth, Ulrike Hildegard

(57) **Abstract**

The present invention provides methods and means to reduce gastrointestinal inflammation. In particular, the invention provides methods and means to treat inflammatory bowel disease (IBD) and related conditions. The invention further provides methods and means to treat psoriasis. The invention further provides methods and means to treat asthma.

## Description

### Cross-reference to Related Applications

This application claims priority under Section 119(e) and the benefit of United States Provisional Application Serial Nos. 61/455,780 filed October 25, 2010, 61/626,838 filed October 3, 2011, and 61/627,493 (Attorney Docket No. GNE-0364-2PR) filed October 12, 2011, each of which the entire disclosures are incorporated herein by reference in their entirety.

### Reference to a Sequence Listing

The instant application contains a Sequence Listing which has been submitted in ASCII format via EFS-Web and is hereby incorporated by reference in its entirety. Said ASCII copy, created on October 17, 2011, is named GNE364US.txt and is 21,655 bytes in size.

### FIELD OF THE INVENTION

The present invention concerns the treatment of diseases associated with gastrointestinal inflammation, such as inflammatory bowel disease, and/or psoriasis and/or asthma. In particular, the invention concerns the treatment of gastrointestinal inflammation and/or psoriasis and/or asthma by administration of an antagonist of the IL-17RA and/or IL-17RE receptors, such as anti-IL-17RA and/or IL-17RE antibodies, or antibody fragments.

### Background of the Invention

Mammalian cutaneous and mucosal epithelial cells constitute the first line of defense against invading pathogens. The crosstalk between the immune system and tissue epithelia is essential for host defense against infections and for the development of autoimmunity¹⁻³ During microbial invasion, factors derived from both leukocytes and epithelial cells orchestrate different defense mechanisms that best control the pathogens. Some cytokines such as interleukin 6 (IL-6) and IL-1 can be produced by both leukocytes and epithelial cells and elicit inflammatory responses ubiquitously from variety of cell types^{4,5}. T cells, especially T helper subsets, play essential regulatory functions on the types of host defense mechanisms elicited by epithelial cells during infection⁶. For example, while interferon γ (IFN-γ) produced by T_{H}1 enhances host defense against intracellular pathogens, IL-4, IL-13 from T_{H}2 cells participate in clearance of parasite infection. Recently, T_{H}17 cells have been identified to exert essential functions in host defense against extracellular bacterial and yeast infections. Th17 cells preferentially produce IL-17A, IL-17F and IL-22, all of which boost the innate defense mechanisms from tissue epithelial cells and fibroblasts^{7,8}.

IL-17A and IL-17F belong to the IL-17 family of cytokines, which also includes IL-17B, IL-17C, IL-17D and IL-17E/IL-25^{9,10}. Prominent features of this family include a highly conserved C-terminus, and five spatially conserved cysteine residues that mediate dimerization¹¹. These cytokines bind to heterodimeric complexes composed of members of the IL-17 receptor family, IL-17RA-IL-17RE, to elicit biological effects^{9,10,12}. IL-17A and IL-17F can form both homodimers and heterodimers, all of which signal through a receptor complex composed of IL-17RA and IL-17RC¹²⁻¹⁴. IL-17E utilizes a different receptor complex, composed of the IL-17RA and IL-17RB subunits to promote Th2 immune responses^{11,12,15,16,17}. IL-17RB is also identified as a receptor chain for IL-17B¹⁸. Currently, the receptors for IL-17C and IL-17D are still unclear.

The best-studied IL-17 family cytokine is IL-17A. Upon binding to its receptor, IL-17A induces the expression of pro-inflammatory chemokines and cytokines, anti-microbial peptides and proteins involved in tissue remodeling and acute phase responses from epithelial cells, fibroblasts, endothelial cells, chondrocytes and adipocytes, which preferentially express IL-17RA and IL-17RC^{9,10}. Additionally, IL-17A displays synergism with other cytokines such as TNF-α, IL-1β and IFN-γ to augment the induction of proinflammatory responses from various target cells. IL-17A is critical for host anti-microbial responses¹⁹. The absence of IL-17A renders susceptibility to fungal and bacterial infections and exacerbates disease in Dextran sulfate sodium (DSS)-induced colitis, in which inflammation is driven by the perturbation of mucosal homeostasis, permitting interaction between commensal bacteria and the colon tissue²⁰⁻²⁴. In addition to these protective functions, IL-17A can also display pathogenic properties, leading to uncontrolled inflammation. Increased IL-17A expression is observed in many human autoimmune diseases including psoriasis, Rheumatoid Arthritis (RA), Multiple Sclerosis (MS) and Inflammatory Bowel Disease (IBD)^{2,25-28}. Studies in pre-clinical animal models and in human clinical trials have demonstrated that inhibition of IL-17A pathway can ameliorate disease activity²⁹⁻³².

IL-17C is also upregulated during inflammation, and is detected in lung and skin tissues following *M. pneumoniae* and *S. aureus* infections respectively, and in psoriatic skin lesions^{26, 33-35}. Although IL-17C has been reported to induce pro-inflammatory cytokine secretion in cellular assays, and ectopic expression *in vivo* causes inflammation, the biological function of IL-17C is still largely unexplored³⁶⁻³⁸. IL-17C expression appears to be tightly regulated, with mRNA and protein only detected during inflammatory states, such as following bacterial infection of mucosal tissues or in lesional psoriatic skin^{26,33,35,37,45}. Functionally, ectopic expression of IL-17C *in vivo* promotes pro-inflammatory pathways, however the target cells, receptors utilized and molecular events following ligand binding are uncharacterized^{36, 38}.

### Summary of the Invention

The present invention is based, at least in part, on experimental data demonstrating that (1) IL-17C uses both IL-17RA and IL-17RE as receptors, (2) IL-17C induces host defense pathways in epithelial cells, and 3) IL-17C is secreted by epithelial cells in response to bacterial or cytokine stimuli.

The present invention provides methods and means to reduce gastrointestinal inflammation. In particular, the invention provides methods and means to treat inflammatory bowel disease (IBD) and related conditions. The invention further provides methods and means to treat psoriasis.

In one aspect, the invention concerns a method of reducing gastrointestinal inflammation, comprising administering to a subject in need at least one antagonist of the IL-17RA and IL-17RE receptors.

Preferably, the gastrointestinal inflammation is associated with inflammatory bowel disease (IBD).

In one embodiment, the antagonist signals through both the IL-17RA and the IL-17RE receptors.

In another embodiment, the antagonist binds to both the IL-17RA and the IL-17RE receptors.

In yet another embodiment, the treatment comprises administration of a combination of a first antagonist of the IL-17RA receptor and a second antagonist of the IL-17RE receptor.

In a further embodiment, the first antagonist signals through the IL-17RA receptor and the second antagonist signals through the IL-17RE receptor.

In a still further embodiment, the first antagonist binds to the IL-17RA receptor and the second antagonist binds to the IL-17RE receptor.

In all embodiments, the antagonist can, for example, be an antibody or an antigen-binding fragment thereof.

In all embodiments, the antagonist can, for example, be a bispecific or bivalent antibody signaling through or binding to both the IL-17RA and IL-17RE receptors, or an antigen-binding fragment thereof.

In all embodiments, the method may further comprise the administration of a further therapeutic agent to treat inflammatory bowel disease.

In a particular embodiment, the further therapeutic agent is an IFN-γ antagonist, such as an anti-IFN-γ antibody, an IFN-γ receptor antibody or a native IFN-γ receptor.

In a different embodiment, the further therapeutic agent is a TNF-α antagonist, such an anti-TNF-α antibody, a TNF-α receptor antibody or a native TNF-α receptor.

In various embodiments, the antibodies can be chimeric, humanized and human.

The subject treated preferably is a human patient.

In another aspect, the invention concerns a method of reducing gastrointestinal inflammation, comprising administering to a subject in need a bispecific or cross-reactive antibody specifically binding to IL-17C and a further cytokine, or an antigen-binding fragment of such antibody. In one embodiment, the further cytokine is a proinflammatory cytokine. In another embodiment, the proinflammatory cytokine is selected from the group consisting of TNFα, IL-1β, IL-22 and IL-17A. In yet another embodiment, the proinflammatory cytokine is TNFα or IL-1β. In a further embodiment, the gastrointestinal inflammation is associated with inflammatory bowel disease. In a particular embodiment, the inflammatory bowel disease is ulcerative colitis. In another embodiment, the antibody is bispecific. In other embodiments, the antibody is chimeric, humanized or human, and may be an antibody fragment, such as Fab, Fab', F(ab')₂, and Fv fragments; diabodies; linear antibodies; single-chain antibody molecules; and multispecific antibodies formed from antibody fragments.

The subject treated preferably is a human patient.

In yet another aspect, the invention concerns the treatment of psoriasis, comprising administering to a subject in need a bispecific or cross-reactive antibody specifically binding to IL-17C and a further cytokine, or an antigen-binding fragment of such antibody.

In one embodiment, the further cytokine is a proinflammatory cytokine.

In another embodiment, the proinflammatory cytokine is selected from the group consisting of TNFα, IL-1β, IL-22 and IL-17A.

In yet another embodiment, the proinflammatory cytokine is TNFα or IL-1β.

In another embodiment, the antibody is bispecific.

In other embodiments, the antibody is chimeric, humanized or human, and may be an antibody fragment, such as Fab, Fab', F(ab')₂, and Fv fragments; diabodies; linear antibodies; single-chain antibody molecules; and multispecific antibodies formed from antibody fragments.

The subject treated preferably is a human patient.

In another aspect, the present invention concerns a pharmaceutical composition comprising an IL-17RA and/or IL-17RE antagonist in admixture with a pharmaceutically acceptable excipient, for the treatment of gastrointestinal inflammation. Preferably, the gastrointestinal inflammation is associated with inflammatory bowel disease (IBD). In one embodiment, the the IL-17RA and/or IL-17RE antagonist is an antibody or a fragment thereof. In another embodiment, the antibody is a monoclonal antibody. In certain embodiments, the antibody is a chimeric, humanized or human antibody. In certain other embodiments, the antibody is a bispecific, multispecific or cross-reactive antibody.

In another aspect, the present invention concerns a pharmaceutical composition comprising an IL-17RA and/or IL-17RE antagonist in admixture with a pharmaceutically acceptable excipient, for the treatment of psoriasis. In one embodiment, the the IL-17RA and/or IL-17RE antagonist is an antibody or a fragment thereof. In another embodiment, the antibody is a monoclonal antibody. In certain embodiments, the antibody is a chimeric, humanized or human antibody. In certain other embodiments, the antibody is a bispecific, multispecific or cross-reactive antibody.

In another aspect, the present invention concerns a pharmaceutical composition comprising an IL-17RA and/or IL-17RE antagonist in admixture with a pharmaceutically acceptable excipient, for the treatment of asthma. In one embodiment, the the IL-17RA and/or IL-17RE antagonist is an antibody or a fragment thereof. In another embodiment, the antibody is a monoclonal antibody. In certain embodiments, the antibody is a chimeric, humanized or human antibody. In certain other embodiments, the antibody is a bispecific, multispecific or cross-reactive antibody.

In yet another aspect, the present invention concerns the use of an IL-17RA and/or IL-17RE antagonist in the preparation of a medicament for the treatment of gastrointestinal inflammation. Preferably, the gastrointestinal inflammation is associated with inflammatory bowel disease (IBD).

In yet another aspect, the present invention concerns the use of an IL-17RA and/or IL-17RE antagonist in the preparation of a medicament for the treatment of psoriasis.

In yet another aspect, the present invention concerns the use of an IL-17RA and/or IL-17RE antagonist in the preparation of a medicament for the treatment of asthma.

In another aspect, the present invention provides a kit for treating gastrointestinal inflammation, said kit comprising: (a) a container comprising an IL-17RA and/or IL-17RE antagonist; and (b) a label or instructions for administering said antibody to treat said inflammation. Preferably, the gastrointestinal inflammation is associated with inflammatory bowel disease (IBD).

In another aspect, the present invention provides a kit for treating psoriasis, said kit comprising: (a) a container comprising an IL-17RA and/or IL-17RE antagonist; and (b) a label or instructions for administering said antibody to treat said psoriasis.

In another aspect, the present invention provides a kit for treating asthma, said kit comprising: (a) a container comprising an IL-17RA and/or IL-17RE antagonist; and (b) a label or instructions for administering said antibody to treat said asthma.

### Brief Description of the Drawings

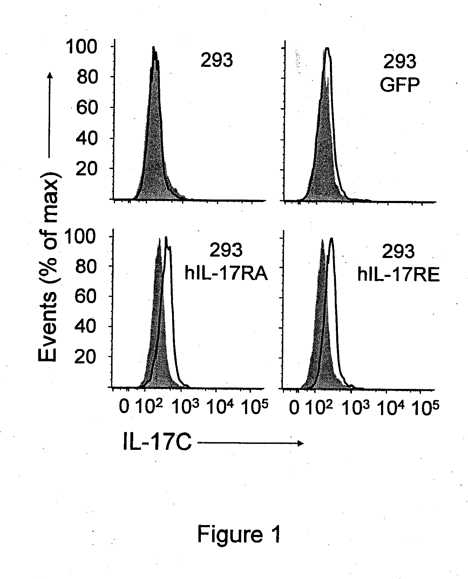

**Figure 1****.** The biological effects of IL-17C are mediated through IL-17RA and IL-17RE heterodimeric receptor complexes. Flow-cytometric detection of human hIL-17C binding to 293, or 293 cells expressing GFP, hIL-17RA, or hIL-17RE. Cells were incubated with FLAG-tagged hIL-17C for 30 min followed by staining with anti-FLAG antibody to assess cytokine binding (bold line). Shaded histograms show staining with anti-FLAG antibody in the absence of hIL-17C. Data are representative of three independent experiments. Data are representative of three independent experiments.

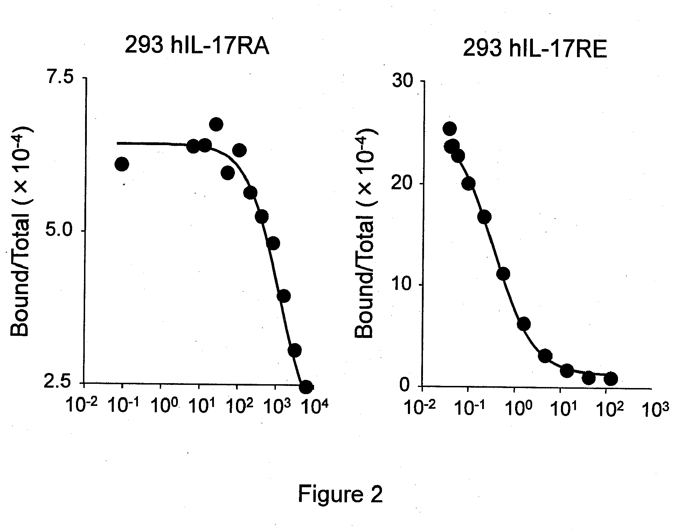

**Figure 2****.** Competition binding of hIL-17C to hIL-17RA or hIL-17RE. The curves show displacement of ¹²⁵I-hIL-17C bound to 293 cells expressing hIL-17RA or hIL-17RE by increasing doses of unlabeled hIL-17C. Data are representative of three independent experiments. Data are representative of three independent experiments.

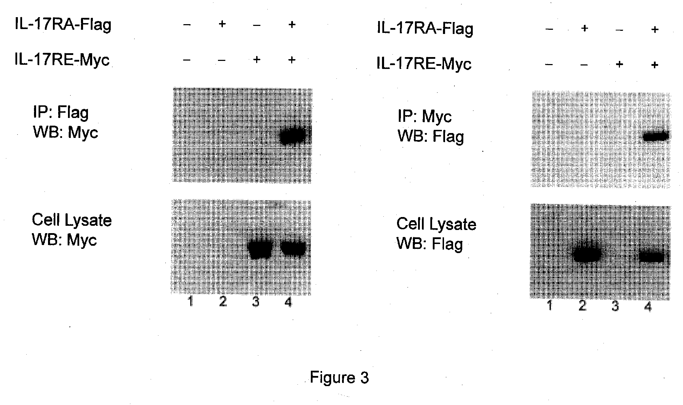

**Figure 3****.** IL-17RA and IL-17RE receptor chains form heterodimeric complexes. 293 cells were transfected with Flag epitope tagged IL-17RA (IL-17RA-Flag), Myc epitope tagged IL-17RE (IL-17RE-Myc) or in combination. Co-immunoprecipitations (IP) were performed using either anti-Flag (right panel) or anti-Myc (left panel) antibodies, followed by western blotting (WB) with anti-Myc or anti-Flag antibodies as indicated. Cell lysates (bottom panels) were blotted with anti-Myc or anti-Flag antibodies as indicated.

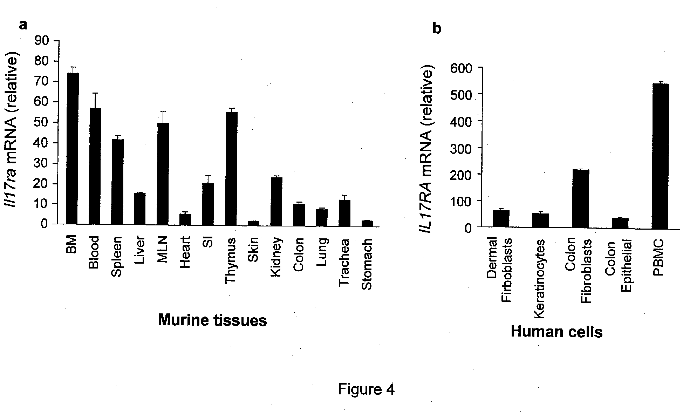

**Figure 4****.** IL-17RA displays broad tissue distribution. qRT-PCR analysis of *IL17RA* mRNA in the indicated murine tissues (top) and human cell-types (bottom). Expression is shown relative to the housekeeping genes *Rpl19* and *RPL19* respectively; error bars, s.d. (n=2).

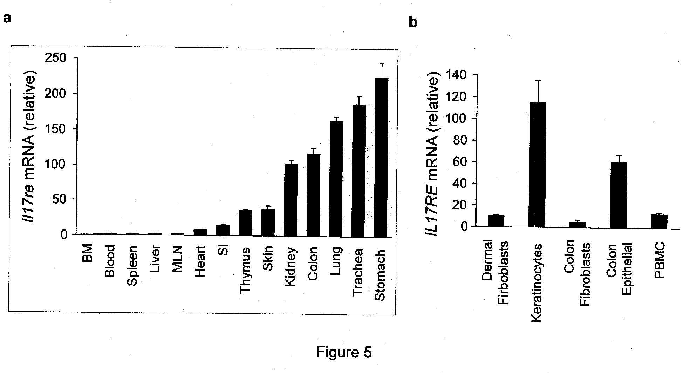

**Figure 5****.** Quantitative PCR (qRT-PCR) analysis of *IL17RE* mRNA in the indicated murine tissues (**a**) and human cell-types (**b**). Expression is shown relative to the housekeeping gene *RPL19*; error bars, s.d. (n=3).

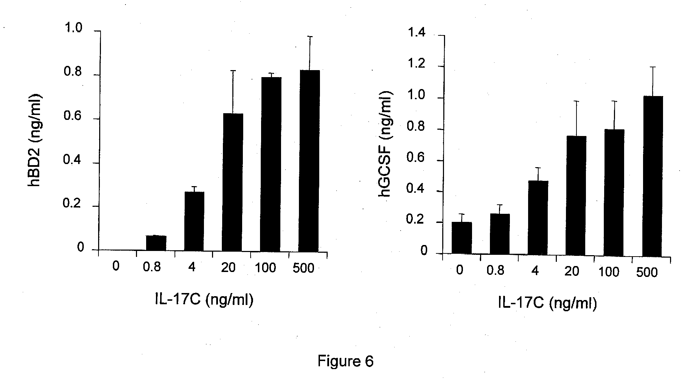

**Figure 6****.** ELISA of human β-Defensin2 (hBD2) and hGCSF secretion from human epidermal keratinocytes (HEKn) stimulated with hIL-17C for 48 h; error bars, s.d. (n=3).

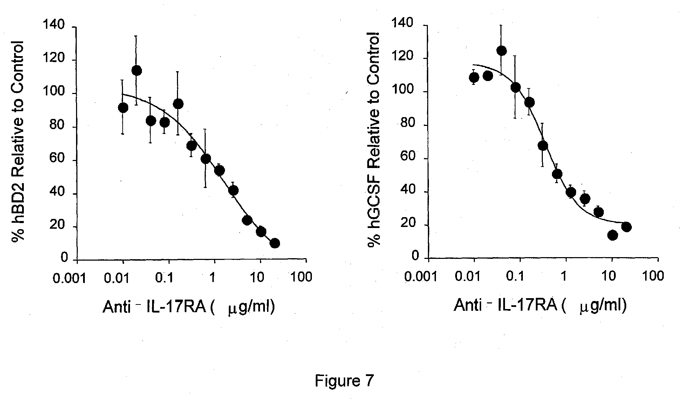

**Figure 7****.** Stimulations and protein measurements, performed as in e, with increasing doses of anti-hIL-17RA blocking antibody; mean values (n=3).

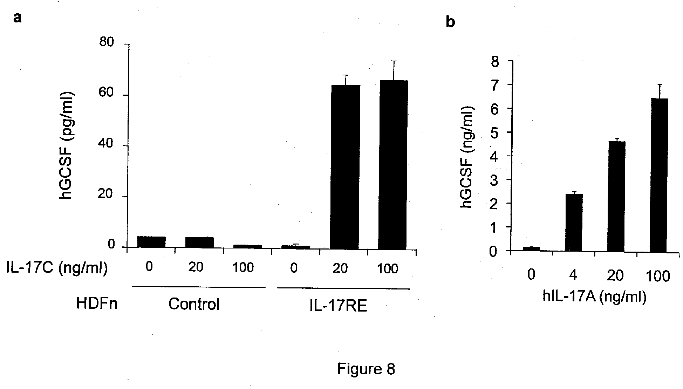

**Figure 8****.** (a) G-CSF production in human dermal fibroblasts (HDFn) retro-virally transduced to express hIL-17RE, stimulated with hIL-17C for 48 h and measured as in e; error bars, s.d. (n=3). (b) Human dermal fibroblasts are responsive to IL-17A. ELISA analysis of G-CSF production in HDFn cells stimulated with hIL-17A for 24 hours; error bars, s.d. (n=3).

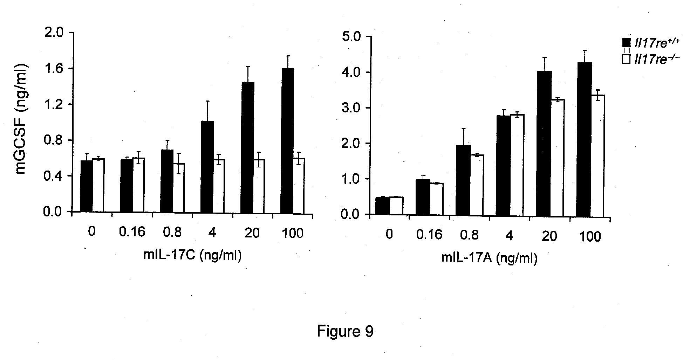

**Figure 9****.** G-CSF secretion in keratinocytes derived from *Il17re*^{*+*/*+*} or *Il17re*^{*-*/*-*} neonatal mice stimulated with IL-17C or IL-17A. Data are representative of three independent experiments.

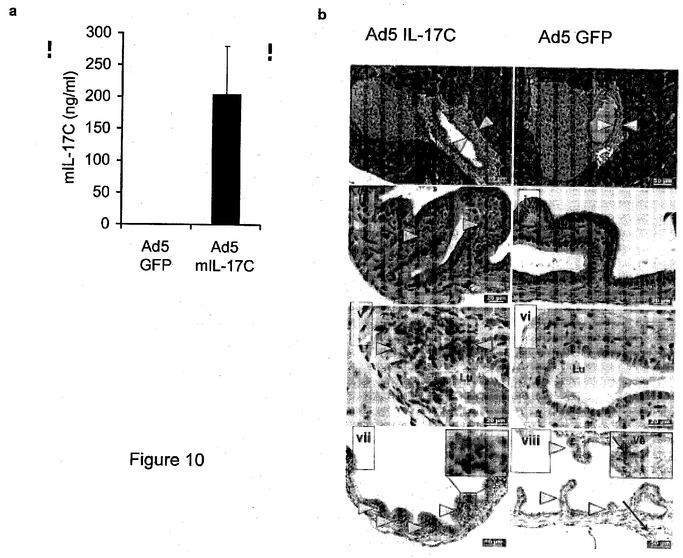

**Figure 10****.** Ectopic expression of IL-17C in vivo promotes neutrophil mobilization in an IL-17RE dependent manner. Wild-type mice were injected with 1 x 10¹⁰ PFU of either Ad5-GFP or murine IL-17C (Ad5-mIL-17C) adenovirus. (**a**) ELISA of mIL-17C in serum of Ad5-GFP or Ad5-mIL-17C infected mice collected on day 7 post-infection. (**b**) Histological analyses of pancreas (**i**-**iv**) and gall bladders **(v-viii)** collected on day 21 post-infection with Ad5-IL-17C (left column) or Ad5-GFP (right column) and stained with H&E (**i**-**iv**) or Ly6G/C **(v-viii).** Yellow arrowheads denote neutrophil clusters within tissues. Arrows illustrate neutrophil infiltration into the tissue. Results are representative of 3 independent experiments. Abbreviations: Isl, islet; Lu, lumen; Ve, venule.

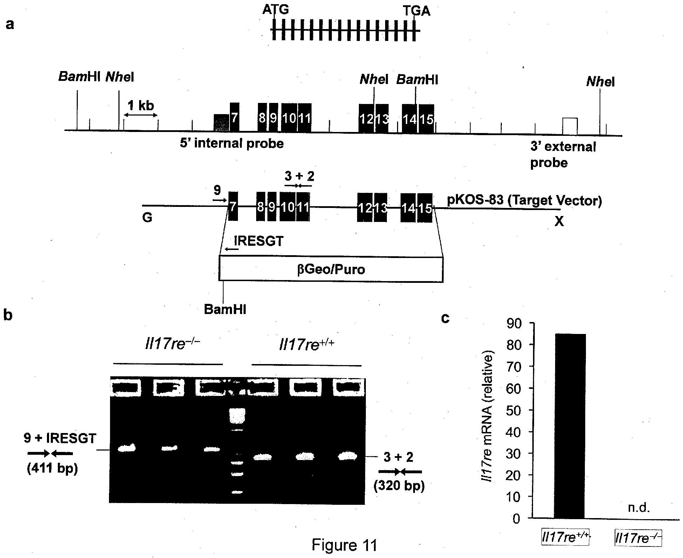

**Figure 11****.** Generation of *Il17re* deficient mice. (**a**) Targeting strategy for generation of *Il17re^{-l-}* mice. Exons 7-15 were replaced with b-galactosidase-neomycin and Puromycin resistance cassettes. (**b**) Genomic PCR analysis of tail DNA from *Il17re⁺¹⁺* and *Il17re^{-l-}* mice confirming genotypes. (**c**) qRT-PCR analysis of *Il17re* mRNA in ear tissue harvested from *Il17re^{+l+}* and *Il17re^{-l-}* mice. Expression is shown relative to the housekeeping genes *Rpl19*; error bars, s.d.

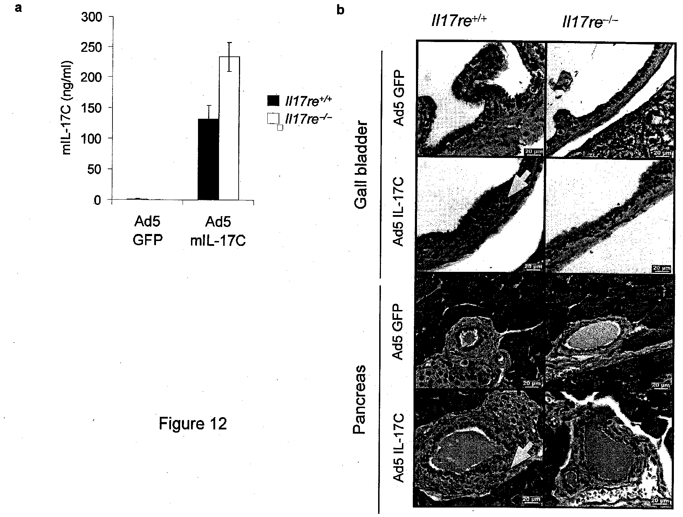

**Figure 12****.** Ectopic expression of IL-17C in vivo promotes neutrophil mobilization in an IL-17RE dependent manner. *Il17re^{+l+}* or *Il17re^{-l-}* mice were injected with 1 x 10¹⁰ PFU of either Ad5-GFP or murine IL-17C (Ad5-mIL-17C) adenovirus. (**a**) ELISA of mIL-17C in serum of Ad5-GFP or Ad5-mIL-17C infected *Il17re^{+l+}* (black bars) and *Il17re^{-l-}* (white bars) mice collected on day 8 post-infection. Bars represent mean of average values ± s.e.m. (**b**) Histological analyses of pancreas and gall bladder tissues derived from *Il17re^{+l+}* or *Il17re^{-l-}* mice infected with Ad5-GFP or Ad5-IL-17C and stained with H&E. Arrows illustrate neutrophil infiltration into the tissue. Results are representative of 3 independent experiments. Abbreviations: Isl, islet; Lu, lumen; Ve, venule.

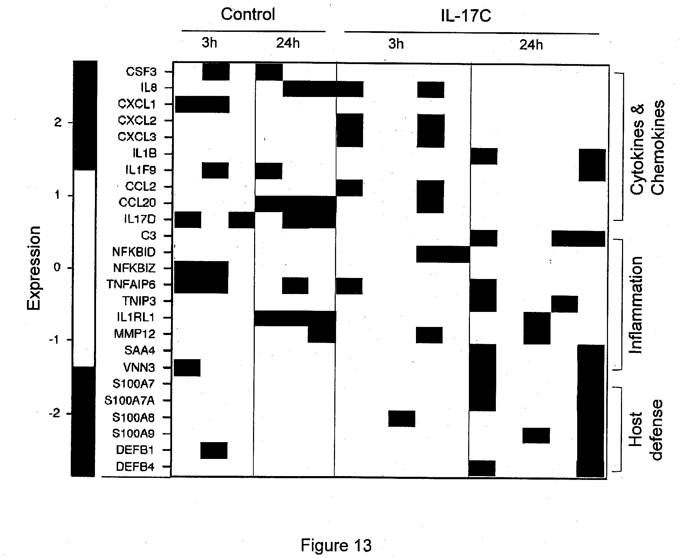

**Figure 13****.** IL-17C induces host defense pathways in epithelial cells. Microarray analysis of RNA from HEKn cells stimulated with hIL-17C for 3 h and 24 h. Control indicates mock stimulation without cytokine.

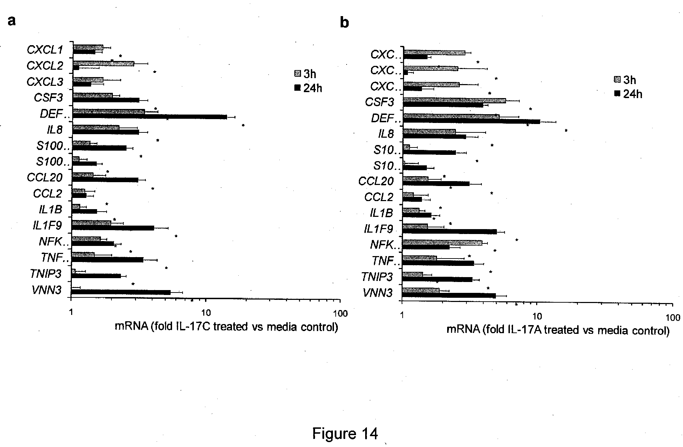

**Figure 14****.** qRT-PCR analyses of mRNA of the indicated chemokines, IL-1 family cytokines and anti-microbial peptides from HEKn cells treated with hIL-17C (**a**) or hIL-17A (**b**) for 3 and 24 h. Data are shown relative to mock treated (control) samples; error bars, s.e.m. (n=5) * = p < 0.05 (Dunnett's test).

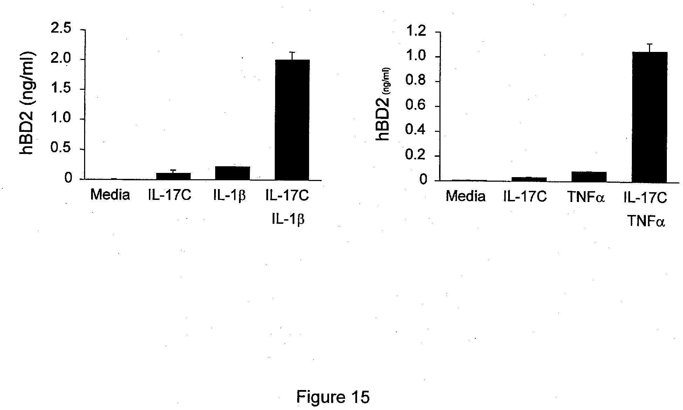

**Figure 15****.** ELISA analysis of hBD2 secretion from HEKn cells stimulated with hIL-17C, hTNFα, hIL-1β or in combination for 48 hours; error bars, s.d. (n=3).

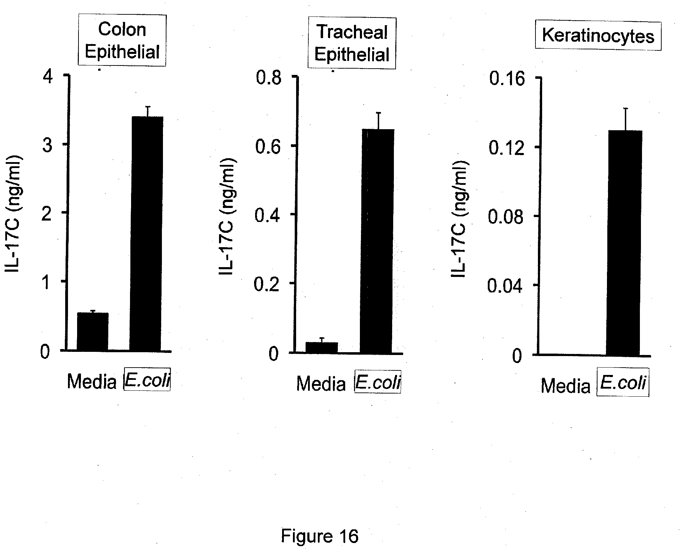

**Figure 16****.** IL-17C is expressed by mucosal epithelial cells in response to inflammation. ELISA of hIL-17C secretion from human epithelial cells (HCT-15 colon epithelial cells, primary tracheal epithelial cells or HEKn keratinocytes) stimulated with heat-killed *E. coli* for 24 h; error bars, s.d. (n=3).

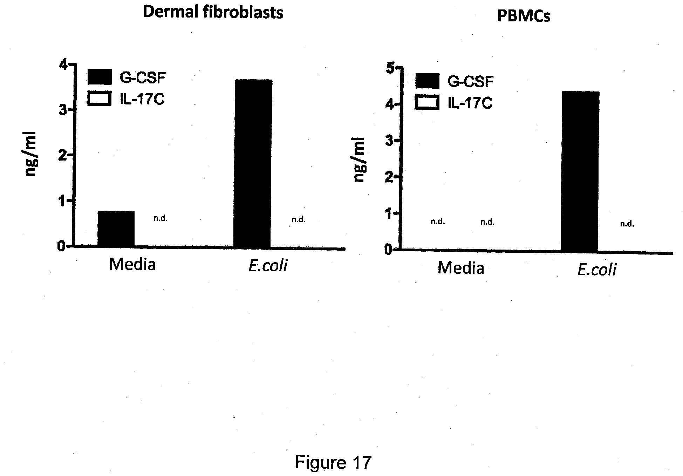

**Figure 17****.** TLR and cytokine stimuli specifically induce IL-17C from epithelial cells. ELISA of hG-CSF (black bars) or hIL-17C (open bars) secretion from HDFn cells or Peripheral blood mononuclear cells (PBMCs) stimulated with heat-killed *E. Coli* for 24 h; n.d.= not detectable.

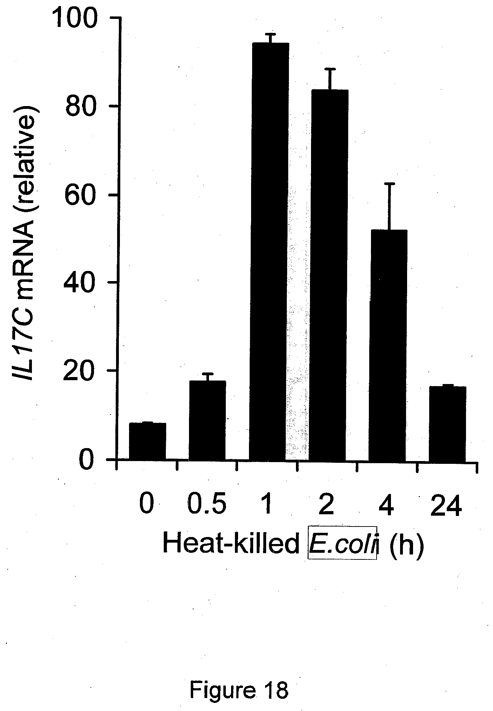

**Figure 18****.** qRT-PCR analyses of *IL17C* mRNA kinetics from HCT-15 cells stimulated with heat-killed *E. coli.*

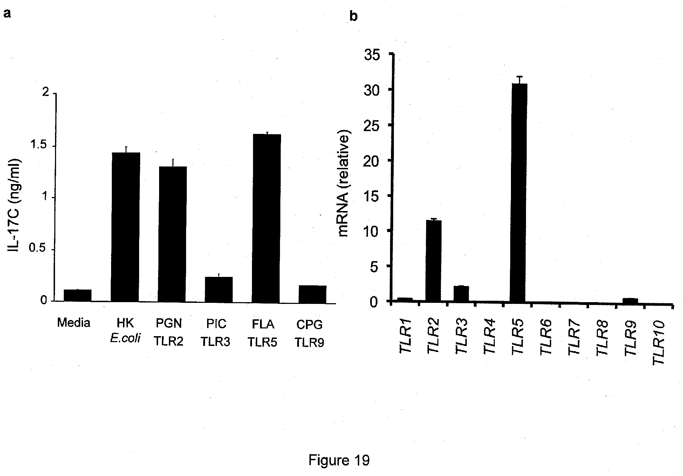

**Figure 19****.** (a) ELISA of hIL-17C secretion from HCT-15 cells stimulated with the indicated TLR agonists for 24 h; error bars, s.d. (n=3). Data are representative of three independent experiments. (b) qRT-PCR analysis of *TLR* mRNA expression in HCT-15 cells.

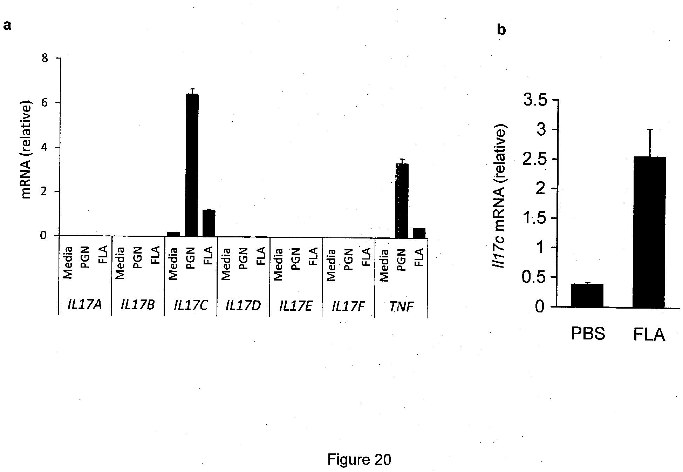

**Figure 20****.** (a) qRT-PCR analyses of *IL17* family or *TNF* mRNA in HCT-15 cells stimulated with agonists to TLR2 (PGN) or TLR5 (FLA) for 2 h. (b) Leukocytes are not a predominant source of IL-17C *in vivo.* qRT-PCR analyses of *Il17c* mRNA in colon tissue harvested from C57BL/6 mice injected intra-peritoneally (i.p.) with PBS or flagellin (FLA) for 2h.

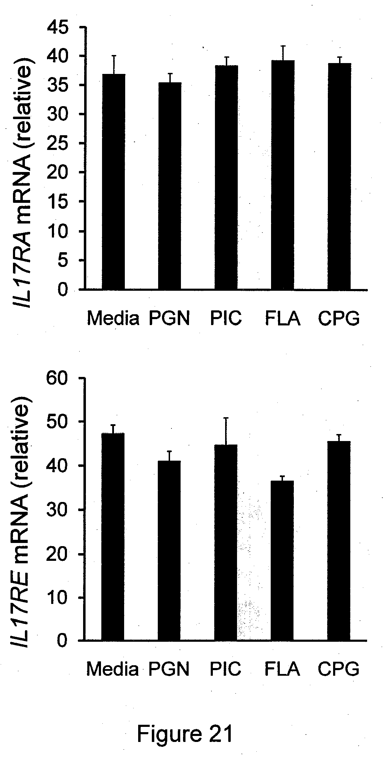

**Figure 21****.** qRT-PCR analyses of *IL17* family or *TNF* mRNA in HCT-15 cells stimulated with TNFa or IL-1b for 2 h. Expression is shown relative to the housekeeping genes *RPL19;* error bars, s.d. (n=3).

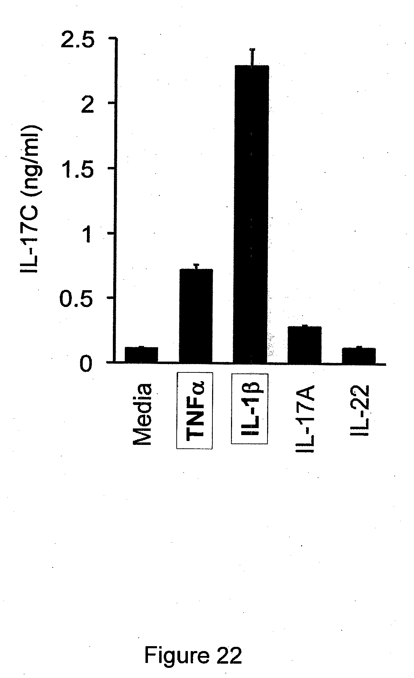

**Figure 22****.** ELISA of hIL-17C secretion from HCT-15 cells stimulated with the indicated cytokines for 24 h; error bars, s.d. (n=3). Data are representative of three independent experiments.

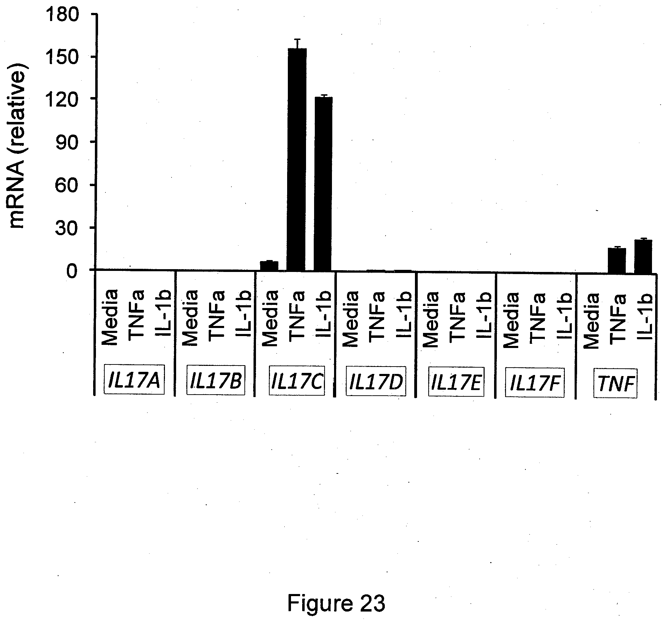

**Figure 23****.** qRT-PCR analyses of *IL17RA* (top) or *IL17RE* (bottom) mRNA from HCT-15 cells stimulated with the indicated TLR agonists for 2 h.

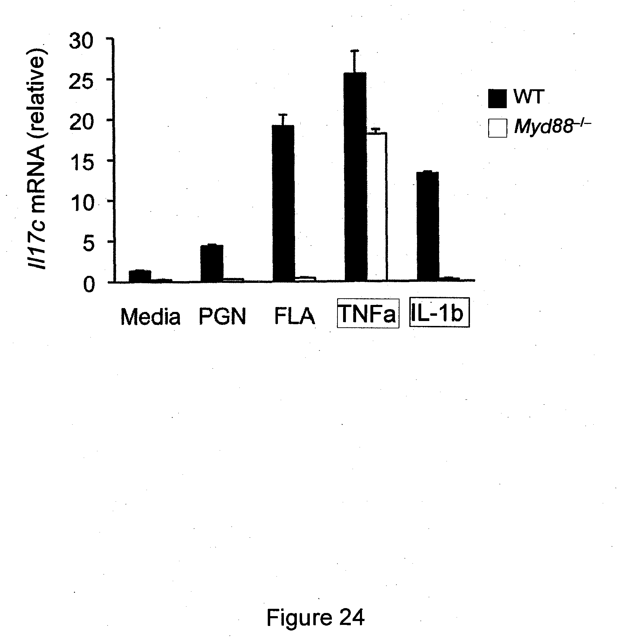

**Figure 24****.** Multiple factors independently regulate IL-17C expression. qRT-PCR analyses of *Il17c* mRNA in epidermal keratinocytes derived from *Myd88*^{*+*/*+*} or *Myd88*^{*-*/*-*} neonatal mice, stimulated with agonists to TLR2 or TLR5, or the cytokines IL-1β or TNFα for 2 hours. Expression shown is relative to housekeeping gene *Rpl19.*

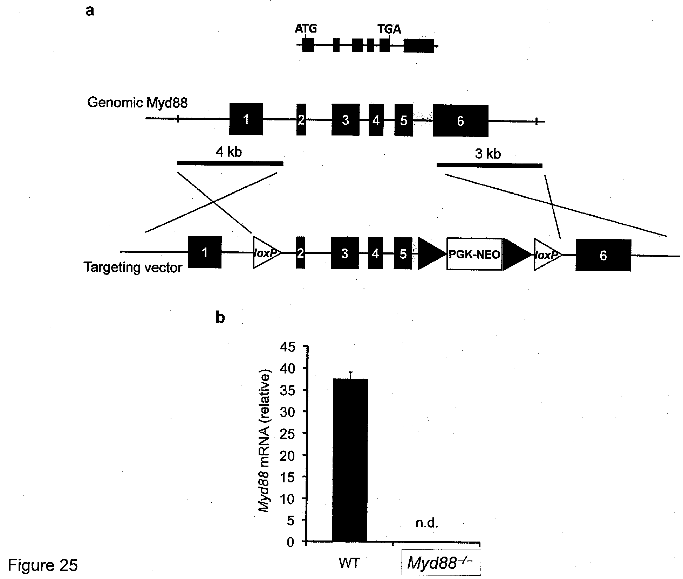

**Figure 25****.** Generation of *Myd88* deficient mice. (**a**) Schematic representation of targeting construct design for generation of *Myd88⁻¹⁻* mice. CRE recombinase excision of exons 2 to 5 was accomplished by crossing of heterozygous mice with ROSA-CRE transgenic mice. The neomycin resistance cassette was excised prior to microinjection. (**b**) qRT-PCR analyses of *Myd88* mRNA expression from *Myd88*^{*+*/*+*} or *Myd88*^{*-*/*-*} derived primary epidermal keratinocytes. Expression is shown relative to the housekeeping genes *Rpl19*; error bars, s.d.; n.d.= not detectable.

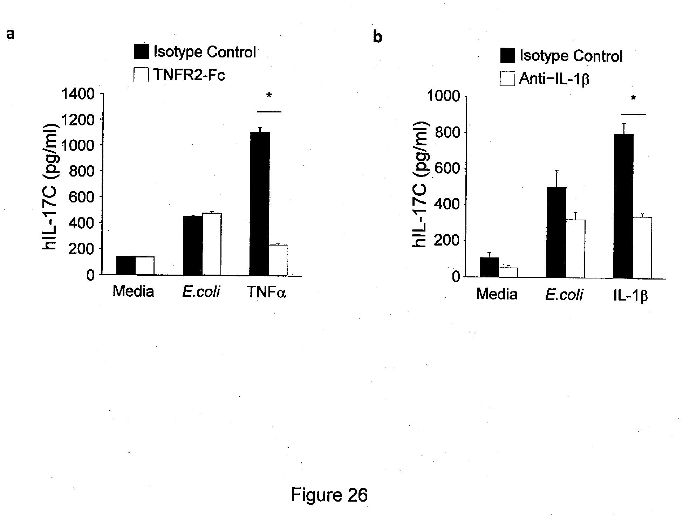

**Figure 26****.** ELISA of hIL-17C secretion from HCT-15 cells stimulated with heat-killed *E.coli,* TLR agonists or cytokines in the presence of TNFRII-Fc (**a**) or anti-IL-1β (**b**) for 24 h. Data shown represent ± s.d. Stimulations were performed in triplicate. * = p < 0.05 (Dunnett's test against isotype control group).

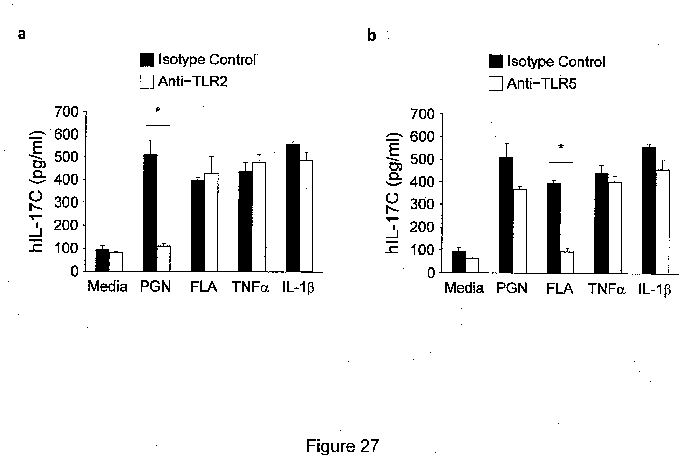

**Figure 27****.** ELISA of hIL-17C secretion from HCT-15 cells stimulated with heat-killed *E.coli,* TLR agonists or cytokines in the presence of anti-TLR2 (**a**), or anti-TLR5 (**b**) for 24 h. Data shown represent ± s.d. Stimulations were performed in triplicate. * = p < 0.05 (Dunnett's test against isotype control group).

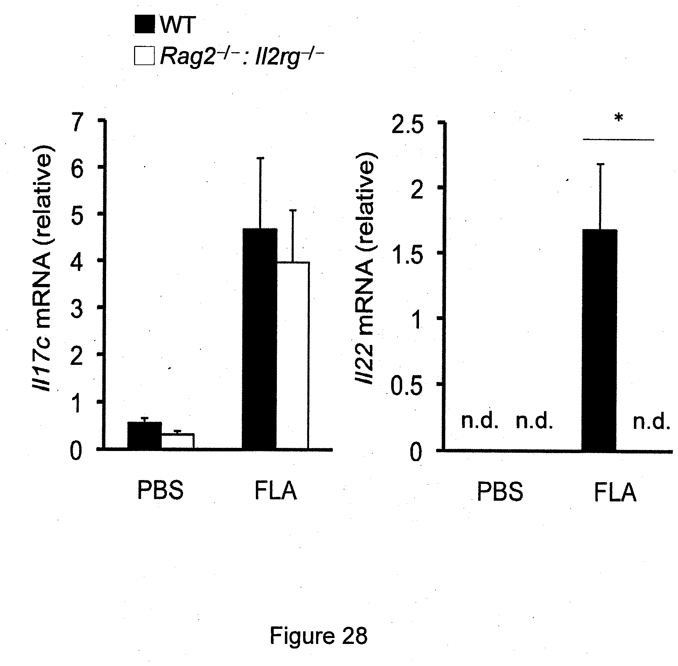

**Figure 28****.** Leukocytes are not a predominant source of IL-17C *in vivo.* qRT-PCR analyses *of Il17c* or *Il22* mRNA in colon tissue harvested from wild-type or *Rag2⁺:Ilrg*^{*-*/*-*} mice treated as in Figure 20(b); * = p < 0.05 (Dunnett's test against wild-type mice). Each data column in (**a**-**c**) represents the mean value ± s.e.m. of 5 animals. Expression is relative to the housekeeping gene *Rpl19.* n.d. = not detectable. Data is representative of 2 independent experiments.

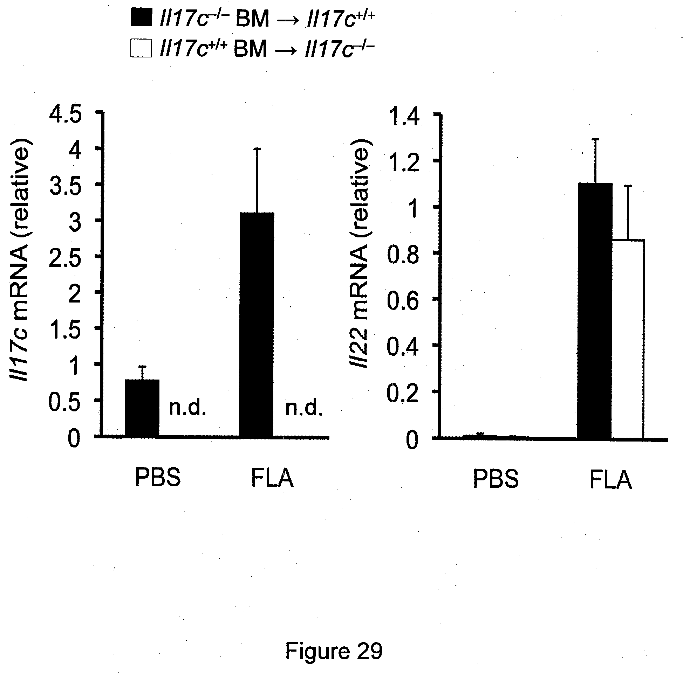

**Figure 29****.** Leukocytes are not a predominant source of IL-17C *in vivo.* qRT-PCR analyses of *Il17c^{-l-}* BM → *Il17c*^{*+*/*+*} and *Il17c*^{*+*/*+*} BM → *Il17c^{-l-}* chimeras treated as in Figure 20(b); * = p < 0.05 (Dunnett's test against wild-type mice). Each data column in represents the mean value ± s.e.m. of 5 animals. Expression is relative to the housekeeping gene *Rpl19.* n.d. = not detectable. Data is representative of 2 independent experiments.

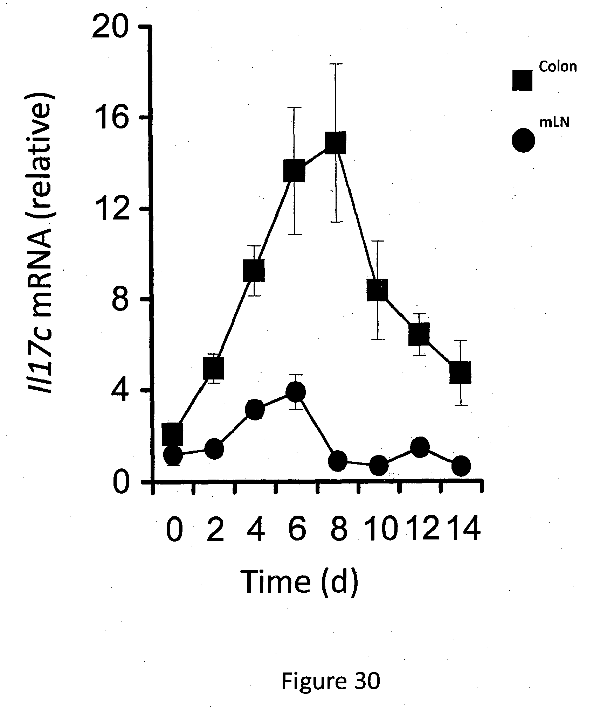

**Figure 30****.** IL-17C is expressed during DSS colitis. qRT-PCR measurement of *Il17c* mRNA from colon (squares) or mLN (circles) harvested from C57BL/6 mice on the indicated days following treatment with 2% DSS.

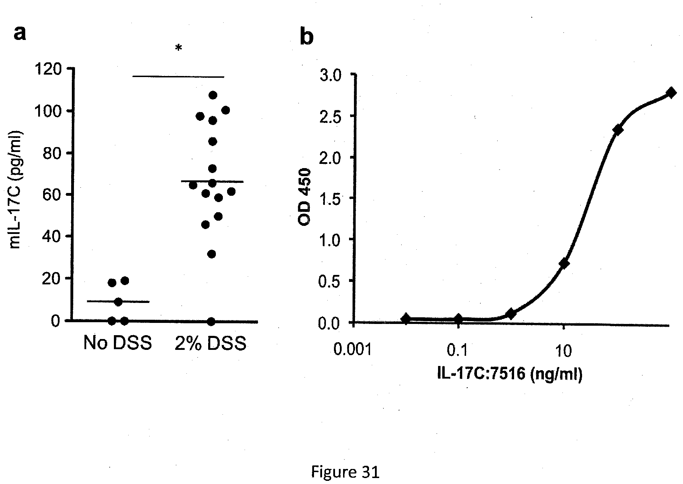

**Figure 31****.** (a) ELISA of mIL-17C production from over-night colon cultures. Colons were harvested on day 8 from C57BL/6 mice treated as in (**b**); * = p < 0.05 (Dunnett's test against No DSS group). (b) Characterization of mouse anti-mouse IL-17C monoclonal antibody. Direct ELISA measurement of the reactivity of increasing concentrations of biotinylated anti-IL-17C monoclonal antibody (IL-17C:7516) to plate bound mouse IL-17C.

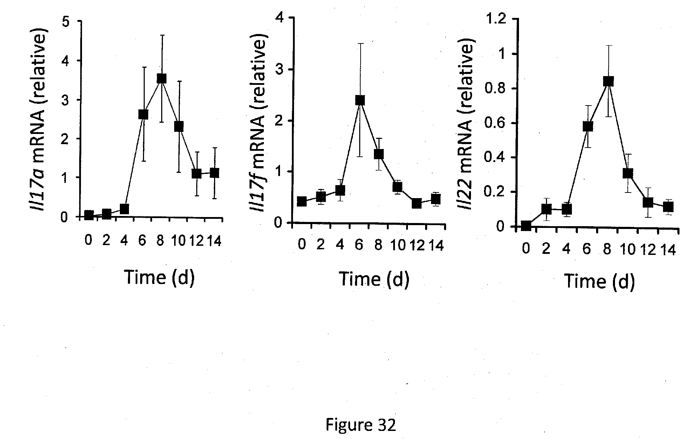

**Figure 32****.** qRT-PCR analyses performed as in Figure 20(b) of *Il17a, Il17f* and *Il22* mRNA. Expression is shown relative to the housekeeping gene *Rpl19.* Each timepoint represents the mean value from 5 animals performed in triplicate; error bars, s.e.m.

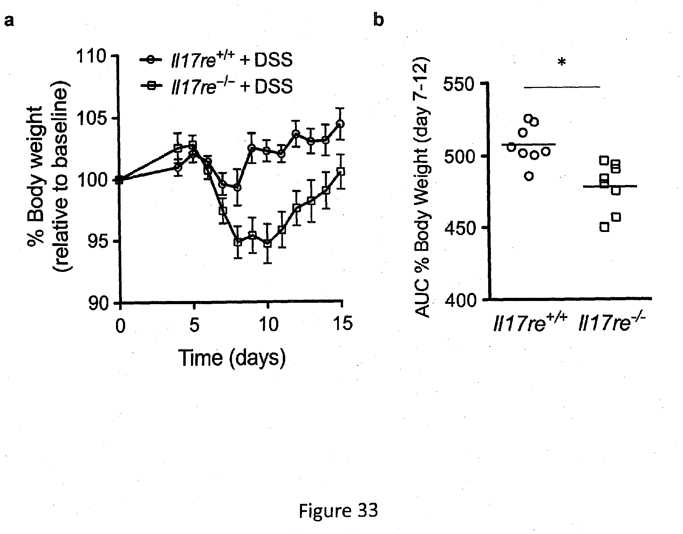

**Figure 33****.** IL-17RE pathway plays a role in the recovery of epithelial cells in the DSS-induced colitis model. 8-10 week old *Il17re*^{*+*/*+*} or *Il17re*^{*-*/*-*} were treated as in Figure 30. (**a**) Percent body weight on days 4 through 15 is plotted relative to body weight at start of study. (**b**) AUC of individual animals for percent body weight in (**a**) calculated for days 7-12. Data represent individual animals (n=4 per group for no DSS controls, n=8 per group for DSS treated animals). Data are representative of two independent experiments. * = p < 0.05 (Dunnett's test against *Il17re*^{*+*/*+*} mice).

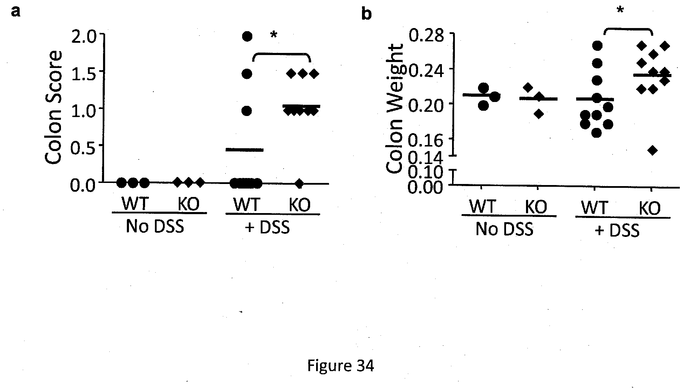

**Figure 34****.** Colon weights (a) and colon scores (b) were measured on day 15

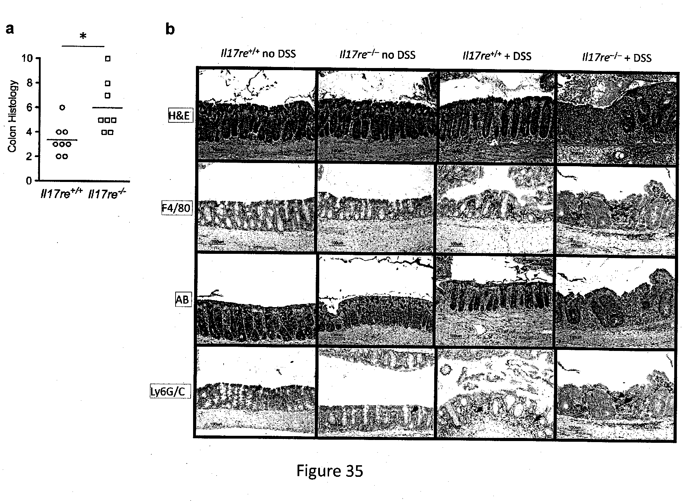

**Figure 35****.** IL-17RE pathway plays a role in the recovery of epithelial cells in the DSS-induced colitis model. 8-10 week old *Il17re*^{*+*/*+*} or *Il7re*^{*-*/*-*} were treated as in Figure 30. Colons were collected on day 15 for histological analyses. Colon histology scores are indicated in (a). (**b**) H&E, F4/80, Alcian Blue (AB) and Ly6G/C staining of colon sections. Arrows indicate F4/80⁺ macrophages (brown staining), AB⁺ goblet cells and mucin, and Ly6G/C⁺ neutrophil infiltrates (brown staining). Data represent individual animals (n=4 per group for no DSS controls, n=8 per group for DSS treated animals). Data are representative of two independent experiments. * = p < 0.05 (Dunnett's test against *Il17re*^{*+*/*+*} mice).

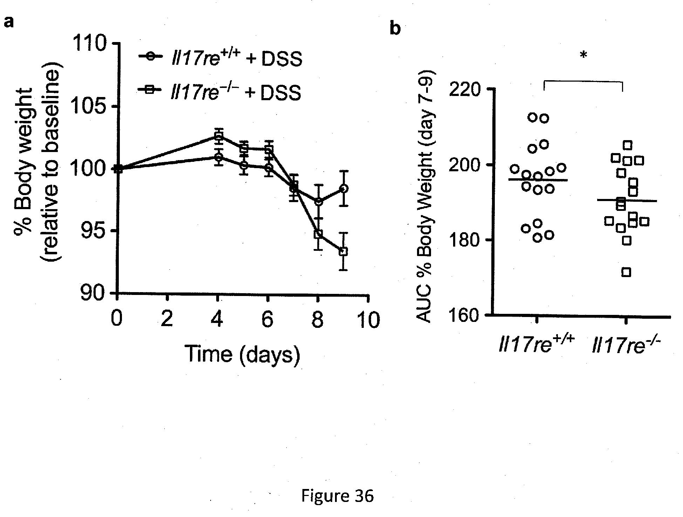

**Figure 36****.** Loss of the IL-17C pathway aggravates disease and augments inflammation during acute DSS induced colitis. 8-10 week old *Il17re*^{*+*/*+*} and *Il17re*^{*-*/*-*} mice were treated as in Figure 30 with 1.5 % DSS. (**a**) Percent body weight on days 4-9 relative to body weight at the start of the study. (**b**) AUC of individual animals for percent body weight in (**a**). Data represent individual animals (n=5 per group for no DSS controls, n=16 per group for DSS treated animals). Data are representative of two independent experiments. *=p<0.05 (Dunnett's test against *Il17re*^{*+*/*+*} mice).

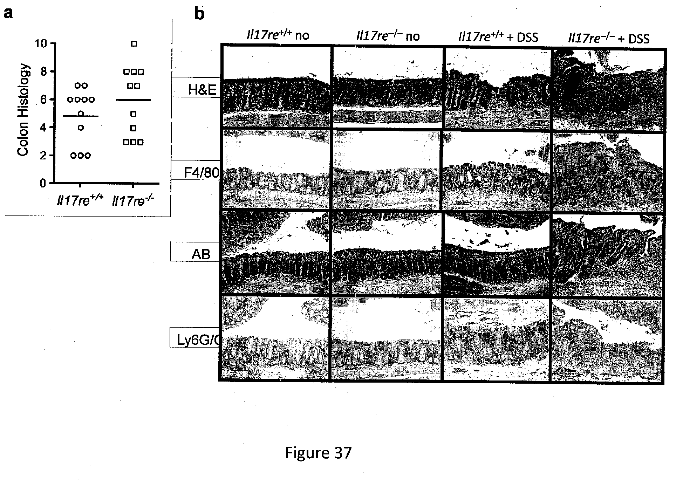

**Figure 37****.** Colons were collected on day 9 for histological analyses. Colon histology scores are indicated in (**a**). (**b**) H&E, F4/80, AB, Ly6G/C staining of colon sections. Arrows indicate F4/80⁺ macrophages (brown staining), AB+ goblet cells and mucin, and Ly6G/C⁺ neutrophil infiltrates (brown staining). Data represent individual animals (n=5 per group for no DSS controls, n=16 per group for DSS treated animals). Data are representative of two independent experiments. *=p<0.05 (Dunnett's test against *Il17re⁺¹⁺* mice).

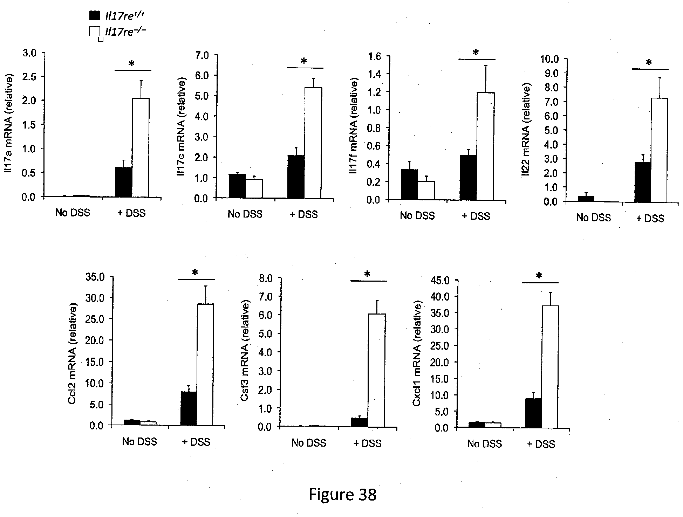

**Figure 38****.** qRT-PCR analyses of mRNA for indicated cytokines in colon tissues. Data represent individual animals (n=5 per group for no DSS controls, n=16 per group for DSS treated animals). Data are representative of two independent experiments. *=p<0.05 (Dunnett's test against *Il17re⁺¹⁺* mice).

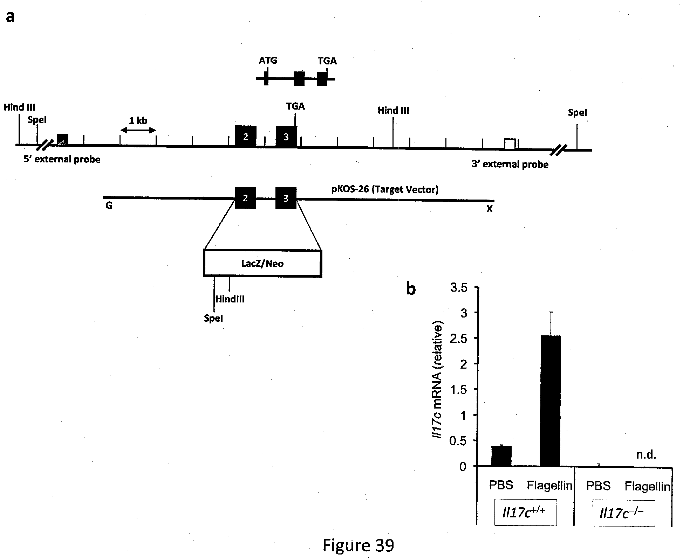

**Figure 39****.** Generation of *Il17c* deficient mice. (**a**) Targeting strategy for generation of *Il17c^{-l-}* mice. Exons 2 and 3 were replaced with a neomycin resistance cassette. (**b**) qRT-PCR analyses of *Il17c* mRNA in colons derived from *Il17c*^{*+*/*+*} or *Il17c^{-l-}* mice injected with flagellin i.p. for 2 hours. Expression is shown relative to the housekeeping genes *Rpl19.* Data shown represent mean ± s.e.m. n.d=not detectable. Data is representative of 3 independent experiments.

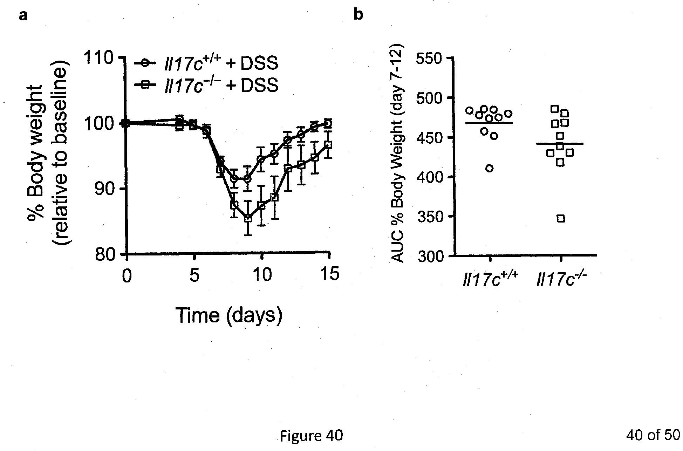

**Figure 40****.** IL-17C plays a role in the recovery of epithelial cells in the DSS-induced colitis model. 8-10 week old *Il17c^{+l+}* and *Il17c^{-l-}* mice were treated as in Figure 30 with 1.5 % DSS. (**a**) Percent body weight on days 4-15 relative to body weight at the start of the study. (**b**) AUC of individual animals for percent body weight in (**a**). Data represent individual animals (n=3 per group for no DSS controls, n=10 per group for DSS treated animals). Data are representative of two independent experiments.

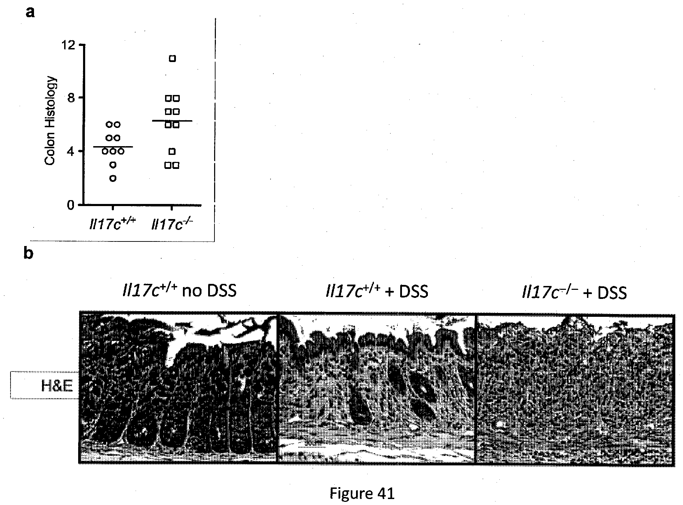

**Figure 41****.** IL-17C plays a role in the recovery of epithelial cells in the DSS-induced colitis model. 8-10 week old *Il17c^{+l+}* and *Il17c^{-l-}* mice were treated as in Figure 30 with 1.5 % DSS. Colons were collected on day 9 for histological analyses. Colon histology scores are indicated in (**a**). (**b**) H&E staining of colon sections. Data represent individual animals (n=3 per group for no DSS controls, n=10 per group for DSS treated animals). Data are representative of two independent experiments.

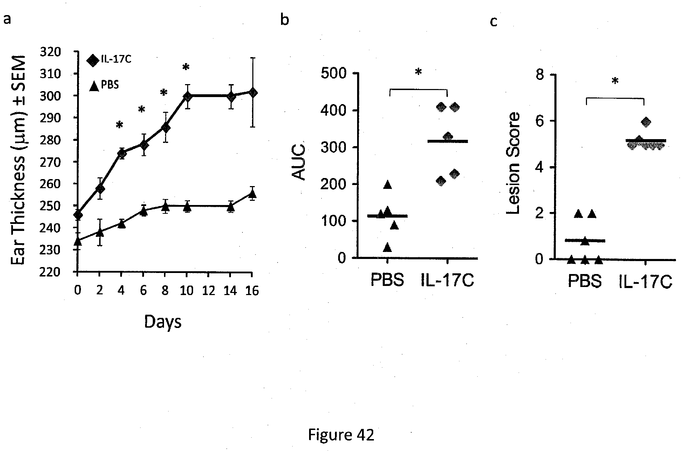

**Figure 42****.** IL-17C induces inflammation in the skin. C57BL/6 mice were injected with recombinant mIL-17C (diamonds) or PBS (triangles) in the ear. (**a**) Ear thickness measurements by caliper, *=p<0.05 (Dunnett's test against mice treated with PBS). (**b**) AUC calculated between days 4-10, *=p<0.004. Ear tissue histology scores are indicated in (**c**). Data is representative of 3 independent experiments. *=p<0.05 (Dunnett's test against mice treated with PBS).

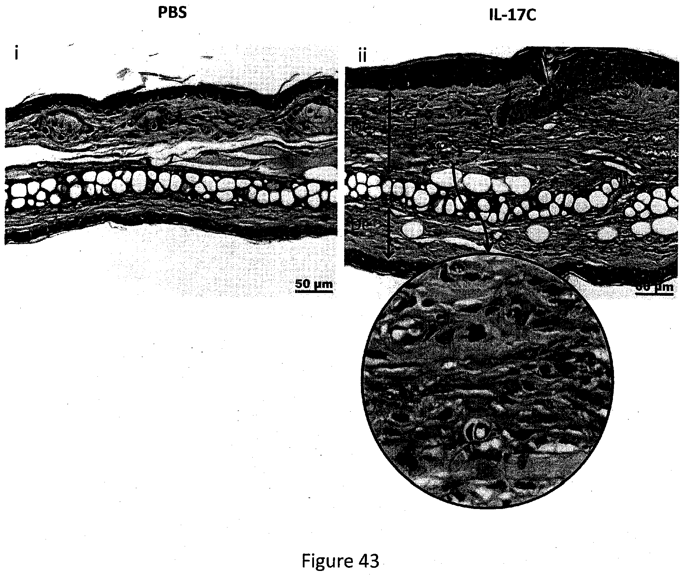

**Figure 43****.** IL-17C induces inflammation in the skin. C57BL/6 mice were injected with recombinant mIL-17C or PBS in the ear. Histological analyses of ear tissues. H&E staining of ear tissues. Yellow arrows highlight dermal (De) and epidermal (Ep) thickening. Data is representative of 3 independent experiments. *=p<0.05 (Dunnett's test against mice treated with PBS).

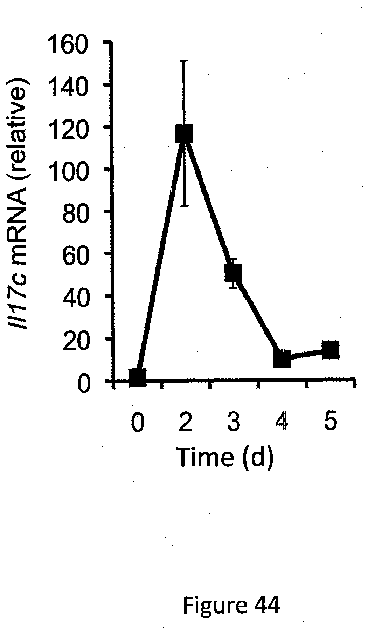

**Figure 44****.** IL-17C pathway plays a pro-inflammatory role in a mouse model of psoriasis. (**a**) qRT-PCR analyses of *Il17c* mRNA kinetics in back skin derived from BALB/c mice treated with 5% topical imiquimod for 5 days. Each timepoint represents the mean from 5 animals performed in triplicate. Data is representative of 2 independent experiments. * p < 0.05 (Dunnett's test against *Il17c*^{*+*/*+*} mice).

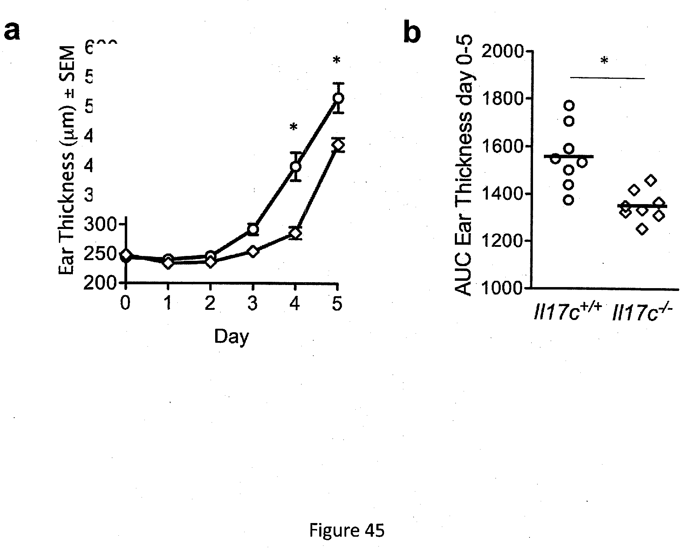

**Figure 45****.** IL-17C pathway plays a pro-inflammatory role in a mouse model of psoriasis. *Il17c*^{*+*/*+*} or *Il17c^{-l-}* mice were treated as in Figure 44. Ear thickness was measured on each day, and plotted over time, mean ± s.e.m. (**a**), or as AUC from day 0 through day 5 (**b**). Data points represent individual animals, lines indicate mean of each group. Data is representative of 2 independent experiments. * p < 0.05 (Dunnett's test against *Il17c*^{*+*/*+*} mice).

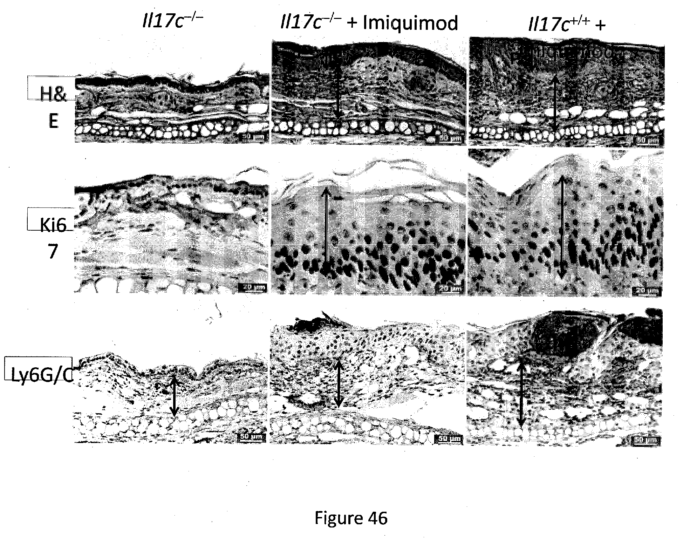

**Figure 46****.** IL-17C pathway plays a pro-inflammatory role in a mouse model of psoriasis. H&E, Ki67 and Ly6G/C staining of pinna from imiquimod-treated *Il17c*^{*+*/*+*} or *Il17c*^{*-*/*-*} mice collected on day 5. Double arrows reflect dermal and epidermal hyperplasia, (P) indicates epidermal pustules. Data is representative of 2 independent experiments. * p < 0.05 (Dunnett's test against *Il17c*^{+*l*+} mice).

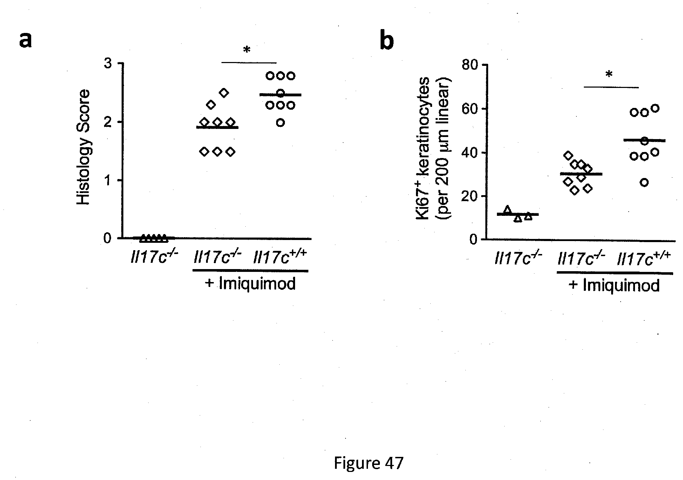

**Figure 47****.** IL-17C pathway plays a pro-inflammatory role in a mouse model of psoriasis. (**a**) Pinna histology scores. (**b**) Proliferative activity of keratinocytes as determined by Ki67 staining. Data is representative of 2 independent experiments. * p < 0.05 (Dunnett's test against *Il17c*^{+*l*+} mice).

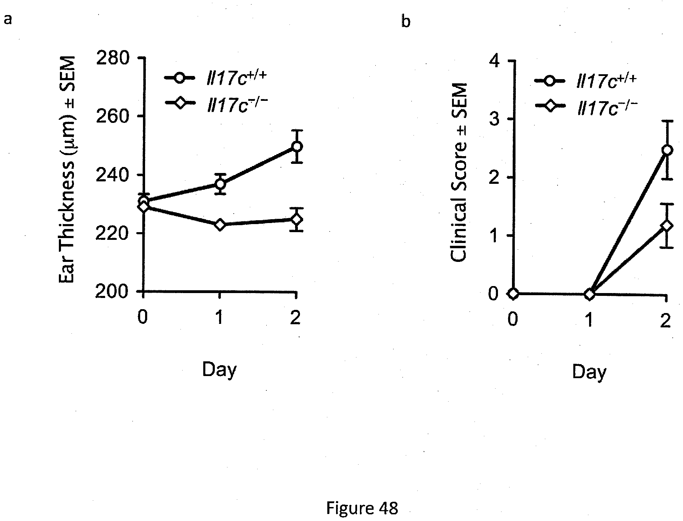

**Figure 48****.** IL-17C deficiency reduces inflammation in a mouse model of psoriasis. *Il17c*^{+*l*+} and *Il17c*^{-/-} mice were treated as in Figure 44. Ear thickness (**a**) and back clinical scores (**b**) are shown for days 0-2. Data point represent the mean of 8 animals ± s.e.m. *=p<0.05 (Dunnett's test against *Il17*c^{+/+} mice). n.d=not detectable.

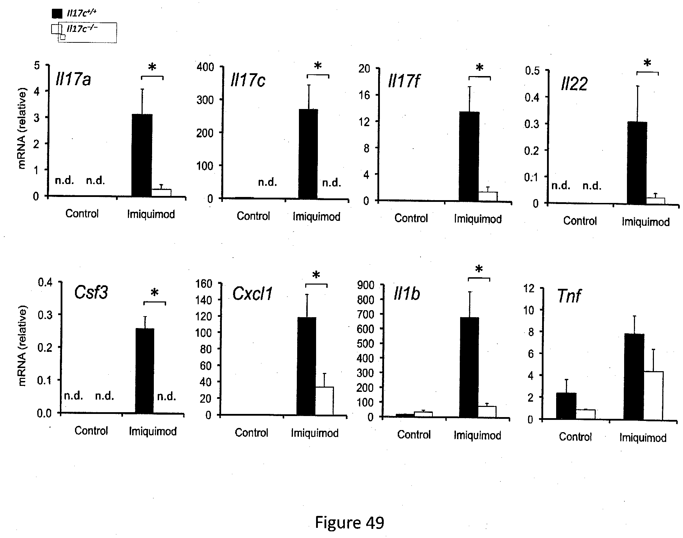

**Figure 49****.** IL-17C deficiency reduces inflammation in a mouse model of psoriasis. *Il17c*^{*+*/*+*} and *Il17c^{-l-}* mice were treated as in Figure 44. qRT-PCR analyses of mRNA for indicated cytokines in back skin derived from *Il17c^{+l+}* (black bar) and *Il17c^{-l-}* (open bar) mice harvested on day 2 of treatment. Expression is shown relative to the housekeeping genes *Rpl19.* Data point represent the mean of 8 animals ± s.e.m. *=p<0.05 (Dunnett's test against *Il17c*^{*+*/*+*} mice). n.d=not detectable.

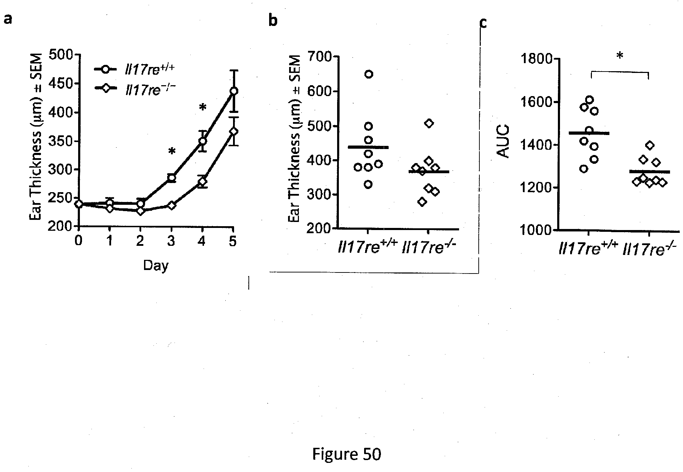

**Figure 50****.** IL-17C pathway is required for disease in a mouse model of psoriasis. *Il17re*^{*+*/*+*} and *Il17re*^{*-*/*-*} mice were treated as in Figure 44. (**a-c**) Ear thickness measurements over the 5-day period (**a**), on day 5 (**b**) and as AUC (**c**). Data points represent each of 8 animals with the mean shown as a line. *=p<0.05 (Dunnett's test against *Il17re*^{*+*/*+*} mice).

### Detailed Description of the Invention

### I. Definitions

The term "IL-17" is used to refer generally to members of the IL-17 family, including IL-17A, IL-17, IL-17B, IL-17C, IL-17D, IL-17E, IL-17F, and IL-17A/F.

A "native sequence IL-17 polypeptide" comprises a polypeptide having the same amino acid sequence as the corresponding IL-17 polypeptide derived from nature. Such native sequence IL-17 polypeptides can be isolated from nature or can be produced by recombinant or synthetic means. The term "native sequence IL-17 polypeptide" specifically encompasses naturally-occurring truncated or secreted forms of the specific IL-17 polypeptide (e.g., an extracellular domain sequence), naturally-occurring variant forms (e.g., alternatively spliced forms) and naturally-occurring allelic variants of the polypeptide. In various embodiments of the invention, the native sequence IL-17 polypeptides disclosed herein are mature or full-length native sequence human IL-17C, IL-17A, IL-17F, etc. polypeptides. In various embodiments of the invention, the native sequence IL-17 polypeptides disclosed herein are mature or full-length native sequence human IL-17C polypeptides comprising the full-length amino acid sequences provided in SEQ ID NO: 29.

The term "native sequence IL-17RA polypeptide" or "native sequence IL-17RA" refers to a polypeptide having the same amino acid sequence as the corresponding IL-17RA polypeptide derived from nature. Such native sequence IL-17RA polypeptides can be isolated from nature or can be produced by recombinant or synthetic means. The term "native sequence IL-17RA polypeptide" specifically encompasses naturally-occurring truncated or secreted forms of the specific IL-17RA polypeptide, naturally-occurring variant forms (e.g., alternatively spliced forms) and naturally-occurring allelic variants of the polypeptide. In various embodiments of the invention, the native sequence IL-17RA polypeptide disclosed herein full-length native sequence human IL-17RA comprising the full-length amino acid provided in SEQ ID NO: 30).

The term "native sequence IL-17RE polypeptide" or "native sequence IL-17RE" refers to a polypeptide having the same amino acid sequence as the corresponding IL-17RE polypeptide derived from nature. Such native sequence IL-17RE polypeptides can be isolated from nature or can be produced by recombinant or synthetic means. The term "native sequence IL-17RE polypeptide" specifically encompasses naturally-occurring truncated or secreted forms of the specific IL-17RE polypeptide, naturally-occurring variant forms (e.g., alternatively spliced forms) and naturally-occurring allelic variants of the polypeptide. In various embodiments of the invention, the native sequence IL-17RE polypeptide disclosed herein full-length native sequence human IL-17RE comprising the full-length amino acid provided in SEQ ID NO: 31).

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide in situ within recombinant cells, since at least one component of the IL-17 polypeptide natural environment will not be present. Ordinarily, however, isolated polypeptide will be prepared by at least one purification step.

The term "antagonist" is used herein in the broadest sense. An IL-17RA and/or IL-17RE "antagonist" is a molecule, which partially or fully bocks, inhibits, neutralizes, prevents or interferes with a biological activity mediated by the IL-17RA and/or IL-17RE receptors. In a preferred embodiment, the "antagonist" partially or fully blocks, inhibits, neutralizes, prevents or interferes with an activity of IL-17C mediated by the IL-17RA and IL-17RE receptors, such as, for example, induction of the host defense pathways in epithelial cells. Antagonists include, without limitation, antagonist antibodies, soluble receptors, peptides and small organic molecules.

The term "agonist" is used herein in the broadest sense. An IL-17RA and/or IL-17RE agonist is any molecule that mimics a biological activity mediated by a native sequence IL-17RA and/or IL-17RE receptor. In a preferred embodiment, the "agonist" mimics an activity of IL-17C mediated by the IL-17RA and IL-17RE receptors, such as, for example, induction of the host defense pathways in epithelial cells. Agonists include, without 1 imitation, agonist antibodies, soluble receptors, peptides and small organic molecules.

Inflammatory bowel disease (IBD)" is used as a collective term for "ulcerative colitis (UC)" and "Crohn's disease (CD)". Although UC and CD are generally considered as two different entities, their common characteristics, such as patchy necrosis of the surface epithelium, focal accumulations of leukocytes adjacent to glandular crypts, and an increased number of intraepithelial lymphocytes (IEL) and certain macrophage subsets, justify their treatment as a single disease group.

"Crohn's disease (CD)" or "ulcerative colitis (UC)" are chronic inflammatory bowel diseases of unknown etiology. Crohn's disease, unlike ulcerative colitis, can affect any part of the bowel. The most prominent feature Crohn's disease is the granular, reddish-purple edmatous thickening of the bowel wall. With the development of inflammation, these granulomas often lose their circumscribed borders and integrate with the surrounding tissue. Diarrhea and obstruction of the bowel are the predominant clinical features. As with ulcerative colitis, the course of Crohn's disease may be continuous or relapsing, mild or severe, but unlike ulcerative colitis, Crohn's disease is not curable by resection of the involved segment of bowel. Most patients with Crohn's disease require surgery at some point, but subsequent relapse is common and continuous medical treatment is usual.

Crohn's disease may involve any part of the alimentary tract from the mouth to the anus, although typically it appears in the ileocolic, small-intestinal or colonic-anorectal regions. Histopathologically, the disease manifests by discontinuous granulomatomas, crypt abscesses, fissures and aphthous ulcers. The inflammatory infiltrate is mixed, consisting of lymphocytes (both T and B cells), plasma cells, macrophages, and neutrophils. There is a disproportionate increase in IgM- and IgG-secreting plasma cells, macrophages and neutrophils.

Anti-inflammatory drugs sulfasalazine and 5-aminosalisylic acid (5-ASA) are useful for treating mildly active colonic Crohn's disease and is commonly perscribed to maintain remission of the disease. Metroidazole and ciprofloxacin are similar in efficacy to sulfasalazine and appear to be particularly useful for treating perianal disease. In more severe cases, corticosteroids are effective in treating active exacerbations and can even maintain remission. Azathioprine and 6-mercaptopurine have also shown success in patients who require chronic administration of cortico steroids. It is also possible that these drugs may play a role in the long-term prophylaxis. Unfortunately, there can be a very long delay (up to six months) before onset of action in some patients.

Antidiarrheal drugs can also provide symptomatic relief in some patients. Nutritional therapy or elemental diet can improve the nutritional status of patients and induce symtomatic improvement of acute disease, but it does not induce sustained clinical remissions. Antibiotics are used in treating secondary small bowel bacterial overgrowth and in treatment of pyogenic complications.

"Ulcerative colitis (UC)" afflicts the large intestine. The course of the disease may be continuous or relapsing, mild or severe. The earliest lesion is an inflammatory infiltration with abscess formation at the base of the crypts of Lieberkühn. Coalescence of these distended and ruptured crypts tends to separate the overlying mucosa from its blood supply, leading to ulceration. Symptoms of the disease include cramping, lower abdominal pain, rectal bleeding, and frequent, loose discharges consisting mainly of blood, pus and mucus with scanty fecal particles. A total colectomy may be required for acute, severe or chronic, unremitting ulcerative colitis.

The clinical features of UC are highly variable, and the onset may be insidious or abrupt, and may include diarrhea, tenesmus and relapsing rectal bleeding. With fulminant involvement of the entire colon, toxic megacolon, a life-threatening emergency, may occur. Extraintestinal manifestations include arthritis, pyoderma gangrenoum, uveitis, and erythema nodosum.

Treatment for UC includes sulfasalazine and related salicylate-containing drugs for mild cases and corticosteroid drugs in severe cases. Topical adminstration of either salicylates or corticosteroids is sometimes effective, particularly when the disease is limited to the distal bowel, and is associated with decreased side effects compared with systemic use. Supportive measures such as administration of iron and antidiarrheal agents are sometimes indicated. Azathioprine, 6-mercaptopurine and methotrexate are sometimes also prescribed for use in refractory corticosteroid-dependent cases.

The term "mammal" for the purposes of treatment refers to any animal classified as a mammal, including but not limited to, humans, rodents, sport, zoo, pet and domestic or farm animals such as dogs, cats, cattle, sheep, pigs, horses, and non-human primates, such as monkeys. Preferably the rodents are mice or rats. Preferably, the mammal is a human, also called herein a patient.

As used herein, "treating" describes the management and care of a mammal for the purpose of combating any of the diseases or conditions targeted in accordance with the present invention, including, without limitation, inflammatory bowel disease or a related condition, and includes administration to prevent the onset of the symptoms or complications, alleviate the symptoms or complications of, or eliminate the targeted diseases or conditions.

For purposes of this invention, beneficial or desired clinical "treatment" results for reducing inflammatory bowel disease (IBD) include, but are not limited to, alleviation of symptoms associated with IBD, diminishment of the extent of the symptoms of IBD, and stabilization (i.e., not worsening) of the symptoms of IBD.

The term "antibody" is used in the broadest sense and specifically covers monoclonal antibodies (including antagonist, e.g. neutralizing antibodies and agonist antibodies), polyclonal antibodies, multi-specific antibodies (e.g., bispecific antibodies), as well as antibody fragments. The monoclonal antibodies specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; Morrison et al., Proc. Natl. Acad. Sci. USA, 81:6851-6855 [1984]). The monoclonal antibodies further include "humanized" antibodies or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a CDR of the recipient are replaced by residues from a CDR of a non-human species (donor antibody) such as mouse, rat or rabbit having the desired specificity, affinity, and capacity. In some instances, Fv FR residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones et al., Nature, 321:522-525 (1986); and Reichmann et al., Nature, 332:323-329 (1988). The humanized antibody includes a PRIMATIZED® antibody wherein the antigen-binding region of the antibody is derived from an antibody produced by immunizing macaque monkeys with the antigen of interest.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (Zapata et al., Protein Eng. 8(10):1057-1062 (1995)); single-chain antibody molecules; and multispecific antibodies formed from antibody fragments.

Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (V_{H}), and the first constant domain of one heavy chain (C_{H1}). Each Fab fragment is monovalent with respect to antigen binding, i.e., it has a single antigen-binding site. Pepsin treatment of an antibody yields a single large F(ab')₂ fragment which roughly corresponds to two disulfide linked Fab fragments having divalent antigen-binding activity and is still capable of cross-linking antigen. Fab' fragments differ from Fab fragments by having additional few residues at the carboxy terminus of the C_{H1} domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The Fc fragment comprises the carboxy-terminal portions of both H chains held together by disulfides. The effector functions of antibodies are determined by sequences in the Fc region, which region is also the part recognized by Fc receptors (FcR) found on certain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv" also abbreviated as "sFv" or "scFv" are antibody fragments that comprise the V_{H} and V_{L} antibody domains connected into a single polypeptide chain. Preferably, the sFv polypeptide further comprises a polypeptide linker between the V_{H} and V_{L} domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994); Borrebaeck 1995, infra.

The term "diabodies" refers to small antibody fragments prepared by constructing sFv fragments (see preceding paragraph) with short linkers (about 5-10 residues) between the V_{H} and V_{L} domains such that inter-chain but not intra-chain pairing of the V domains is achieved, resulting in a bivalent fragment, i.e., fragment having two antigen-binding sites. Bispecific diabodies are heterodimers of two "crossover" sFv fragments in which the V_{H} and V_{L} domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).

The term "multispecific antibody" is used in the broadest sense and specifically covers an antibody comprising a heavy chain variable domain (V_{H}) and a light chain variable domain (V_{L}), where the V_{H}V_{L} unit has polyepitopic specificity (i.e., is capable of binding to two different epitopes on one biological molecule or each epitope on a different biological molecule). Such multispecific antibodies include, but are not limited to, full length antibodies, antibodies having two or more V_{L} and V_{H} domains, antibody fragments such as Fab, Fv, dsFv, scFv, diabodies, bispecific diabodies and triabodies, antibody fragments that have been linked covalently or non-covalently.

A "cross-reactive antibody" is an antibody which recognizes identical or similar epitopes on more than one antigen. Thus, the cross-reactive antibodies of the present invention recognize identical or similar epitopes present on both IL-17RA and IL-17RE. In a particular embodiment, the cross-reactive antibody uses the same or essentially the same paratope to bind to both IL-17RA and IL-17RE. Preferably, the cross-reactive antibodies herein also block both IL-17RA and IL-17RE function (activity).

Autoimmune diseases include, for example, Acquired Immunodeficiency Syndrome (AIDS, which is a viral disease with an autoimmune component), alopecia areata, ankylosing spondylitis, antiphospholipid syndrome, autoimmune Addison's disease, autoimmune hemolytic anemia, autoimmune hepatitis, autoimmune inner ear disease (AIED), autoimmune lymphoproliferative syndrome (ALPS), autoimmune thrombocytopenic purpura (ATP), Behcet's disease, cardiomyopathy, celiac sprue-dermatitis hepetiformis; chronic fatigue immune dysfunction syndrome (CFIDS), chronic inflammatory demyelinating polyneuropathy (CIPD), cicatricial pemphigold, cold agglutinin disease, crest syndrome, Crohn's disease, Degos' disease, dermatomyositis-juvenile, discoid lupus, essential mixed cryoglobulinemia, fibromyalgia-fibromyositis, Graves' disease, Guillain-Barré syndrome, Hashimoto's thyroiditis, idiopathic pulmonary fibrosis, idiopathic thrombocytopenia purpura (ITP), IgA nephropathy, insulin-dependent diabetes mellitus, juvenile chronic arthritis (Still's disease), juvenile rheumatoid arthritis, Ménière's disease, mixed connective tissue disease, multiple sclerosis, myasthenia gravis, pernacious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomena, Reiter's syndrome, rheumatic fever, rheumatoid arthritis, sarcoidosis, scleroderma (progressive systemic sclerosis (PSS), also known as systemic sclerosis (SS)), Sjögren's syndrome, stiff-man syndrome, systemic lupus erythematosus, Takayasu arteritis, temporal arteritis/giant cell arteritis, ulcerative colitis, uveitis, vitiligo and Wegener's granulomatosis.

Inflammatory disorders include, for example, chronic and acute inflammatory disorders. Examples of inflammatory disorders include Alzheimer's disease, asthma, atopic allergy, allergy, atherosclerosis, bronchial asthma, eczema, glomerulonephritis, graft vs. host disease, hemolytic anemias, osteoarthritis, sepsis, stroke, transplantation of tissue and organs, vasculitis, diabetic retinopathy and ventilator induced lung injury.

"Chronic" administration refers to administration of the agent(s) in a continuous mode as opposed to an acute mode, so as to maintain the desired effect for an extended period of time.

"Intermittent" administration is treatment that is not consecutively done without interruption, but rather is cyclic in nature.

Administration "in combination with" one or more further therapeutic agents includes simultaneous (concurrent) and consecutive administration in any order.

### II. Detailed Description

Mammalian cutaneous and mucosal epithelial cells are in direct contact with environmental microbial organisms, forming the first line of defense against potential invading pathogens ⁵¹. The immune system, in particular tissue imbedded dendritic cells (DCs), sense these microbes through various pattern recognition receptors (PRRs), such as Toll-like receptors (TLRs), and induce proper immune responses, including enhancement of epithelial innate immunity, to control the infection⁴³. On the other hand, although epithelial cells also express TLRs, it is unclear whether TLR activation on epithelial cells directly promotes the epithelial innate defense mechanisms⁵²⁻⁵⁴.

As shown herein, IL-17C is an IL-17 family member that is selectively induced in epithelia upon sensing bacterial challenges and inflammatory stimuli. The data presented herein provide the first evidence that IL-17C is as an epithelial cell derived cytokine that is regulated by Toll-like receptors (TLR) agonists to promote host defense responses. In addition, the results presented herein demonstrate IL-17C binding to a unique and novel heterodimeric receptor complex composed of the IL-17RA and IL-17RE subunits, which are predominantly expressed on epithelial cells. The results presented herein further reveal that IL-17C utilizes a novel autocrine mechanism to induce innate immune pathways within epithelial cells. In particular, the results show that IL-17C stimulates epithelial inflammatory responses including expression of pro-inflammatory cytokines, chemokines, and anti-microbial peptides, similar to those induced by IL-17A and IL-17F. Intriguingly, these pathways are similar to IL-17A mediated responses, suggesting overlapping functions of the two cytokines in host defense mechanisms. However, IL-17C is produced by distinct cellular sources, such as epithelial cells, in contrast to IL-17A, which is mainly produced by leukocytes, especially T_{H}17 cells. Furthermore, the results presented herein demonstrate that similar to IL-17A and IL-22, IL-17C has both protective and pathogenic properties. Loss of IL-17C signaling augmented disease in the DSS colitis model, while it attenuated disease in a mouse model of psoriasis, suggesting that IL-17C modulates differing responses in epithelial tissue, which, depending on the type of insult, can be either beneficial or detrimental to the host. Thus, IL-17C is an essential autocrine cytokine regulating innate epithelial immune responses.

The invention is based, at least in part, on experimental findings demonstrating that (1) IL-17C uses IL-17RA and IL-17RE; (2) IL-17C induces host defense pathways in epithelial cells, and (3) IL-17C is secreted by epithelial cells in response to bacterial or cytokine stimuli.

Due to the complexity of inflammatory bowel disease and related conditions, depending on the stage and nature of the disease or condition and the timing of administration, in certain embodiments, IL-17RA and/or IL-17RE agonists may also be useful in the methods of the present invention.

### 1. Therapeutic uses

Diseases or disorders related to IL-17C signaling include autoimmune diseases or inflammatory diseases or disorders, including but not limited to inflammatory bowel disease (IBD), psoriasis, and asthma.

The anti-IL-17RA and/or anti-IL-17RE antibodies or IL-17C bispecific or cross-reactive antibodies of the present invention can be used in the management of inflammatory conditions, such as gastrointestinal inflammation, inflammatory bowel disease, Crohn's disease, ulcerative colitis, and psoriasis. The anti-IL-17RA and/or anti-IL-17RE antibodies or IL-17C bispecific or cross-reactive antibodies of the present invention are also useful for the treatment of asthma and other disorders associated with airway hyperreactivity, typically characterized by episodes of coughs, wheezing, chest tightness, and/or breathing problems.

The present invention concerns the treatment of gastrointestinal inflammation, psoriasis and asthma by administration of an IL-17RA and/or IL-17RE antagonist or an IL-17C antagonist. An IL-17C antagonist may be any molecule that interferes with the function of IL-17C, or blocks or neutralizes a relevant activity of IL-17C, by whatever means, depending on the indication being treated. It may prevent the interaction between IL-17C and IL-17RA and/or IL-17RE. For example, it may block IL-17C from binding and/or signaling through IL-17RA and/or IL-17RE. Such agents accomplish this effect in various ways. For instance, the class of antagonists that neutralize an IL-17C activity will bind to IL-17C, or a receptor of IL-17C, IL-17RA and/or IL-17RE, with sufficient affinity and specificity to interfere with IL-17C.

### 2. Administration and formulations

The IL-17RA and/or IL-17RE or IL-17C antagonist may be administered by any suitable route, including a parenteral route of administration such as, but not limited to, intravenous (IV), intramuscular (IM), subcutaneous (SC), and intraperitoneal (IP), as well as transdermal, buccal, sublingual, intrarectal, intranasal, and inhalant routes. IV, IM, SC, and IP administration may be by bolus or infusion, and in the case of SC, may also be by slow-release implantable device, including, but not limited to pumps, slow-release formulations, and mechanical devices. Preferably, administration is systemic.

One specifically preferred method for administration of IL-17RA and/or IL-17RE or IL-17C antagonist is by subcutaneous infusion, particularly using a metered infusion device, such as a pump. Such pump can be reusable or disposable, and implantable or externally mountable. Medication infusion pumps that are usefully employed for this purpose include, for example, the pumps disclosed in U.S. Pat. Nos. 5,637,095; 5,569,186; and 5, 527,307. The compositions can be administered continaully from such devices, or intermittently.

Therapeutic formulations of IL-17RA and/or IL-17RE or IL-17C antagonists suitable for storage include mixtures of the antagonist having the desired degree of purity with pharmaceutically acceptable carriers, excipients, or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEENT™, PLURONICS™ or polyethylene glycol (PEG).

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences, supra.

The IL-17RA and/or IL-17RE antagonists, such as anti- IL-17RA and/or anti-IL-17RE antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); U.S. Pat. Nos. 4,485,045 and 4,544,545; and WO97/38731 published Oct. 23, 1997. Liposomes with enhanced circulation time are disclosed in U.S. Pat. No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem., 257: 286-288 (1982) via a disulfide interchange reaction.

Sustained-release preparations may be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and y ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

Any of the specific antagonists can be joined to a carrier protein to increase the serum half-life of the therapeutic antagonist. For example, a soluble immunoglobulin chimera, such as described herein, can be obtained for each specific IL-17RA and/or IL-17RE antagonist or antagonistic portion thereof, as described in U.S. Pat. No. 5,116,964. The immunoglobulin chimera are easily purified through IgG-binding protein A-Sepharose chromatography. The chimera have the ability to form an immunoglobulin-like dimer with the concomitant higher avidity and serum half-life.

The formulations to be used for in vivo administration must be sterile. This is readily accomplished by filtration through sterile filtration membranes.

The formulation can also contain more than one active compound as necessary for the particular indication being treated, preferably those with complementary activities that do not adversely affect each other. Alternatively, or in addition, the composition can comprise an agent that enhances its function, such as, for example, a cytotoxic agent, cytokine, chemotherapeutic agent, or growth-inhibitory agent. Such molecules are suitably present in combination in amounts that are effective for the purpose intended.

In one embodiment, the active compounds are administered in combination therapy, *i.e.,* combined with other agents, *e.g*., therapeutic agents, that are useful for treating pathological conditions or disorders, such as autoimmune disorders and inflammatory diseases. The term "in combination" in this context means that the agents are given substantially contemporaneously, either simultaneously or sequentially. If given sequentially, at the onset of administration of the second compound, the first of the two compounds is preferably still detectable at effective concentrations at the site of treatment.

For example, the combination therapy can include one or more antagonists of the invention coformulated with, and/or coadministered with, one or more additional therapeutic agents, *e.g.,* one or more cytokine and growth factor inhibitors, immunosuppressants, anti-inflammatory agents, metabolic inhibitors, enzyme inhibitors, and/or cytotoxic or cytostatic agents, as described in more detail below. Furthermore, one or more antibodies described herein may be used in combination with two or more of the therapeutic agents described herein. Such combination therapies may advantageously utilize lower dosages of the administered therapeutic agents, thus avoiding possible toxicities or complications associated with the various monotherapies.

Preferred therapeutic agents used in combination with an antagonist of the invention are those agents that interfere at different stages in an inflammatory response. In one embodiment, one or more antagonists described herein may be coformulated with, and/or coadministered with, one or more additional agents such as other cytokine or growth factor antagonists (*e.g*., soluble receptors, peptide inhibitors, small molecules, ligand fusions); or antibodies or antigen binding fragments thereof that bind to other targets (*e.g*., antibodies that bind to other cytokines or growth factors, their receptors, or other cell surface molecules); and anti-inflammatory cytokines or agonists thereof. Nonlimiting examples of the agents that can be used in combination with the antibodies described herein, include, but are not limited to, antagonists of one or more interleukins (ILs) or their receptors, *e.g*., antagonists of IL-1, IL-2, IL-6, IL-7, IL-8, IL-12, IL-13, IL-15, IL-16, IL-17 A, IL-18, IL-21 and IL-22; antagonists of cytokines or growth factors or their receptors, such as tumor necrosis factor (TNF), IFN-γ, LT, EMAP-II, GM-CSF, FGF and PDGF. Antibodies of the invention can also be combined with inhibitors of, *e.g*., antibodies to, cell surface molecules such as CD2, CD3, CD4, CD8, CD20 (*e.g.,* the CD20 inhibitor rituximab (RITUXAN^{®})), CD25, CD28, CD30, CD40, CD45, CD69, CD80 (B7.1), CD86 (B7.2), CD90, or their ligands, including CD154 (gp39 or CD4OL), or LFA-1/ICAM-1 and VLA-4/VCAM-1 (Yusuf-Makagiansar et al. (2002) Med. Res. Rev. 22:146-67). Preferred antagonists that can be used in combination with the antagonists described herein include antagonists of IFN-γ, IL-1β, TNFα, IL-17A, and IL-22.

Examples of TNF antagonists include chimeric, humanized, human or *in vitro-*generated antibodies (or antigen binding fragments thereof) to TNF (*e.g*., human TNFα), such as (HUMIRA™, D2E7, human TNFα antibody), CDP-571/CDP-870/BAY-10-3356 (humanized anti-TNFα antibody; Celltech/Pharmacia), cA2 (chimeric anti-TNFα antibody; REMICADE^{®}, Centocor); anti-TNF antibody fragments (*e.g*., CPD870); soluble fragments of the TNF receptors, *e.g.,* p55 or p75 human TNF receptors or derivatives thereof, *e.g.,* 75 kdTNFR-IgG (75 kD TNF receptor-IgG fusion protein, ENBREL™; Immunex), p55 kdTNFR-IgG (55 kD TNF receptor-IgG fusion protein (LENERCEPT^{®})); enzyme antagonists, *e.g*., TNFα converting enzyme (TACE) inhibitors (*e.g*., an alpha-sulfonyl hydroxamic acid derivative, and N-hydroxyformamide TACE inhibitor GW 3333, -005, or -022); and TNF-bp/s-TNFR (soluble TNF binding protein). Preferred TNF antagonists are soluble fragments of the TNF receptors, *e.g.,* p55 or p75 human TNF receptors or derivatives thereof, *e.g.,* 75 kdTNFR-IgG, and TNFα converting enzyme (TACE) inhibitors.

The formulation herein may also contain more than one active compound as necessary for the particular indication being treated, preferably those with complementary activities that do not adversely affect each other. Also, such active compound can be administered separately to the mammal being treated.

Nonlimiting examples of agents for treating or preventing inflammatory bowel disease (*e.g.,* Crohn's disease, ulcerative colitis) with which an antibody of the invention can be combined include the following: budenoside; epidermal growth factor; corticosteroids; cyclosporine; sulfasalazine; aminosalicylates; 6-mercaptopurine; azathioprine; metronidazole; lipoxygenase inhibitors; mesalamine; olsalazine; balsalazide; antioxidants; thromboxane inhibitors; IFN-γ antagonists, *e.g*., an anti-IFN-γ antibody, an IFN-γ receptor antibody, or a native IFN-γ receptor; IL-1 receptor antagonists; anti-IL-1 monoclonal antibodies; anti-IL-6 monoclonal antibodies; growth factors; elastase inhibitors; pyridinyl-imidazole compounds; TNF-α antagonists, *e.g*., an anti-TNF-α antibody, a TNF-α receptor antibody, or a native TNF-α receptor; IL-4, IL-10, IL-13 and/or TGFβ cytokines or agonists thereof (*e.g*., agonist antibodies); IL-11; glucuronide- or dextran-conjugated prodrugs of prednisolone, dexamethasone or budesonide; ICAM-1 antisense phosphorothioate oligodeoxynucleotides (ISIS 2302; Isis Pharmaceuticals, Inc.); soluble complement receptor 1 (TP10; T Cell Sciences, Inc.); slow-release mesalazine; methotrexate; antagonists of platelet activating factor (PAF); ciprofloxacin; and lignocaine. For the treatment of Crohn's disease, the antagonists of the present invention can be administered in combination with other treatment regimens known for the treatment of these conditions. For example, the IL-17RA and/or IL-17RE antagonists herein can be administered in combination with Remicade® (Infliximab, Centocor), or Enbrel® (Etanercept, Wyeth-Ayerst). The present invention also includes bispecific antibodies targeting these diseases. For example, a bispecific antibody could include an anti-TNF specificity combined with the IL-17RA- or IL-17RE-binding ability of the antibodies herein.

Nonlimiting examples of agents for treating or preventing psoriasis with which an antibody of the invention can be combined include the following: corticosteroids; vitamin D₃ and analogs thereof; retinoiods (*e.g*., soriatane); methotrexate; cyclosporine, 6-thioguanine; Accutane; hydrea; hydroxyurea; sulfasalazine; mycophenolate mofetil; azathioprine; tacrolimus; fumaric acid esters; biologics such as Amevive, Enbrel, Humira, Raptiva and Remicade, Ustekinmab, and XP-828L; phototherapy; and photochemotherapy (*e.g*., psoralen and ultraviolet phototherapy combined).

For the treatment of asthma, it might be particularly advantageous to use the antagonists herein in combination with anti-IgE antibodies, in particular rhuMAb-E25 (Xolair™), or with second-generation antibody molecule rhuMAb-E26 (Genentech, Inc.). The rhuMAb-E25 antibody is a recombinant humanized anti-IgE monoclonal antibody that was developed to interfere early in the allergic process. Combination use also includes the possibility of administering the two antibodies, for example, in a single pharmaceutical formulation, or using a bispecific antibody, with anti-IL-17RA or anti-IL-17RE and anti-IgE specificities. In another preferred embodiment, the IL-17RA and/or IL-17RE antagonists herein are administered in combination with inhaled corticosteroids, such as beclomethasone diproprionate (BDP) treatment. Other non-limiting examples of therapeutic agents for asthma with which an antagonist of the present invention may be combined include the following: albuterol, salmeterol/fluticasone, montelukast sodium, fluticasone propionate, budesonide, prednisone, salmeterol xinafoate, levalbuterol hcl, albuterol sulfate/ipratropium, prednisolone sodium phosphate, triamcinolone acetonide, beclomethasone dipropionate, ipratropium bromide, azithromycin, pirbuterol acetate, prednisolone, theophylline anhydrous, methylprednisolone sodium succinate, clarithromycin, zafirlukast, formoterol fumarate, influenza virus vaccine, methylprednisolone, amoxicillin trihydrate, flunisolide, allergy injection, cromolyn sodium, fexofenadine hydrochloride, flunisolide/menthol, amoxicillin/clavulanate, levofloxacin, inhaler assist device, guaifenesin, dexamethasone sodium phosphate, moxifloxacin hcl, doxycycline hyclate, guaifenesin/d-methorphan, p-ephedrine/cod/chlorphenir, gatifloxacin, cetirizine hydrochloride, mometasone furoate, salmeterol xinafoate, benzonatate, cephalexin, pe/hydrocodone/chlorphenir, cetirizine hcl/pseudoephed, phenylephrine/cod/promethazine, codeine/promethazine, cefprozil, dexamethasone, guaifenesin/pseudoephedrine, chlorpheniramine/hydrocodone, nedocromil sodium, terbutaline sulfate, epinephrine, methylprednisolone, metaproterenol sulfate.

Such additional molecules are suitably present or administered in combination in amounts that are effective for the purpose intended, typically less than what is used if they are administered alone without the antagonist to IL-17RA and/or IL-17RE. If they are formulated together, they may be formulated in the amounts determined according to, for example, the type of indication, the subject, the age and body weight of the subject, current clinical status, administration time, dosage form, administration method, etc. For instance, a concomitant drug is used preferably in a proportion of about 0.0001 to 10,000 weight parts relative to one weight part of the antagonist to IL-17C, IL-17RA, and/or IL-17RE herein.

For the prevention or treatment of disease, the appropriate dosage of the IL-17RA and/or IL-17RE antagonist will depend on the IL-17RA and/or IL-17RE antagonist employed, the type of disease to be treated, the severity and course of the disease, whether the antibody is administered for preventive or therapeutic purposes, previous therapy, the patient's clinical history and response to the antibody, and the discretion of the attending physician. Typically the clinician will administer the IL-17RA and/or IL-17RE antagonist until a dosage is reached that achieves the desired result.

The IL-17RA and/or IL-17RE antagonist is suitably administered to the patient at one time or over a series of treatments. The dosage ranges presented herein are not intended to limit the scope of the invention in any way. A "therapeutically effective" amount for purposes herein depends on the type and severity of the disease and is determined by the above factors, but is generally about 0.01 to 100 mg/kg body weight/day. The preferred dose is about 0.1-50 mg/kg/day, more preferably about 0.1 to 25 mg/kg/day. More preferred still, when the IL-17RA and/or IL-17RE antagonist is administered daily, the intravenous or intramuscular dose for a human is about 0.3 to 10 mg/kg of body weight per day, more preferably, about 0.5 to 5 mg/kg. For subcutaneous administration, the dose is preferably greater than the therapeutically-equivalent dose given intravenously or intramuscularly. Preferably, the daily subcutaneous dose for a human is about 0.3 to 20 mg/kg, more preferably about 0.5 to 5 mg/kg for both indications. The progress of this therapy is easily monitored by conventional techniques and assays.

### 3. Articles of manufacture and kits

The invention also provides kits for the reduction of gastrointestinal inflammation, the treatment of inflammatory bowel disease, ulcerative colitis, psoriasis, and asthma. The kits of the invention comprise one or more containers of at least one antagonist of IL-17RA and/or IL-17RE, preferably antibody, in combination with a set of instructions, generally written instructions, relating to the use and dosage of IL-17RA and/or IL-17RE antagonist for the reduction of gastrointestinal inflammation, treatment of psoriasis or treatment of asthma. The instructions included with the kit generally include information as to dosage, dosing schedule, and route of administration for the treatment of the target disease, such as inflammatory bowel disease, psoriasis, or asthma. The containers of IL-17RA and/or IL-17RE antagonist may be unit doses, bulk packages (e.g., multi-dose packages), or sub-unit doses.

The article of manufacture comprises a container and a label or package insert on or associated with the container. Suitable containers include, for example, bottles, vials, syringes, etc. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). At least one active agent in the composition is an IL-17RA and/or IL-17RE antagonist of the invention. The label or package insert indicates that the composition is used for treating the particular condition. The label or package insert will further comprise instructions for administering the antibody composition to the patient. Articles of manufacture and kits comprising combinatorial therapies described herein are also contemplated.

Package insert refers to instructions customarily included in commercial packages of therapeutic products that contain information about the indications, usage, dosage, administration, contraindications and/or warnings concerning the use of such therapeutic products

Additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### 4. Preparation of antibodies

### Monoclonal antibodies

Monoclonal antibodies may be made using the hybridoma method first described by Kohler et al., Nature, 256:495 (1975), or may be made by recombinant DNA methods (U.S. Pat. No. 4,816,567). In the hybridoma method, a mouse or other appropriate host animal, such as a hamster or macaque monkey, is immunized as hereinabove described to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, Calif. USA, and SP-2 or X63-Ag8-653 cells available from the American Type Culture Collection, Rockville, Md. USA. Human myeloma and mouse-human heteromyeloma cell lines also have been described for the production of human monoclonal antibodies (Kozbor, J. Immunol., 133:3001 (1984); Brodeur et al, Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA).

After hybridoma cells are identified that produce antibodies of the desired specificity, affinity, and/or activity, the clones may be subloned by limiting dilution procedures and grown by standard methods (Goding, MonoclonalAntibodies: Principles and Practice, pp.59-103 (Academic Press, 1986)). Suitable culture media for this purpose include, for example, D-MEM or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. Recombinant production of antibodies will be described in more detail below.

In a further embodiment, antibodies or antibody fragments can be isolated from antibody phage libraries generated using the techniques described in McCafferty et al., Nature, 348:552-554 (1990).

Clackson et al., Nature, 352:624-628 (1991) and Marks etal., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and in vivo recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy- and light-chain constant domains in place of the homologous murine sequences (U.S. Pat. No. 4,816,567; Morrison, et al., Proc. Natl. Acad. Sci. USA, 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### Humanized and human antibodies

A humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Pat. No. 4,816,567) wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987)). Another method uses a particular framework derived from the consensus sequence of all human antibodies of a particular subgroup of light or heavy chains. The same framework may be used for several different humanized antibodies (Carter et al., Proc. Natl. Acad Sci. USA, 89:4285 (1992); Presta et al., J. Immnol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the CDR residues are directly and most substantially involved in influencing antigen binding.

Alternatively, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J.sub.H) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al, Proc. Natl. Acad. Sci. USA, 90:2551 (1993); Jakobovits et al., Nature, 362:255-258 (1993); Bruggermann et al., Year in Immuno., 7:33 (1993); and Duchosal et al. Nature 355:258 (1992). Human antibodies can also be derived from phage-display libraries (Hoogenboom et al, J. Mol. Biol., 227:381 (1991); Marks et al, J. MoL Biol., 222:581-597 (1991); Vaughan et al. Nature Biotech 14:309 (1996)). Generation of human antibodies from antibody phage display libraries is further discussed in WO 2010/114859.

### Antibody Fragments

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992) and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from E. coli and chemically coupled to form F(ab')₂ fragments (Carter et al., Bio/Technology 10:163-167 (1992)). In another embodiment as described in the example below, the F(ab')₂ is formed using the leucine zipper GCN4 to promote assembly of the F(ab')₂ molecule. According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185. The antibody fragment may also be a "linear antibody", e.g., as described in U.S. Patent 5,641,870 for example. Such linear antibody fragments may be monospecific or bispecific.

### Multispecific Antibodies

Multispecific antibodies have binding specificities for at least two different epitopes, where the epitopes are usually from different antigens. While such molecules normally will only bind two different epitopes (i.e. bispecific antibodies, BsAbs), antibodies with additional specificities such as trispecific antibodies are encompassed by this expression when used herein. Methods for making bispecific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991). According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach described in WO96/27011, the interface between a pair of antibody molecules can be engineered to maximize the percentage of heterodimers which are recovered from recombinant cell culture. The preferred interface comprises at least a part of the CH3 domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Pat. No. 4,676,980), and for treatment of HIV infection (WO 91/00360, WO92/200373). Heteroconjugate antibodies may be made using any convenient cross-linking methods. Suitable cross-linking agents are well known in the art, and are disclosed in U.S. Pat. No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab'-SH fragments can also be directly recovered from E. coli, and can be chemically coupled to form bispecific antibodies. Shalaby et al., J. Exp. Med., 175: 217-225 (1992) describe the production of a fully humanized bispecific antibody F(ab')₂ molecule. Each Fab' fragment was separately secreted from E. coli and subjected to directed chemical coupling in vitro to form the bispecific antibody.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Nati. Acad. Sci. USA, 90:6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber et al, J. Immunol, 152:5368 (1994).

Antibodies with more than two valencies are contemplated. For example, trispecific antibodies can be prepared. Tuft et al. J. Immunol. 147: 60 (1991).

### Effector Function Engineering

It may be desirable to modify the antibody of the invention with respect to effector function, so as to enhance the effectiveness of the antibody. For example cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumor activity may also be prepared using heterobifunctonal cross-linkers as described in Wolff et al. Cancer Research 53:2560-2565 (1993). Alternatively, an antibody can be engineered which has dual Fc regions and may thereby have enhanced complement lysis and ADCC capabilities. See Stevenson et al Anti-Cancer Drug Design 3:219-230 (1989).

### Antibody-Salvage Receptor Binding Epitope Fusions.

In certain embodiments of the invention, it may be desirable to use an antibody fragment, rather than an intact antibody. In this case, it may be desirable to modify the antibody fragment in order to increase its serum half life. This may be achieved, for example, by incorporation of a salvage receptor binding epitope into the antibody fragment (e.g. by mutation of the appropriate region in the antibody fragment or by incorporating the epitope into a peptide tag that is then fused to the antibody fragment at either end or in the middle, e.g., by DNA or peptide synthesis).

The salvage receptor binding epitope preferably constitutes a region wherein any one or more amino acid residues from one or two loops of a Fc domain are transferred to an analogous position of the antibody fragment. Even more preferably, three or more residues from one or two loops of the Fc domain are transferred. Still more preferred, the epitope is taken from the CH2 domain of the Fc region (e.g., of an IgG) and transferred to the CH1, CH3, or V.sub.H region, or more than one such region, of the antibody. Alternatively, the epitope is taken from the CH2 domain of the Fc region and transferred to the CL region or VL region, or both, of the antibody fragment.

### Other Covalent Modifications of Antibodies

Covalent modifications of antibodies are included within the scope of this invention. They may be made by chemical synthesis or by enzymatic or chemical cleavage of the antibody, if applicable. Other types of covalent modifications of the antibody are introduced into the molecule by reacting targeted amino acid residues of the antibody with an organic derivatizing agent that is capable of reacting with selected side chains or the N- or C-terminal residues. Examples of covalent modifications are described in U.S. Pat. No. 5,534,615, specifically incorporated herein by reference. A preferred type of covalent modification of the antibody comprises linking the antibody to one of a variety of nonproteinaceous polymers, e.g., polyethylene glycol, polypropylene glycol, or polyoxyalkylenes, in the manner set forth in U.S. Pat. Nos. 4,640,835; 4,496,689; 4,301,144; 4,670,417; 4,791,192 or 4,179,337.

### 5. Design and Generation of Other Therapeutics

In accordance with the present invention and based on the activity of the antibodies that are produced and characterized herein with respect to IL-17C and/or the heterodimeric IL-17RA/IL-17RE complex, the design of other therapeutic modalities beyond antibody moieties is facilitated. Such modalities include, without limitation, advanced antibody therapeutics, such as bispecific antibodies, immunotoxins, and radiolabeled therapeutics, generation of peptide therapeutics, gene therapies, particularly intrabodies, antisense therapeutics, and small molecules.

In connection with immunotoxins, antibodies can be modified to act as immunotoxins utilizing techniques that are well known in the art. *See e.g.,* Vitetta Immunol Today 14:252 (1993). *See* also U.S. Patent No. 5,194,594. In connection with the preparation of radiolabeled antibodies, such modified antibodies can also be readily prepared utilizing techniques that are well known in the art. *See e.g.,* Junghans et al. in Cancer Chemotherapy and Biotherapy 655-686 (2d edition, Chafner and Longo, eds., Lippincott Raven (1996)). *See* also U.S. Patent Nos. 4,681,581, 4,735,210, 5,101,827, 5,102,990 (RE 35,500), 5,648,471, and 5,697,902. Each of immunotoxins and radiolabeled molecules would be likely to kill cells expressing IL-17C and/or the heterodimeric IL-17RA/IL-17RE complex.

In connection with the generation of therapeutic peptides, through the utilization of structural information related to IL-17C and/or the heterodimeric IL-17RA/IL-17E complex and antibodies thereto, such as the antibodies of the invention or screening of peptide libraries, therapeutic peptides can be generated that are directed against IL-17C and/or the heterodimeric IL-17RA/IL-17RE complex. Design and screening of peptide therapeutics is discussed in connection with Houghten et al. Biotechniques 13:412-421 (1992), Houghten PNAS USA 82:5131-5135 (1985), Pinalla et al. Biotechniques 13:901-905 (1992), Blake and Litzi-Davis BioConjugate Chem. 3:510-513 (1992). Immunotoxins and radiolabeled molecules can also be prepared, and in a similar manner, in connection with peptidic moieties as discussed above in connection with antibodies. Assuming that the IL-17C and/or the heterodimeric IL-17RA/IL-17RE complex molecule (or a form, such as a splice variant or alternate form) is functionally active in a disease process, it will also be possible to design gene and antisense therapeutics thereto through conventional techniques. Such modalities can be utilized for modulating the function of IL-17C and/or the heterodimeric IL-17RA/IL-17RE complex. In connection therewith the antibodies of the present invention facilitate design and use of functional assays related thereto. A design and strategy for antisense therapeutics is discussed in detail in International Patent Application No. WO 94/29444. Design and strategies for gene therapy are well known. However, in particular, the use of gene therapeutic techniques involving intrabodies could prove to be particularly advantageous. *See e.g.,* Chen et al. Human Gene Therapy 5:595-601 (1994) and Marasco Gene Therapy 4:11-15 (1997). General design of and considerations related to gene therapeutics is also discussed in International Patent Application No. WO 97/38137.

Knowledge gleaned from the structure of the IL-17C and/or the heterodimeric IL-17RA/IL-17RE complex molecule and its interactions with other molecules in accordance with the present invention, such as the antibodies of the invention, and others can be utilized to rationally design additional therapeutic modalities. In this regard, rational drug design techniques such as X-ray crystallography, computer-aided (or assisted) molecular modeling (CAMM), quantitative or qualitative structure-activity relationship (QSAR), and similar technologies can be utilized to focus drug discovery efforts. Rational design allows prediction of protein or synthetic structures which can interact with the molecule or specific forms thereof which can be used to modify or modulate the activity of IL-17C, and/or the heterodimeric IL-17RA/IL-17RE complex. Such structures can be synthesized chemically or expressed in biological systems. This approach has been reviewed in Capsey et al. Genetically Engineered Human Therapeutic Drugs (Stockton Press, NY (1988)). Further, combinatorial libraries can be designed and synthesized and used in screening programs, such as high throughput screening efforts.

### 6. Screening Assays

The invention also provides methods (also referred to herein as "screening assays") for identifying modulators, *i.e.,* candidate or test compounds or agents (*e.g.,* peptides, peptidomimetics, small molecules or other drugs) that modulate, block, inhibit, reduce, antagonize, neutralize or otherwise interfere with binding of IL-17C and/or the heterodimeric IL-17RA/IL-17RE complex to their innate receptor, or candidate or test compounds or agents that modulate, block, inhibit, reduce, antagonize, neutralize or otherwise interfere with the signaling function of IL-17C and/or the heterodimeric IL-17RA/IL-17RE complex. Also provided are methods of identifying compounds useful to treat disorders associated with IL-17C and/or heterodimeric IL-17RA/IL-17E complex signaling. The invention also includes compounds identified in the screening assays described herein.

For example, antibodies useful in the present invention are those that neutralize the activity of IL-17C, in one aspect, by binding to or signaling through IL-17RA and/or IL-17RE. In another aspect, antibodies useful in the present invention are bispecific or cross-reactive antibodies specifically binding to IL-17C and a further cytokine. Thus, for example, the neutralizing IL-17RA and/or IL-17RE antibodies or anti-IL-17C bispecific or cross-reactive antibodies of the present invention can be identified by incubating a candidate antibody with IL-17RA and/or IL-17RE or IL-17C and monitoring binding and neutralization of a biological activity of IL-17C. The binding assay may be performed with purified IL-17RA and/or IL-17RE or IL-17C polypeptide(s), or with cells naturally expressing, or transfected to express, IL-17RA and/or IL-17RE or IL-17C polypeptide(s). In one embodiment, the binding assay is a competitive binding assay, where the ability of a candidate antibody to compete with a known IL-17RA and/or IL-17RE or anti-IL-17C antibody for IL-17C binding is evaluated. The assay may be performed in various formats, including the ELISA format.

To screen for antibodies which bind to an epitope on IL-17RA and/or IL-17RE or IL-17C bound by an antibody of interest, a routine cross-blocking assay such as that described in Antibodies, A Laboratory Manual, Cold Spring Harbor Laboratory, Ed Harlow and David Lane (1988), can be performed. Alternatively, or additionally, epitope mapping can be performed by methods known in the art. For example, the IL-17RA and/or IL-17RE epitope(s) or IL-17C epitope bound by a monoclonal antibody of the present invention can be determined by competitive binding analysis as described in Fendly et al. Cancer Research 50:1550-1558 (1990). Cross-blocking studies can be done by direct fluorescence on intact cells using the PANDEXTM Screen Machine to quantitate fluorescence. In this method the monoclonal antibody is conjugated with fluorescein isothiocyanate (FITC), using established procedures (Wofsy et al. Selected Methods in Cellular Immunology, p. 287, Mishel and Schiigi (eds.) San Francisco: W.J. Freeman Co. (1980)). IL-17RA and/or IL-17RE or IL-17C expressing cells in suspension and purified monoclonal antibodies are added to the PANDEXTM plate wells and incubated, and fluorescence is quantitated by the PANDEXTM. Monoclonal antibodies are considered to share an epitope if each blocks binding of the other by 50% or greater in comparison to an irrelevant monoclonal antibody control.

Anti-IL-17RA and/or IL-17RE or anti- IL-17C antibodies useful in the present invention can also be identified using combinatorial libraries to screen for synthetic antibody clones with the desired activity or activities. Such methods are well known in the art. Briefly, synthetic antibody clones are selected by screening phage libraries containing phage that display various fragments (e.g. Fab, F(ab')₂, etc.) of antibody variable region (Fv) fused to phage coat proteins. Such phage libraries are panned by affinity chromatography against the desired antigen. Clones expressing Fv fragments capable of binding to the desired antigen are adsorbed to the antigen and thus separated from the non-binding clones in the library. The binding clones are then eluted from the antigen and can be further enriched by additional cycles of antigen adsorption/elution. Suitable anti-IL-17RA and/or IL-17RE antibodies or anti-IL-17C antibodies for use in the present invention can be obtained by designing a suitable antigen screening procedure to select for the phage done of interest, followed by construction of a full length anti-IL-17RA and/or IL-17RE or anti- IL-17C antibody clone by using the Fv sequences from the phage clone of interest and a suitable constant region (Fc) sequence.

In one embodiment, the invention provides assays for screening candidate or test compounds which modulate the signaling function of IL-17C and/or the heterodimeric IL-17RA/IL-17RE complex. The test compounds of the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds. (*See, e.g.,* Lam, 1997. Anticancer Drug Design 12: 145).

A "small molecule" as used herein, is meant to refer to a composition that has a molecular weight of less than about 5 kD and most preferably less than about 4 kD. Small molecules can be, *e.g.,* nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention.

Examples of methods for the synthesis of molecular libraries can be found in the art, for example in: DeWitt, et al., 1993. Proc. Natl. Acad. Sci. U.S.A. 90: 6909; Erb, et al., 1994. Proc. Natl. Acad. Sci. U.S.A. 91: 11422; Zuckermann, et al., 1994. J. Med. Chem. 37: 2678; Cho, et al., 1993. Science 261: 1303; Carrell, et al., 1994. Angew. Chem. Int. Ed. Engl. 33: 2059; Carell, et al., 1994. Angew. Chem. Int. Ed. Engl. 33: 2061; and Gallop, et al., 1994. J. Med. Chem. 37: 1233.

Libraries of compounds may be presented in solution (*see e.g.,* Houghten, 1992. Biotechniques 13: 412-421), or on beads (*see* Lam, 1991. Nature 354: 82-84), on chips (*see* Fodor, 1993. Nature 364: 555-556), bacteria (*see* U.S. Patent No. 5,223,409), spores (*see* U.S. Patent 5,233,409), plasmids (*see* Cull, et al., 1992. Proc. Natl. Acad. Sci. USA 89: 1865-1869) or on phage (*see* Scott and Smith, 1990. Science 249: 386-390; Devlin, 1990. Science 249: 404-406; Cwirla, et al., 1990. Proc. Natl. Acad. Sci. U.S.A. 87: 6378-6382; Felici, 1991. J. Mol. Biol. 222: 301-310; and U.S. Patent No. 5,233,409.).

In one embodiment, a candidate compound is introduced to an antibody-antigen complex and determining whether the candidate compound disrupts the antibody-antigen complex, wherein a disruption of this complex indicates that the candidate compound modulates the signaling function of IL-17C and/or the heterodimeric IL-17RA/IL-17RE complex.

In another embodiment, the IL-17C homodimer is provided and exposed to at least one neutralizing monoclonal antibody. Formation of an antibody-antigen complex is detected, and one or more candidate compounds are introduced to the complex. If the antibody-antigen complex is disrupted following introduction of the one or more candidate compounds, the candidate compounds is useful to treat disorders associated with IL-17C signaling.

In another embodiment, a soluble protein of IL-17C is provided and exposed to at least one neutralizing monoclonal antibody. Formation of an antibody-antigen complex is detected, and one or more candidate compounds are introduced to the complex. If the antibody-antigen complex is disrupted following introduction of the one or more candidate compounds, the candidate compounds is useful to treat disorders associated with IL-17C signaling.

Determining the ability of the test compound to interfere with or disrupt the antibody-antigen complex can be accomplished, for example, by coupling the test compound with a radioisotope or enzymatic label such that binding of the test compound to the antigen or biologically-active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemission or by scintillation counting. Alternatively, test compounds can be enzymatically-labeled with, for example, horseradish peroxidase, alkaline phosphatase, or luciferase, and the enzymatic label detected by determination of conversion of an appropriate substrate to product.

In more than one embodiment, it may be desirable to immobilize either the antibody or the antigen to facilitate separation of complexed from uncomplexed forms of one or both following introduction of the candidate compound, as well as to accommodate automation of the assay. Observation of the antibody-antigen complex in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-antibody fusion proteins or GST-antigen fusion proteins can be adsorbed onto glutathione sepharose beads (Sigma Chemical, St. Louis, MO) or glutathione derivatized microtiter plates, that are then combined with the test compound, and the mixture is incubated under conditions conducive to complex formation (*e.g*., at physiological conditions for salt and pH). Following incubation, the beads or microtiter plate wells are washed to remove any unbound components, the matrix immobilized in the case of beads, complex determined either directly or indirectly. Alternatively, the complexes can be dissociated from the matrix, and the level of antibody-antigen complex formation can be determined using standard techniques.

The results obtained in the screening assays, for example, the cell-based biological assays, can be followed by testing in animal, e.g. murine, models and human clinical trials. If desired, murine monoclonal antibodies identified as having the desired properties can be converted into chimeric antibodies, or humanized by techniques well known in the art, including the "gene conversion mutagenesis" strategy, as described in U.S. Pat. No. 5,821,337. Agents suitable in the treatment of inflammatory bowel disease as described herein can be selected, using well known receptor binding assays and/or animal models of IBD, for example, as described herein.

### In vitro Model of IBD

An *in vitro* model of IBD has been developed by MacDonald, T. and co-workers, and in described by Braegger, C. P. and MacDonald, T. in Chapter 8 of "Immunology of Gastrointestinal Disease", and has earlier been reported by MacDonald, T. and Spencer, J., J. Exp. Med. 167, 1341-1349 (1988). In this model, small explants (1-2 mm across) of human fetal gut tissue (small or large bowel) containing T lymphocytes at the stage of 15-20 weeks gestation are cultured. The human fetal gut can be maintained in organ culture for several weeks with retention of morphology, epithelial cell renewal and enterocyte function. All of the T cells in the explant can be activated by culturing in the presence of pokeweed mitogen or monoclonal anti-CD3 antibodies. The gross appearance of the explants shows major changes as a result of T cell activation. The changes in the small bowel explants as a result of T cell activation are reminiscent of the mucosal change seen in early stages of Crohn's disease, and the goblet cell depletion seen in colon explants is also a feature of ulcerative colitis. This model can be used to study the interaction of T cells with the gut epithelium and specifically, to observe responses to T cell activation.

### Animal Models of IBD

The first group of animal models of IBD includes animals spontaneously developing diseases reminiscent of some forms of IBD. Spontaneous animal models include C3H/HeJ mouse, Japanese waltzing mice, swine dysentery and equine colitis, caused by C. difficile, and the cotton top tamarin. The diseases that these animals suffer have recently been subdivided into five types, two of which resemble UC. Of these models, tamarin are preferred, as a large proportion of these animals have some form of gut disorder, and many of them also develop bowel cancer, as do patients with UC.

In another approach, various irritants, such as ethanol, acetic acid, formalin, immune complexes, trinitrobenzene sulphonic acid (TNBS), bacterial products or carrageenan are used to generate acute or chronic inflammation. A model of this kind has been developed by Wallace, J. and coworkers [Morris et al. Gastroenterology 96, 795 (1989)].

According to a third approach, transgenic animals are used to model IBD. Most human patients who have ankylosing spondylitis also carry the gene for HLA-B27. It has been observed that such patients are at greater risk of developing IBD. HLA-B27 transgenic rats, which were attempted to model spondyloarthropathies, in addition to the joint disease, also showed symptoms of chronic inflammation of the bowel which, though not identical, had many similarities with CD. Accordingly, the HL-B27 transgenic rats can be used to model IBD.

Another suitable animal model is described in the Example below.

The invention further pertains to novel agents identified by any of the aforementioned screening assays and uses thereof for treatments as described herein.

The following examples are offered for illustrative purposes only, and are not intended to limit the scope of the present invention in any way.

All patent and literature references cited in the present specification are hereby expressly incorporated by reference in their entirety.

### Example

IL-C is an autocrine cytokine that regulates the innate immune function of epithelial cells

### Materials and Methods

**Mice.** C57BL/6 mice were purchased from Charles River Laboratories. *Rag2^{-l-}:Il2rg^{-l-}* mice were purchased from Taconic Farms Inc. Both *Il17re^{-l-}* and *Il17c^{-l-}* mice were backcrossed to the C57BL/6 strain (N=10), as described in Figure 11. *Myd88*^{*-*/*-*} mice were generated as described in Figure 39. Homozygous null *Il17re*^{*-*/}*⁻, Il17c^{-l-}* and *Myd88*^{*-*/*-*} mice were generated by intercrossing the respective heterozygote animals. The genotypes of the *Il17re*^{*-*/}*⁻, Il17c*^{*-*/*-*} and *Myd88*^{*-*/*-*} mice occurred at the expected Mendelian frequency. All three strains were fertile, and healthy under specific pathogen free conditions. All animal experiments were approved by the Genentech Institutional Animal Care and Use Committee (IACUC).

**Recombinant proteins and antibodies.** Recombinant cytokines TNFα, IL-1β, IL-17A and IL-22 and anti-human IL-17RA blocking antibody (MAB 177) were purchased from R&D Systems. Mature human and mouse IL-17C were generated as described below.

**Generation of recombinant hIL-17C and mIL-17C.** Mature human IL-17C (His-19 to Val-197) and mature mouse IL17C (Asp-17 to Gln-194) were cloned into the expression vector pRK5 as an N-terminal Flag fusions. To remove an internal cleavage site and improve protein purification of hIL-17C, residues Gly-77 and Arg-78 were substituted to the corresponding murine residues Arg and Thr, respectively. The modified hIL-17C dimerized and had almost identical functional and receptor binding activities as partially truncated preparations of unmodified hIL-17C as well as recombinant hIL-17C purchased from R&D Systems and eBioscience. Flag-fusion proteins were produced by transient transfection of Chinese hamster ovary (CHO) cells. Two week transient conditioned media was batch adsorbed overnight at 4°C to M2 anti-flag resin (Sigma), washed with ice cold 0.1% Triton X-114 in PBS, then PBS washed to baseline and eluted with 0.1M acetic acid which was immediately neutralized with a 4% volume of 1.5 M Tris pH 8.6. The eluted pool was separated on a Superdex 200 gel filtration column (GE) in PBS containing 0.15M NaCl; the desired protein peak was pooled, concentrated, dialyzed into PBS and sterile filtered.

**Heat-killed E. coli stocks.** DH5α cells (Invitrogen) were cultured overnight in LB broth at 37°C; the suspension was centrifuged and suspended in water at a density of 1 x 10¹⁰ CFU/ml and heat-killed in boiling water for 30 minutes.

**In-vitro cell culture growth conditions.** For stimulation experiments, epithelial cells were grown to confluence in 24-well plates. HCT-15 cells were cultured in DMEM supplemented with 10% FBS (Hyclone), 2 mM glutamine and 100 µg/ml penicillin/streptomycin. HEKn cells were cultured in Epilife medium supplemented with human keratinocyte growth supplement (HKGS) in plates treated with Coating Matrix (Invitrogen). Primary human tracheal epithelial cells (HTEpC, Cell Applications) were cultured in Bronchial/Tracheal Epithelial Growth Medium (Cell Applications). HDFn cells were cultured in medium 106 supplemented with Low Serum Growth Supplement (LSGS, Invitrogen).

**In vitro cell culture stimulations.** For stimulation experiments, epithelial cells were grown to confluence in 24-well plates and stimulated for 24 hours in the presence or absence of 1 x 10⁸ CFU heat killed *E. coli,* 1 µg/ml of endotoxin-free TLR ligands peptidoglycan (PGN-SA), Pam2CSK4, Pam3CSK4, Poly(I:C), flagellin (FLA-ST Ultrapure), and CpG oligodinucleotides (ODN2006) (all from Invivogen), and 10 ng/ml TNFα, IL-1β and 100 ng/ml IL-17A, IL-22. For RNA expression analysis, 1 x 10⁶ HCT-15 human colon epithelial cells (ATCC) were plated on 6-well plates and cultured overnight prior to stimulations. For hIL-17C functional analysis, 1 x 10⁴ primary human epidermal keratinocytes (HEKn, Invitrogen) or human dermal fibroblasts (HDFn, Invitrogen) were plated in 96-well plates and cultured overnight prior to stimulation with recombinant human IL-17C for 48 hours. For antibody blocking experiments, HEKn cells were pre-incubated with anti-human IL-17RA antibody for 15 minutes at 37°C prior to stimulation with hIL-17C for 48 hours. For mouse IL-17C functional analysis, 1 x 10⁴ primary mouse epidermal keratinocytes (MPEK, Cellntec) were plated in 96-well plates and cultured overnight prior to stimulation with mouse IL-17C or mouse IL-17A for 48 hours.

**Antibody blockade studies.** HCT-15 cells were grown to confluence in 12-well plates and stimulated with 1 µg/ml PGN-SA, 1 µg/ml FLA-ST, 10 ng/ml TNFα or 10 ng/ml IL- Iβ for 24 hours in the presence of isotype control antibodies and either 10 µg/ml anti-IL-1β (R&D Systems), 1 µg/ml TNFR2-Fc (described previously⁵⁰), 10 µg/ml anti-TLR2 (Invivogen), or 10 µg/ml anti-TLR5 (Invivogen). After 24h, supernatants were collected and analyzed for IL-17C expression by ELISA.

**Primary keratinocyte cultures.** Isolation and culture of mouse primary epidermal keratinocytes from neonatal mice was performed as previously described⁵⁵. Isolation and culture of epidermal keratinocytes derived from tail skin of adult mice was accomplished by following the above protocol. Similar results were obtained using either neonatal or adult tail skin derived primary epidermal keratinocytes.

**In vivo flagellin stimulations.** C57BL/6 or *Rag2*^{*-*/}*⁻ :Il2rg*^{*-*/*-*} mice were injected intra-peritoneally with 5 µg or 3.3 µg of flagellin (Rec FLA-ST, Invivogen) in 500 µl volume (10 µlg/ml) or saline (PBS). RNA was isolated from colon tissues after 2 hours of treatment.

**Bone marrow chimeras.** For bone marrow (BM) chimera, recipient mice were irradiated with 1100 rads and reconstituted 4 hours later with BM cells (5 x 10⁶ cells i.v.). These mice were used for flagellin i.p. injection experiments 7 weeks post-transplantation as described above.

**Microarray analysis.** 1 µg of total RNA was amplified using the Low RNA Input Fluorescent Linear Amplification protocol (Agilent Technologies, Palo Alto, CA). The cRNA was purified using the RNeasy Mini Kit (Qiagen). 750 ng of Universal Human Reference (Stratagene) cRNA labeled with Cyanine-3 and 750 ng of the test sample cRNA labeled with Cyanine-5 were fragmented for 30 minutes at 60°C before loading onto Agilent Whole Human Genome arrays (Agilent Technologies). The samples were hybridized for 18 hours at 60°C with constant rotation. Arrays were washed, dried and scanned on the Agilent scanner according to the manufacturer's protocol (Agilent Technologies). Microarray image files were analyzed using Agilent's Feature Extraction software version 9.5 (Agilent Technologies). Statistical analysis of microarrays was done using software from the R project (http://r-project.org) and the Bioconductor project (http://bioconductor.org). Background subtracted microarray data were quantile normalized between arrays. Normalized data were then log₂-transformed, and probes were filtered such that only probes that mapped to Entrez genes were retained. A non-specific filter that removed the 50% least variable probes was then applied⁵⁶. To identify differentially expressed genes, we used the limma package⁵⁷ to calculate attenuated t-statistics. The false discovery rate (FDR) was calculated using the Benjamini-Hochberg method. Genes were identified as differentially expressed at an FDR of 0.1.

**ELISA.** Secreted levels of human IL-17C and human G-CSF were measured using DuoSet ELISA Kits according to the manufacturer's protocol (R&D Systems). Similarly, secreted levels of human BD-2 were measured as instructed in Human BD-2 ELISA Development Kit (PeproTech). Mouse IL-17C ELISA was developed by coating plates with 2 µg/ml anti-mouse IL-17C antibody (R&D Systems, MAB2306) in PBS overnight at 4°C. Plates were washed 3 times and incubated with blocking buffer (PBS, 1% BSA) for 1 hour at room temperature. After 3 washes, mouse IL-17C protein standards and samples were incubated for 2 hours at room temperature. To detect mouse IL-17C levels, plates were incubated with 1 µg/ml of biotinylated anti-IL-17C MAb (clone IL17C:7516, in-house generated, Ab characterization in Figure 31b) for 1 hour and followed by 3 additional washes and incubation with Strepavidin-horseradish peroxidase (R&D Systems) for 20 minutes.

**Adenoviral infections.** Eight-week-old wildtype C57BL/6 or *Il17re^{-l-}* mice were injected with 1 x 10¹⁰ PFU of adenovirus intravenously. Mice were bled retro orbitally at day 7 post-infection to measure serum IL-17C levels. Tissues were collected on day 21 post-infection for histopathological and RNA analysis.

**Retroviral transduction.** cDNAs of human *IL17RA, IL17RB, IL17RC, IL17RD,* and *IL17RE* were cloned into a bicistronic retroviral vector that expresses GFP (pMSCV-IRES-GFP) and used to transfect Phoenix Ecotropic cells (ATCC). Retroviral supernatant containing polybrene were used to infect 293 cells and human dermal fibroblasts, HDFn. GFP⁺ cells were purified by flow cytometry. Receptor expression was confirmed via FACS analysis immuno-staining and/or western blot.

**Radioligand Cell Binding Assay.** hIL-17C was iodinated using the Iodogen method and the radiolabeled hIL-17C had a specific activity of 72.5 µCI/µg. 293 cells expressing the IL-17 receptors were incubated for 2 hours at room temperature with a fixed concentration of iodinated hIL-17C combined with increasing concentrations of unlabeled hIL-17C and including a zero-added, buffer only sample. After the 2-hour incubation, the competition reactions were transferred to a Millipore Multiscreen filter plate and washed 4 times with binding buffer to separate the free from bound iodinated hIL-17C. The filters were counted on a Wallac Wizard 1470 gamma counter and binding data was evaluated using NewLigand software (Genentech), which uses the fitting algorithm of Munson and Rodbard to determine the binding affinity of IL-17C⁵⁸.

**FACS analysis of IL-17C-IL-17R association.** 1 x 10⁵ 293 cells, 293 cells expressing GFP or hIL-17 receptors (RA, RE) were incubated with 50 ng flag-tagged hIL-17C in FACS buffer (PBS, 0.5% BSA, 0.2% sodium azide) for 30 minutes at room temperature. Cells were then washed and incubated with biotinylated anti-flag antibody (Sigma). Biotinylated antibodies were visualized with streptavidin-APC (BD). Doublets were excluded with forward-scatter area versus width pulses and 4,6-diamidino-2-phenylindole (DAPI) was used for exclusion of dead cells.

**Intra-dermal ear injections.** Wild-type C57BL/6 mice received intra-dermal injections of 0.5 µg of recombinant mouse IL-17C or the equivalent volume of PBS every other day for 10 days. Ear thickness was measured prior to and on the indicated days of the experiment. **DSS-induced colitis.** Acute colitis was induced by administration of 1.5-2% DSS (w/v, molecular mass 36-50 kDa; MP Biomedicals) in drinking water ad libitum. DSS was given for a total of 5 days (day 0 through 5), after which animals were given regular drinking water from day 6 through 15. Animals were weighed daily starting at day 4 of DSS administration until day 15. On day 15, animals euthanized by carbon dioxide inhalation and colons and mesenteric lymph nodes were removed and analyzed. For evaluation of colitis, colons were flushed with cold sterile PBS, scored, and weighed. Colons were assessed a visual score on a scale of 0-4 (0 = no inflammation, 1 = 25% of the colon inflamed and/or thickened, 2 = 50% of the colon inflamed and/or thickened, 3 = 75% of the colon inflamed and/or thickened, 4 = 100% of the colon inflamed and/or thickened). Colon weight was recorded using a standard laboratory scale. For interim analyses, experiments were performed as above, but terminated on day 9. Colons were used for histological and RNA analyses. For colon organ cultures, colons from DSS-treated or untreated C57BL/6 mice were flushed with cold RPM1 1640 supplemented with 100 µg/ml penicillin/streptomycin. Colons were cut longitudinally and cultured in 12-well plates containing 1 ml of RPMI 1640 supplemented with 100 µg/ml penicillin/streptomycin for 24 hours at 37°C. Supernatants were collected and centrifuged at 14,000 rpm for 5 minutes at 4°C.

**Imiquimod model of skin inflammation.** 10-12 week old BALB/c, *Il17c*^{*+*/*+*} or *Il17c*^{*-*/}*⁻,* or *Il17re*^{*+*/*+*} or *Il17re*^{*-*/*-*} mice were administered 50 mg Aldara cream (5% Imiquimod in Graceway, 3M) in the shaved back and right ear daily for 5 days. Clinical scoring and ear thickness measurements were performed daily. Scoring was based upon the manifestation of psoriatic symptoms, such as erythema, scaling and thickness: 0, No disease. 1, Very mild erythema with very mild thickening and scaling involving a small area. 2, Mild erythema with mild thickening and scaling involving a small area. 3, Moderate erythema with moderate thickening and scaling (irregular and patchy) involving a small area (<25%). 4, Severe erythema with marked thickening and scaling (irregular and patchy) involving a moderate area (25-50%). 5, Severe erythema with marked thickening and scaling (irregular and patchy) involving a large area (>50%). Ear and back tissue were harvested on day 5 for histological evaluation.

**Generation of adenoviral stocks.** Mature mouse IL-17C (Asp-17 to Gln-194) was cloned into pShuttle vector (Clontech) as an N-terminal Flag fusion. Subsequently, Ad5 vectors were generated using the AdenoX Expression System (Clontech). Viral production, titering, and expansion were performed by the Vector Development Labortatory at the Baylor College of Medicine, Houston, TX.

**Histology.** Immunohistochemistry and staining was performed on 4 µm thick formalin-fixed paraffin embedded (FFPE) tissue sections mounted on glass slides. For Imiquimod experiments, right pinnas were collected at day 5, fixed in 10% neutral buffered formalin, processed and embedded longitudinally to yield sections stained with hematoxylin and eosin (H&E), 1 µg/ml rabbit anti-Ki-67 (Clone SP6; Thermo Scientific) and 10 µg/ml rat anti-Ly6G/C (clone Gr-1; Pharmingen). For adenoviral over-expression experiments, the gall bladder and pancreas were harvested and fixed formalin. After fixation, sections were prepared using a cryotome (X, Y) and stained with H&E and anti-Ly6G for histological analysis. For DSS-induced colitis experiments, colons were prepared for histology by gut roll technique and stained with H&E, Alcian blue (AB), 10 µg/ml rat anti-F4/80 (Clone C1:A3-1; Serotec) and anti-Ly6G/C.

**Generation of adenoviral stocks.** Mature mouse IL-17C (Asp-17 to Gln-194) was cloned into pShuttle vector (Clontech) as an N-terminal Flag fusion. Subsequently, Ad5 vectors were generated using the AdenoX Expression System (Clontech). Viral production, titering, and expansion were performed by the Vector Development Labortatory at the Baylor College of Medicine, Houston, TX.

**Real-time RT-PCR primers and probes.** Real-time RT-PCR was conducted on an ABI 7500 Real-Time PCR system (Applied Biosystems) with primers and probes (see below) and Taqman one-step RT-PCR master mix (Applied Biosystems). Samples were normalized to the control housekeeping gene *RPL19* and relative expression was calculated by the ΔΔC_{T} method. The sequences of custom made primers and probes, SEQ ID NOS 1-28, respectively, in order of appearance, are shown in Table 1.

**Table 1: Custom made primers and probes**

| **Species** | **Gene** | | **Sequence** | **Label** |
|---|---|---|---|---|
| mouse | RPL19 | forward | GCG CAT CCT CAT GGA GCA CA | |
| mouse/human | RPL19 | reverse | GGT CAG CCA GGA GCT TCT TG | |
| mouse/human | RPL19 | probe | CAC AAG CTG AAG GCA GAC AAG GCC C | FAM, TAMRA |
| human | RPL19 | forward | GCG GAT TCT CAT GGA ACA CA | |
| mouse | IL17RE | forward | AAT TCC TTC TGC CCT GCA T | |
| mouse | IL17RE | reverse | ACA CTT TTT GCG CCT CAC AG | |
| mouse | IL17RE | probe | TAG AGG CCT CCT ACC TGC AAG AGG AC | FAM, TAMRA |
| human | IL17RA | forward | TTC TGT CCA AAC TGA GGC ATC A | |
| human | IL17RA | reverse | AGG GTC AAC CAC AAA GTG GC | |
| human | IL17RA | probe | CAC AGG CGG TGG CGT TTT ACC TTC | FAM, TAMRA |
| human | IL1B | forward | GAA TTT GAG TCT GCC CAG TTC | |
| human | IL1B | reverse | AAG ACG GGC ATG TTT TCT G | |
| human | IL1B | probe | CCA ACT GGT ACA TCA GCA CCT CTC AAG | FAM, TAMRA |
| human | IL17C | forward | TTG GAG GCA GAC ACC CAC C | |
| human | IL17C | reverse | GAT AGC GGT CCT CAT CCG TG | |
| human | IL17C | probe | CCA TCT CAC CCT GGA GAT ACC GTG TG | FAM, TAMRA |
| human | TNF | forward | CCT GCC CCA ATC CCT TTA TT | |
| human | TNF | reverse | CCC CAA TTC TCT TTT TGA GCC | |
| human | TNF | probe | CCC CCT CCT TCA GAC ACC CTC AAC C | |
| mouse | I117c | forward | CTG GAA GCT GAC ACT CAC G | |
| mouse | I117c | reverse | GGT AGC GGT TCT CAT CTG TG | |
| mouse | I117c | probe | CCA TCT CAC CAT GGA GAT ATC GCA TC | |
| mouse | Illb | forward | TGG TAC ATC AGC ACC TCA CA | |
| mouse | Illb | reverse | TTA TGT CCT GAC CAC TGT TGT TT | |
| mouse | Illb | probe | AGA GCA CAA GCC TGT CTT CCT GGG | |
| mouse | Tnf | forward | GAC CAG GCT GTC GCT ACA TCA | |
| mouse | Tnf | reverse | CCC GTA GGG CGA TTA CAG TCA | |
| mouse | Tnf | probe | TGA ACC TCT GCT CCC CAC GGG AG | |

**Table 2: Applied Biosystems Taqman Gene Expression Assays used:**

| **Species** | **Gene** | **ABI Cat#** |
|---|---|---|
| mouse | Il17c | Mm00521397_m1 |
| mouse | Il17a | Mm00439619_m1 |
| mouse | Il17f | Mm00521423_m1 |
| mouse | Il22 | Mm0044424_m1 |
| human | CXCL1 | Hs00236937_m1 |
| human | CXCL2 | Hs00236966_m1 |
| human | CXCL3 | Hs00171061_m1 |
| human | CSF3 | Hs99999083_m1 |
| human | IL8 | Hs00174103_m1 |
| human | CCL2 | Hs00234140_m1 |
| human | IL1F9 | Hs00219742_m1 |
| human | CCL20 | Hs01011368_m1 |
| human | SAA4 | Hs00197854_m1 |
| human | DEFB4 | Hs00823638_m1 |
| human | S100A7 | Hs00161488_m1 |

**Statistical analysis.** Statistical analyses were made in JMP version 8.0.2 software (SAS Institute). Dunnett's test was used to compare group means between all tested groups and a reference group. P values < 0.05 were considered significant.

### Results and Discussion

### IL-17C binds to the IL-17RA and IL-17RE subunits

To understand the biological functions of IL-17C, identification of IL-17C responsive cells through characterization of the IL-17C receptor complex was a first focus. The binding of IL-17C to HEK293 (293) cell lines which retrovirally overexpressed all known IL-17 receptor family members including IL-17RA to IL-17RE chains was directly screened¹⁰⁻¹². Expression of the receptors on the cell surface was confirmed by immunofluorescence with an anti-Flag antibody (data not shown). IL-17C specifically bound to 293 cells expressing either IL-17RA or IL-17RE (Figure 1), but not cells expressing IL-17RB, IL-17RC, or IL-17RD (data not shown). In addition, interactions between IL-17RE expressing cells and IL-17A, IL-17B, IL-17E, and IL-17F were not detected (data not shown). Consistent with the results of the cellular screen, biochemical interactions were specifically detected between IL-17C and IL-17RA as well as IL-17RE. Competition binding experiments with radiolabeled IL-17C identified a high affinity interaction between IL-17C and IL-17RE, K_{D}= 0.35nM, and a low affinity interaction with IL-17RA, K_{D} =1.4mM (Figure 2). Co-immunoprecipitation studies demonstrated the association of IL-17RA and IL-17RE subunits on the cell surface when both chains are overexpressed in 293 cells (Figure 3). These data suggested that IL-17C also signals through a heterodimeric receptor complex, which shares the common IL-17RA chain with IL-17A, IL-17F, and IL-17E and also uses its unique IL-17RE receptor for its selectivity and high affinity recognition.

### IL-17RE and IL-17RA are required for IL-17C signaling

To identify IL-17C responsive tissues and cells, coexpression of IL-17RA and IL-17RE was screened. In agreement with previous studies, IL-17RA was ubiquitously expressed (Figure 4a,b)³⁹. However, gene expression profiling on multiple tissues revealed the greatest expression of IL-17RE in mucosal organs, including the trachea, lung, skin and colon (Figure 5a) ⁴⁰, suggesting these tissue might be target organs of IL-17C. Further examination of specific cell-types within these mucosal tissues revealed co-expression IL-17RA and IL-17RE on cells of epithelial origin, including primary human keratinocytes and human colon epithelial cells (Figure 5b). Interestingly, while IL-17RA was detected on primary human fibroblasts and human peripheral blood mononuclear cells (PBMCs), expression of IL-17RE was very low in these cells, with some detectable expression in T cells (data not shown). These results predicted mucosal epithelial cells, but not PBMCs or fibroblasts, could serve as primary target cells for IL-17C.

IL-17A and IL-17F target both tissue epithelial cells and fibroblasts⁵². Given the sequence homology of IL-17C with IL-17A and IL-17F, and their shared IL-17RA chain, it was hypothesized that IL-17C may induce similar signaling responses in epithelial cells as those by IL-17A and IL-17F. As assessed by ELISA, treatment of human primary keratinocytes with different doses of IL-17C induced the expression of Granulocyte colony-stimulating factor (G-CSF) and β-Defensin2 proteins, two known targets of IL-17A, in a dose-dependent manner (Figure 6), demonstrating that keratinocytes are responsive to IL-17C. Similar results were detected with other primary epithelial cell types (data not shown).

The requirement for IL-17RA and IL-17RE in IL-17C signaling was next confirmed. To further confirm that IL-17RA and IL-17RE are used by IL-17C in these cells, keratinocyte stimulation with IL-17C in the presence of an anti-IL-17RA blocking antibody resulted in a dose dependent inhibition of IL-17C mediated G-CSF and β-Defensin2 induction thereby demonstrating a functional requirement for IL-17RA (Figure 7). Given the lack of a blocking antibody for IL-17RE, two complementary systems were used, gain and loss-of-function, to validate the requirement of IL-17RE for the signaling functions of IL-17C. Human primary dermal fibroblasts, which have minimal IL-17RE expression, were unable to induce G-CSF in response to IL-17C (Figure 8a). Notably, these cells did induce G-CSF in response to the stimulation of IL-17A (Figure 8b). Because human primary dermal fibroblasts express IL-17RA, it was tested whether ectopic expression of IL-17RE would confer responsiveness to IL-17C. Expression of IL-17RE in these cells allowed the IL-17C-dependent induction of G-CSF, providing evidence that IL-17RE is required for IL-17C responsiveness and function (Figure 8a). Additionally, IL-17C responsiveness was lost in keratinocytes derived from *Il17re* ^{*-*/*-*}mice, while IL-17A effects remained intact (Figure 9). These results highlight the distinct receptor usage between IL-17C and IL-17A.

To confirm the role of IL-17RE for the signaling functions of IL-17C *in vivo,* an adenoviral system was used to systemically over-express IL-17C in mice. This system induced rapid systemic expression of IL-17C (Figure 10a). Consistent with previous reports, over-expression of IL-17C in wild-type mice promoted neutrophil influx into tissues³⁶. Histological examination of the gall bladder mucosa and the pancreatic ductal system revealed loose mural and intraepithelial infiltration of neutrophils (Figure 10b). To address the functional requirement for IL-17RE in IL-17C biology *il17re^{-l-}* mice were generated (Figure 11). While IL-17C over-expression promoted the influx of neutrophils to the gall bladder and pancreas in wild-type mice, no infiltration was detected in *il17re^{-l-}* mice (Figure 12a,b). Taken together, this data demonstrated that IL-17RA and IL-17RE are required for IL-17C signaling and implicate them as subunits of a novel, heterodimeric IL-17 family receptor.

### IL-17C has similar biological functions as IL-17A

To fully understand the biological functions, and specifically the downstream activities, of IL-17C on epithelial cells, the microarray analysis on IL-17C treated human primary keratinocytes was performed. Similar to IL-17A, IL-17C indcued the expression of genes encoding proteins involved in innate immunity, including cytokines and chemokines, inflammatory mediators, and anti-microbial peptides from keratinocytes (Figure 13). The induction of a subset of these genes by either IL-17C or IL-17A was further confirmed by quantitative PCR (Figure 14). Interestingly, analogous to the biological activities of IL-17A, IL-17C induced a number of genes involved in neutrophil function, including CCL20, G-CSF as well as anti-microbial peptides such as defensins and S100 proteins ^{42,59}. Moreover, similar to IL-17A, IL-17C displayed synergism with TNFα and IL-1γ in inducing β-Defensin2 (Figure 15) ⁶⁰⁻⁶². The similarity in the genes up-regulated by two cytokines suggests they are functionally redundant on epithelial cells.

The augmentation of immune responses through synergy with pro-inflammatory cytokines, such as TNFα, is a hallmark of IL-17A biology. The similarity between IL-17A and IL-17C responses prompted an examination of whether IL-17C could also synergize with other cytokines. Akin to IL-17A, IL-17C functioned synergistically with TNFα and IL-1β in inducing β-Defensin2 from keratinocytes (Figure 15)⁴¹. These results indicate a functional similarity between these two IL-17 family members.

### IL-17C is induced by bacterial and inflammatory stimuli

The induction of antimicrobial responses from epithelial cells by IL-17C led to the speculation that IL-17C expression might be regulated by bacterial pathogens. Therefore, IL-17C induction was examined in human dermal fibroblasts, PBMCs, and three types of epithelial cells following treatment with heat-killed *E. coli.* Strikingly, while the bacterial stimulations induced IL-17C in the colon epithelial cells, tracheal epithelial cells and keratinocytes (Figure 16), no IL-17C protein was detected in the fibroblast and PBMCs cultures (Figure 17). In addition, kinetic analysis revealed induction of IL-17C mRNA expression in response to bacterial stimuli is rapid (Figure 18). Kinetic analysis revealed that induction of IL-17C mRNA expression is a rapid response to bacterial stimuli (Figure 18). These data, along with the elucidation of the IL-17C receptor system and its selective expression pattern, support the premise that IL-17C is a cytokine produced by epithelial cells upon their sensing of microbial challenges such as a bacterial encounter and that functions in an autocrine manner to induce a rapid innate immune response in the epithelium.

As Toll-like-receptors, or TLRs, facilitate the cellular recognition and response to bacterial products, it was examined whether TLR stimulation itself is sufficient to induce IL-17C⁴³. TLR gene expression profiling was performed to determine which receptors would be physiological targets of the bacterial stimuli on epithelial cells. Only stimulation with agonist ligands of TLR2 and TLR5, which recognize bacterial lipopeptides and flagellin respectively, induced the production of IL-17C from colon epithelial cells (Figure 19a), as well as from primary tracheal epithelial cells and keratinocytes (data not shown), consistent with the expression of TLR2 and TLR5 in all three types of epithelial cells examined (Figure 19b). Importantly, stimulation of TLR2 and TLR5 signaling in epithelial cells did not result in the induction of other IL-17 family members (Figure 20a), suggesting the IL-17C is a unique family member expressed by epithelial cells upon sensing bacteria. Specifically, quantitative RT-PCR revealed expression of the extra-cellular receptors TLR2 and TLR5, which recognize bacterial lipopeptides and flagellin respectively, and the intracellular receptors, TLR3, which binds viral dsRNA, and TLR9, the receptor for unmethylated CpG oligonucleotides found in viral and bacterial DNA (Figure 19b). Stimulation with agonists to each of these TLRs revealed IL-17C induction was restricted to activation of the extracellular receptors TLR2 and TLR5, both specific for bacterial products (Figure 19a). In agreement with the cellular studies, *in vivo* stimulation with flagellin also induced expression of IL-17C in colon tissue (Figure 20b). In contrast, other IL-17 family members were not detected under these conditions (Figure 20a), suggesting the IL-17C is a unique family member produced by epithelial cells. No changes in either IL-17RA or IL-17RE expression upon stimulation with these agonists were detected (Figure 21).

Next, since host defense pathways initiated by bacterial insult include the secretion of the pro-inflammatory cytokines TNFα, IL-1β, IL-22 and IL-17A, it was tested whether these cytokines could regulate IL-17C ^{4,8,44}. IL-17C was detected in colon epithelial cell cultures stimulated with TNFα or IL-1β, but not with IL-22 or IL-17A (Figure 22). This is consistent with other reports of TNFα regulating IL-17C expression⁴³. Similar to the TLR stimulations, there was no detection of expression of other IL-17 family members in these cultures (Figure 23) or changes in IL-17RA and IL-17RE expression upon stimulation with these cytokines (data not shown).

The requirement for cross-talk between the different stimuli in regulating IL-17C expression was also evaluated. Given the obligate requirement for MyD88 in TLR2, TLR5 and IL-1β mediated responses, no IL-17C induction was detected in *Myd88*^{*-*/*-*} keratinocytes following TLR2, TLR5, or IL-1β stimulation (Figure 24 and Figure 25)⁴³. In contrast, TNFα-mediated induction *of Il17c* remained intact in *Myd8*^{*-*/*-*} derived keratinocytes (Figure 24). Likewise, although TNFRII-Fc and anti-IL-1β blocked TNFα and IL-1β induction of IL-17C from human colon epithelial cells respectively, *E.coli* mediated IL-17C secretion was unperturbed by these treatments (Figure 26). Similar results were observed in experiments where TLR2 and TLR5 signals were inhibited with blocking antibodies. Blockade of these receptors did not affect IL-17C induction following TNFα or IL-1β stimulation, suggesting the TLR and cytokine pathways regulate IL-17C expression independently of one another (Figure 27). Altogether, the results indicate that IL-17C is an epithelial cell derived cytokine, secreted as a direct response to bacterial products or through the pro-inflammatory cytokines TNFα and IL-1β.

### Leukocytes are not a predominant source of IL-17C in vivo

To further exclude lymphocytes as the major cellular source of IL-17C in vivo, flagellin was administrated to *Rag2*^{*-*/}*⁻:Il2rg*^{*-*/*-*} mice. While the induction of IL-22 from colon by flagellin was compromised in these mice, the induction of IL-17C was comparable to that from WT mice, indicating that lymphocytes are not the major source of IL-17C (Figure 28). To further exclude the role of leukocytes in the induction of IL-17C, bone marrow chimeric mice, in which bone marrow from either WT mice or *Il17c*^{*-*/*-*} mice was transferred to lethally irradiated WT or *Il17c*^{*-*/-} mice, were generated. Upon stimulation with flagellin, only colons from WT recipient mice, but not from *Il17c*^{*-*/*-*} recipients, expressed IL-17C, regardless whether they received WT or *Il17c^{-l-}* bone marrow (Figure 29). All together, these results corroborate with previous *in vitro* observations that leukocytes are not a major source of IL-17C, which is instead produced by epithelial cells upon bacterial challenges through the activation of TLR pathways. This is in contrast to the expression of IL-22, which is lost when leukocytes are absent.

### IL-17C displays a protective role in the DSS colitis model

Intestinal epithelial cells interact with commensal microbial flora constantly. Given the production of IL-17C by epithelial cells and its autocrine functions on epithelial cells, it was hypothesized that IL-17C might exert an important role in maintaining the homeostasis of the intestinal epithelial layer. Similar to IL-17C, IL-17A and IL-22 can also target tissue epithelial cells. Previous studies have suggested that these two cytokines exert essential host defense responses against invading pathogens.^{10,20} The expression of IL-17C was examined in the colon upon DSS treatment, a chemical that disrupts colon epithelium and perturbs the homeostasis of the commensal gut flora and the immune system, resulting in immune cell activation and cytokine secretion, inflammation and tissue damage followed by a resolution phase where immune homeostasis is re-established. In the colitis model induced by DSS, mice deficient in either IL-17A or IL-22 developed exacerbated disease partially due to defects in epithelial cell control of intestinal microfloras.^{24,46} We, thus, surmised a similar role for IL-17C. Analysis of IL-17C expression in the colon tissues of DSS challenged wild-type mice revealed mRNA induction beginning at day 2 post-treatment, and peaking between days 6-8 (Figure 30). The rapid induction of IL-17C following DSS treatment is in agreement with the cellular studies, but in contrast to the expression of IL-17A, IL-17F and IL-22, which are not detected until day 6 post-DSS challenge (Figure 32). Colon cultures confirmed elevated IL-17C protein expression at day 8, which corresponds to the peak of colitis in this model (Figure 31). These results highlight a distinction between the kinetics of IL-17C inducible expression to that of IL-17A and IL-17F *in vivo.*

To understand the function of IL-17C in this model, wild-type and *Il17re^{-l-}* mice were treated with 2% DSS in their drinking water for five days, after which they were allowed to recover for nine days. Weights were measured every day for a gross evaluation of disease. The weight recovery of *Il17re*^{*-*/*-*} mice was significantly retarded compared to littermate controls (Figure 33). In addition, the terminal colon scores, which reflect the degree of inflammation and delayed recovery from DSS treatment, were significantly higher in the *il17re^{-l-}* mice (Figure 34a). There was also a trend towards higher terminal colon weights, again reflecting the lack of epithelial cell recovery (Figure 34b). Microscopic analysis of colon tissue also revealed inadequate resolution of inflammation, characterized by the continued presence of cellular infiltrates, including F4/80⁺ macrophages and Ly6G⁺ neutrophils, expansion of the lamina propria, and crypt epithelial cell hyperplasia in *Il17re^{-l-}* mice compared to controls (Figure 35). DSS treated *Il17re*^{*-*/*-*}mice display greater goblet cell loss as seen by reduced Alcian Blue (AB) staining (Figure 35b). Interim analysis of colon tissues on day 9 also revealed greater inflammation and crypt loss, and elevated expression of pro-inflammatory cytokines and chemokines in *Il17re*^{*-*/*-*} mice. (Figures 36-38). DSS-treated *Il17c*^{*-*/*-*} mice exhibited a similar defect in the resolution of disease (Figures 39 and 41). These data reflect the requirement for IL-17C mediated host defense pathways to control the initial bacterial driven inflammation caused by DSS treatment. The absence of this early response leads to commensal mediated cytokine and chemokine secretion leading to leukocyte infiltration and tissue damage. Taken together, these data support an indispensable role of IL-17C, similar to that of IL-17A and IL-22, in restoring and/or maintaining intestinal epithelial homeostasis with intestinal microbes upon inflammatory challenges.

### IL-17C promotes an inflammatory skin phenotype

Although beneficial during host defense, the over-expression of protective cytokines, such as IL-17A and IL-22, can cause tissue inflammation and damage under certain conditions^{2,10,20}. These pro-inflammatory functions of IL-17A and IL-22 are exemplified in preclinical psoriatic models, in which both cytokines were shown to play pathogenic functions.³²_{'} ^{47,48} Given that IL-17C induced very similar downstream biological functions as those by IL-17A, it was hypothesized that IL-17C could elicit pathogenic functions in skin inflammation. Indeed, it was observed that intra-dermal IL-17C injections stimulated leukocyte infiltration and epidermal thickening of the tissue (Figures 42 and 43). Consistent with the role in G-CSF induction, neutrophils are the predominant cellular infiltrate in IL-17C injected ears.

To further analyze the pro-inflammatory function of IL-17C in the skin, the role of this pathway in mouse model of psoriasis was examined. A non-infectious cutaneous inflammation model was adopted, in which a topical TLR7-8 agonist, imiquimod, was applied to induce psoriatic-like skin lesions, characterized by epidermal proliferation and leukocyte infiltration, which is dependent on pathogenic Th17 cytokines⁴⁸. However, disease is reduced, but not lost, in the absence of lymphocytes, suggesting that non-lymphocyte derived factors also contribute to inflammation⁴⁸. RNA analysis revealed IL-17C induction following imiquimod treatment of wild-type mice, most likely due to the downstream effects following TLR7-8 stimulation, which include expression of TNFα and IL-1β or through TLR7, which is expressed at very low levels in murine keratinocytes (Figure 44 and data not shown)⁴⁹. Imiquimod-treated *Il17c*^{*-*/*-*} mice exhibited a significant reduction in inflammation and epidermal thickening compared to controls (Figures 45-47). Histological analyses revealed decreased keratinocyte proliferation as determined by Ki67 staining, and fewer Ly6/G⁺ neutrophilic infiltrates in the dermis and epidermal pustules of pinna from imiquimod-treated *Il7c*^{*-*/*-*} (Figure 46). Expression of pro-inflammatory cytokines was diminished in *Il17c*^{*-*/}*⁻,* reflecting the overall disease reduction in these animals (Figures 48 and 49). Similar results were detected in the *Il17re*^{*-*/*-*} strain (Figure 50). These data again highlight the functional similarity between IL-17C with IL-17A and IL-22 in mediating pro-inflammatory functions in non-infectious inflammation.

As shown above, the cellular source, receptors and biological function of the IL-17 cytokine family member, IL-17C was determined. IL-17C is rapidly induced in mucosal and cutaneous epithelial cells in response to bacterial stimulation or the pro-inflammatory cytokines IL-1β and TNFα. Although multiple pathways induce IL-17C, these pathways function independently of each other. Conversely, IL-17C is not detected in other cell-types, including PBMCs, stimulated under the same conditions. *In vivo* studies confirmed a non-obligate role for leukocytes in TLR induced IL-17C expression.

Furthermore, the above data demonstrate that IL-17C utilizes a unique heterodimeric complex consisting of the IL-17RA and IL-17RE subunits, which is preferentially expressed on epithelial cells. Although IL-17C binds with high affinity to IL-17RE and with low affinity to IL-17RA, both chains are essential for IL-17C function. In contrast to the IL-17RE subunit, which was considered an orphan receptor, a requirement for the IL-17RA subunit has been shown for the function of IL-17A, IL-17F and IL-17E. The above data demonstrates an obligate role for this receptor in IL-17C biology and further strengthens the hypothesis that IL-17RA is a shared receptor amongst all IL-17 cytokine family members¹². In addition, these results suggest the specificity for each family member lies in the second subunit of the heterodimeric complex. Binding of IL-17C to this receptor complex induces expression of pro-inflammatory chemokines and cytokines, anti-microbial peptides and other host defense pathways.

*In vivo,* IL-17C displays both protective and pathogenic functions. Loss of the IL-17C pathway exacerbates disease and delays recovery in the DSS colitis model. In this model, DSS treatment disrupts immune homeostasis in the colon, exposing host tissue to commensal bacteria, which initiates an immune response. In agreement with the cellular data, kinetic analysis reveals expression of IL-17C soon after initiating DSS treatment. This rapid induction can initiate host defense pathways, such as secretion of anti-microbial peptides, to control the bacterial burden caused by the DSS-induced breach of the epithelial barrier. Thus, in the absence of this pathway, this initial epithelial innate defense response is lost, causing the colon to be exposed to the massive number of resident bacteria. This in turn promotes increased immune responses from leukocytes, as seen by neutrophil and macrophage infiltration and induction of pro-inflammatory cytokines and chemokines, resulting in both greater and inadequate resolution of disease. Thus, IL-17C, as part of the immediate response by the epithelium, plays a unique role in controlling homeostasis in the gut mucosa.

In contrast to the DSS model, the above results show that IL-17C promotes pathogenic responses in the skin. These differences likely reflect the distinct environments of the cutaneous vs. gut mucosal epithelial barriers, and the nature of epithelial injury in each model. Unlike DSS, imiquimod induced inflammation has a minimal bacterial component, and is initiated by TLR7-TLR8 agonism of dendritic cells and possibly keratinocytes within the skin, which is a relatively sterile environment compared to the gut⁴⁸. Imiquimod induces a considerable number of inflammatory responses, but because the inflammation is not amplified by microbial products, expression of cytokines, such as IL-17C, are no longer beneficial, and instead enhance the inflammation and cause significant tissue pathology. Indeed, neutrophil infiltration, epidermal hyperplasia and edema are reduced in imiquimod treated *Il17c*^{*-*/*-*} and *Il17re*^{*-*/*-*} mice. Likewise, over-expression of IL-17C in the ear causes inflammation, further highlighting the pathogenic potential of this cytokine during a non-infectious state.

While the cellular studies indicate IL-17C shares many functional properties with IL-17A, such as the induction of host defense and tissue remodeling pathways, and synergism with pro-inflammatory cytokines, these proteins do not display redundancy *in vivo.* The distinction between IL-17A and IL-17C is dictated by differences in their regulation and cellular targets. While induction of IL-17C by epithelial cells is modulated by innate signals, both innate and adaptive immune stimuli promote secretion of IL-17A by leukocytes^{9,10}. Furthermore, IL-17A and IL-17C use different receptors, which vary in cellular distribution. As IL-17RA is broadly expressed, target cell specificity is dictated by the second subunit of the heterodimeric receptor complex. While a wide number of cells express IL-17RC, IL-17RE is primarily detected on epithelial cells^{9, 10}. Thus, the restricted expression of both IL-17C and IL-17RE to epithelia limits activity to this cell-type. Conversely, the extensive number of cell-types that secrete IL-17A, coupled with their migratory potential, and the broad distribution of the receptor, allows IL-17A to exert effects in multiple cellular systems^{9, 10}. These differences are exemplified in the *in vivo* studies, where unique functions for IL-17C were demonstrated. It was found that similar to IL-17A, IL-17C has both protective and pathogenic functions, however, these cytokines are not redundant. Kinetic studies reveal IL-17C induction precedes that of IL-17A and other T_{H}17 cytokines in the DSS colitis model, thus precluding compensation for the loss of IL-17C. Likewise, imiquimod mediated skin inflammation is reduced in the absence of IL-17C, again illustrating the non-overlapping role this cytokine plays in the epithelium.

The communication between the immune system and epithelial cells helps to enhance the defense functions of the epithelium against potentially dangerous environmental microorganisms. Expression of pathogen recognition receptors, such as the TLRs, allows epithelial cells to survey the microenvironment for potential infections. In the advent of pathogenic triggers, these TLR-mediated signals alert leukocytes to initiate host defense responses. The significance of these epithelial cell-derived signals is exemplified by the number of human diseases that result from dysfunction of the epithelial barrier. Diseases such as IBD, asthma and psoriasis all have an underlying epithelial cell component, and understanding how epithelial cells contribute to disease pathogenesis will provide therapeutic benefit¹⁻³.

Altogether, the above describe IL-17C biology as a unique contribution of mucosal and cutaneous epithelial cells to the innate immune response. Functioning in an autocrine manner to initiate an innate immune response in the epithelium upon a breach to the barrier and bacterial encounter, IL-17C represents a novel mechanism by which the epithelium participates in host defense. Given this mode of action, we propose that IL-17C provides an essential local stimulus to enhance the epithelial immune response and may play important functions in many infectious and autoimmune diseases.

### References

1. Abraham, C. & Medzhitov, R. Interactions between the host innate immune system and microbes in inflammatory bowel disease. Gastroenterology 140, 1729-37 (2011).
2. Nestle, F. O., Di Meglio, P., Qin, J. Z. & Nickoloff, B. J. Skin immune sentinels in health and disease. Nat Rev Immunol 9, 679-91 (2009).
3. Saenz, S. A., Taylor, B. C. & Artis, D. Welcome to the neighborhood: epithelial cell-derived cytokines license innate and adaptive immune responses at mucosal sites. Immunol Rev 226, 172-90 (2008).
4. Sims, J. E. & Smith, D. E. The IL-1 family: regulators of immunity. Nat Rev Immunol 10, 89-102 (2010).
5. Kishimoto, T. IL-6: from its discovery to clinical applications. Int Immunol 22, 347-52 (2010).
6. Jager, A. & Kuchroo, V. K. Effector and regulatory T-cell subsets in autoimmunity and tissue inflammation. Scand J Immunol 72, 173-84 (2010).
7. Blaschitz, C. & Raffatellu, M. Th17 cytokines and the gut mucosal barrier. J Clin Immunol 30, 196-203 (2010).
8. Kolls, J. K. & Khader, S. A. The role of Th17 cytokines in primary mucosal immunity. Cytokine Growth Factor Rev 21, 443-8 (2010).
9. Ahmed, M. & Gaffen, S. L. IL-17 in obesity and adipogenesis. Cytokine Growth Factor Rev 21, 449-53 (2010).
10. Iwakura, Y., Ishigame, H., Saijo, S. & Nakae, S. Functional specialization of interleukin-17 family members. Immunity 34, 149-62 (2011).
11. Hymowitz, S. G. et al. IL-17s adopt a cystine knot fold: structure and activity of a novel cytokine, IL-17F, and implications for receptor binding. Embo J 20, 5332-41 (2001).
12. Ely, L. K., Fischer, S. & Garcia, K. C. Structural basis of receptor sharing by interleukin 17 cytokines. Nat Immunol 10, 1245-51 (2009).
13. Liang, S. C. et al. An IL-17F/A heterodimer protein is produced by mouse Th17 cells and induces airway neutrophil recruitment. J Immunol 179, 7791-9 (2007).
14. Wright, J. F. et al. Identification of an interleukin 17F/17A heterodimer in activated human CD4+ T cells. J Biol Chem 282, 13447-55 Epub 2007 Mar 13 (2007).
15. Fort, M. M. et al. IL-25 induces IL-4, IL-5, and IL-13 and Th2-associated pathologies in vivo. Immunity 15, 985-95 (2001).
16. Lee, J. et al. IL-17E, a novel proinflammatory ligand for the IL-17 receptor homolog IL-17Rh1. J Biol Chem 276, 1660-4 (2001).
17. Rickel, E. A. et al. Identification of functional roles for both IL-17RB and IL-17RA in mediating IL-25-induced activities. J Immunol 181, 4299-310 (2008).
18. Shi, Y. et al. A novel cytokine receptor-ligand pair. Identification, molecular characterization, and in vivo immunomodulatory activity. J Biol Chem 275, 19167-76 (2000).
19. Liu, Y. et al. IL-17A and TNF-alpha exert synergistic effects on expression of CXCL5 by alveolar type II cells in vivo and in vitro. J Immunol 186, 3197-205 (2011).
20. Aujla, S. J. et al. IL-22 mediates mucosal host defense against Gram-negative bacterial pneumonia. Nat Med 14, 275-81 (2008).
21. Ishigame, H. et al. Differential roles of interleukin-17A and -17F in host defense against mucoepithelial bacterial infection and allergic responses. Immunity 30, 108-19 (2009).
22. Khader, S. A. et al. IL-23 and IL-17 in the establishment of protective pulmonary CD4+ T cell responses after vaccination and during Mycobacterium tuberculosis challenge. Nat Immunol 8, 369-77 (2007).
23. Ogawa, A., Andoh, A., Araki, Y., Bamba, T. & Fujiyama, Y. Neutralization of interleukin-17 aggravates dextran sulfate sodium-induced colitis in mice. Clin Immunol 110, 55-62 (2004).
24. Yang, X. O. et al. Regulation of inflammatory responses by IL-17F. J Exp Med 205, 1063-75 Epub 2008 Apr 14 (2008).
25. Fujino, S. et al. Increased expression of interleukin 17 in inflammatory bowel disease. Gut 52, 65-70 (2003).
26. Johansen, C. et al. Characterization of the interleukin-17 isoforms and receptors in lesional psoriatic skin. Br J Dermatol 160, 319-24 (2009).
27. Leipe, J. et al. Role of Th17 cells in human autoimmune arthritis. Arthritis Rheum 62, 2876-85 (2010).
28. Lock, C. et al. Gene-microarray analysis of multiple sclerosis lesions yields new targets validated in autoimmune encephalomyelitis. Nat Med 8, 500-8 (2002).
29. Hofstetter, H. H. et al. Therapeutic efficacy of IL-17 neutralization in murine experimental autoimmune encephalomyelitis. Cell Immunol 237, 123-30 Epub 2005 Dec 28 (2005).
30. Hueber, W. et al. Effects of AIN457, a fully human antibody to interleukin-17A, on psoriasis, rheumatoid arthritis, and uveitis. Sci Transl Med 2, 52ra72 (2010).
31. Lubberts, E. et al. Treatment with a neutralizing anti-murine interleukin-17 antibody after the onset of collagen-induced arthritis reduces joint inflammation, cartilage destruction, and bone erosion. Arthritis Rheum 50, 650-9 (2004).
32. Rizzo, H. L. et al. IL-23-mediated psoriasis-like epidermal hyperplasia is dependent on IL-17A. J Immunol 186, 1495-502 (2011).
33. Holland, D. B., Bojar, R. A., Farrar, M. D. & Holland, K. T. Differential innate immune responses of a living skin equivalent model colonized by Staphylococcus epidermidis or Staphylococcus aureus. FEMS Microbiol Lett 290, 149-55 (2009).
34. Johansen, C., Riis, J. L., Gedebjerg, A., Kragballe, K. & Iversen, L. Tumor necrosis factor alpha-mediated induction of interleukin 17C in human keratinocytes is controlled by nuclear factor kappaB. J Biol Chem 286, 25487-94 (2011).
35. Wu, Q. et al. IL-23-dependent IL-17 production is essential in neutrophil recruitment and activity in mouse lung defense against respiratory Mycoplasma pneumoniae infection. Microbes Infect 9, 78-86 (2007).
36. Hurst, S. D. et al. New IL-17 family members promote Th1 or Th2 responses in the lung: in vivo function of the novel cytokine IL-25. J Immunol 169, 443-53 (2002).
37. Li, H. et al. Cloning and characterization of IL-17B and IL-17C, two new members of the IL-17 cytokine family. Proc Natl Acad Sci U S A 97, 773-8 (2000).
38. Yamaguchi, Y. et al. IL-17B and IL-17C are associated with TNF-alpha production and contribute to the exacerbation of inflammatory arthritis. J Immunol 179, 7128-36 (2007).
39. Spriggs, M. K. Interleukin-17 and its receptor. J Clin Immunol 17, 366-9 (1997).
40. Li, T. S., Li, X. N., Chang, Z. J., Fu, X. Y. & Liu, L. Identification and functional characterization of a novel interleukin 17 receptor: a possible mitogenic activation through ras/mitogen-activated protein kinase signaling pathway. Cell Signal 18, 1287-98 (2006).
41. Chiricozzi, A. et al. Integrative Responses to IL-17 and TNF-alpha in Human Keratinocytes Account for Key Inflammatory Pathogenic Circuits in Psoriasis. J Invest Dermatol (2011).
42. Kao, C. Y. et al. IL-17 markedly up-regulates beta-defensin-2 expression in human airway epithelium via JAK and NF-kappaB signaling pathways. J Immunol 173, 3482-91 (2004).
43. Abreu, M. T. Toll-like receptor signalling in the intestinal epithelium: how bacterial recognition shapes intestinal function. Nat Rev Immunol 10, 131-44 (2010).
44. Apostolaki, M., Armaka, M., Victoratos, P. & Kollias, G. Cellular mechanisms of TNF function in models of inflammation and autoimmunity. Curr Dir Autoimmun 11, 1-26 (2010).
45. Van Maele, L. et al. TLR5 signaling stimulates the innate production of IL-17 and IL-22 by CD3(neg)CD127+ immune cells in spleen and mucosa. J Immunol 185, 1177-85 (2010).
46. Sugimoto, K. et al. IL-22 ameliorates intestinal inflammation in a mouse model of ulcerative colitis. J Clin Invest 118, 534-44 (2008).
47. Ma, H. L. et al. IL-22 is required for Th17 cell-mediated pathology in a mouse model of psoriasis-like skin inflammation. J Clin Invest 118, 597-607 (2008).
48. van der Fits, L. et al. Imiquimod-induced psoriasis-like skin inflammation in mice is mediated via the IL-23/IL-17 axis. J Immunol 182, 5836-45 (2009).
49. Reiter, M. J., Testerman, T. L., Miller, R. L., Weeks, C. E. & Tomai, M. A. Cytokine induction in mice by the immunomodulator imiquimod. J Leukoc Biol 55, 234-40 (1994).
50. Chiang, E. Y. et al. Targeted depletion of lymphotoxin-alpha-expressing TH1 and TH17 cells inhibits autoimmune disease. Nat Med 15, 766-73 (2009).
51. Marchiando, A. M., Graham, W. V. & Turner, J. R. Epithelial barriers in homeostasis and disease. Annu Rev Pathol 5, 119-44.
52. Brandl, K., Plitas, G., Schnabl, B., DeMatteo, R. P. & Pamer, E. G. MyD88-mediated signals induce the bactericidal lectin RegIII gamma and protect mice against intestinal Listeria monocytogenes infection. J Exp Med 204, 1891-900 (2007).
53. Lebeis, S. L., Bommarius, B., Parkos, C. A., Sherman, M. A. & Kalman, D. TLR signaling mediated by MyD88 is required for a protective innate immune response by neutrophils to Citrobacter rodentium. J Immunol 179, 566-77 (2007).
54. Wells, J. M., Loonen, L. M. & Karczewski, J. M. The role of innate signaling in the homeostasis of tolerance and immunity in the intestine. Int J Med Microbiol 300, 41-8.
55. Caldelari, R. & Muller, E. J. Short- and long-term cultivation of embryonic and neonatal murine keratinocytes. Methods Mol Biol 633, 125-38.
56. Bourgon, R., Gentleman, R. & Huber, W. Independent filtering increases detection power for high-throughput experiments. Proc Natl Acad Sci U S A 107, 9546-51.
57. Smyth, G. K. Linear models and empirical bayes methods for assessing differential expression in microarray experiments. Stat Appl Genet Mol Biol 3, Article3 (2004).
58. Munson, P. J. & Rodbard, D. Ligand: a versatile computerized approach for characterization of ligand-binding systems. Anal Biochem 107, 220-39 (1980).
59. Onishi, R. M. et al. SEF/IL-17R (SEFIR) Is Not Enough: AN EXTENDED SEFIR DOMAIN IS REQUIRED FOR IL-17RA-MEDIATED SIGNAL TRANSDUCTION. J Biol Chem 285, 32751-9.
60. Chabaud, M., Fossiez, F., Taupin, J. L. & Miossec, P. Enhancing effect of IL-17 on IL-1-induced IL-6 and leukemia inhibitory factor production by rheumatoid arthritis synoviocytes and its regulation by Th2 cytokines. J Immunol 161, 409-14 (1998).
61. Katz, Y., Nadiv, O. & Beer, Y. Interleukin-17 enhances tumor necrosis factor alpha-induced synthesis of interleukins 1,6, and 8 in skin and synovial fibroblasts: a possible role as a "fine-tuning cytokine" in inflammation processes. Arthritis Rheum 44, 2176-84 (2001).
62. Miossec, P. Interleukin-17 in rheumatoid arthritis: if T cells were to contribute to inflammation and destruction through synergy. Arthritis Rheum 48, 594-601 (2003).

## Claims

1. A method of reducing gastrointestinal inflammation, comprising administering to a subject in need at least one antagonist of the IL-17RA and IL-17RE receptors.

2. The method of claim 1 wherein said gastrointestinal inflammation is associated in inflammatory bowel disease.

3. The method of claim 1 wherein said antagonist signals through both the IL-17RA and the IL-17RE receptors.

4. The method of claim 1 wherein said antagonist binds to both the IL-17RA and the IL-17RE receptors.

5. The method of claim 1 wherein said treatment comprises administration of a combination of a first antagonist of the IL-17RA receptor and a second antagonist of the IL-17RE receptor.

6. The method of claim 5 wherein said first antagonist signals through the IL-17RA receptor and said second antagonist signals through the IL-17RE receptor.

7. The method of claim 5 wherein said first antagonist binds to the IL-17RA receptor and said second antagonist binds to the IL-17RE receptor.

8. The method of claim 1 wherein said antagonist is an antibody or an antigen-binding fragment thereof.

9. The method of claim 3 wherein said antagonist is a bispecific or cross-reactive antibody signaling through or binding to both the IL-17RA and IL-17RE receptors, or an antigen-binding fragment thereof.

10. The method of claim 1 further comprising the administration of a further therapeutic agent to treat inflammatory bowel disease.

11. The method of claim 10 wherein said further therapeutic agent is an IFN-γ antagonist.

12. The method of claim 11 wherein said IFN-γ antagonist comprises an anti-IFN-γ antibody, an IFN-γ receptor antibody or a native IFN-γ receptor.

13. The method of claim 10 wherein said further therapeutic agent is a TNF-α antagonist.

14. The method of claim 13 wherein said TNF-α antagonist comprises an anti-TNF-α antibody, a TNF-α receptor antibody or a native TNF-α receptor.

15. A method of reducing gastrointestinal inflammation, comprising administering to a subject in need a bispecific or cross-reactive antibody specifically binding to IL-17C and a further cytokine, or an antigen-binding fragment of said antibody.

16. The method of claim 15 wherein said further cytokine is a proinflammatory cytokine.

17. The method of claim 16 wherein said proinflammatory cytokine is selected from the group consisting of TNFα, IL-1β, IL-22 and IL-17A.

18. The method of claim 17 wherein said proinflammatory cytokine is TNFα or IL-1β.

19. The method of claim 15 wherein said gastrointestinal inflammation is associated with inflammatory bowel disease.

20. The method of claim 19 wherein said subject is a human patient.

21. The method of claim 20 wherein the inflammatory bowel disease is ulcerative colitis.

22. The method of claim 15 wherein said antibody is bispecific.

23. The method of claim 15 wherein said antibody is chimeric, humanized or human.

24. The method of claim 23 wherein said antibody is an antibody fragment.

25. The method of claim 24 wherein said antibody fragment is selected from the group consisting of Fab, Fab', F(ab')₂, and Fv fragments; diabodies; linear antibodies; single-chain antibody molecules; and multispecific antibodies formed from antibody fragments.

26. A method for the treatment of psoriasis, comprising administering to a subject in need a bispecific or cross-reactive antibody specifically binding to IL-17C and a further cytokine, or an antigen-binding fragment of such antibody.

27. The method of claim 16, wherein the further cytokine is a proinflammatory cytokine.

28. The method of claim 27 wherein the proinflammatory cytokine is selected from the group consisting of TNFα, IL-1β, IL-22 and IL-17A.

29. The method of claim 28 wherein the proinflammatory cytokine is TNFα or IL-1β.

30. The method of claim 26, wherein the antibody is bispecific.

31. The method of claim 26, wherein the antibody is chimeric, humanized or human.

32. The method of claim 26, wherein the antibody fragment is selected from the group consisting of Fab, Fab', F(ab')₂, and Fv fragments; diabodies; linear antibodies; single-chain antibody molecules; and multispecific antibodies formed from antibody fragments.

33. The method of claim 26, wherein the subject is a human patient.

34. A pharmaceutical composition comprising an IL-17RA and/or IL-17RE antagonist in admixture with a pharmaceutically acceptable excipient, for the treatment of gastrointestinal inflammation.

35. The pharmaceutical composition of claim 34 wherein the IL-17RA and/or IL-17RE antagonist is an antibody or a fragment thereof.

36. The pharmaceutical composition of claim 35 wherein the antibody is a monoclonal antibody.

37. The pharmaceutical composition of claim 36 wherein the antibody is a chimeric, humanized or human antibody.

38. The pharmaceutical composition of claim 37 wherein the antibody is a bispecific, multispecific or cross-reactive antibody.

39. The use of an IL-17RA and/or IL-17RE antagonist in the preparation of a medicament for the treatment of gastrointestinal inflammation.

40. A kit for treating gastrointestinal inflammation, said kit comprising: (a) a container comprising an IL-17RA and/or IL-17RE antagonist; and (b) a label or instructions for administering said antibody to treat said inflammation.


SEQUENCE LISTING

<110> GENENTECH, INC.

<120> TREATMENT OF GASTROINTESTINAL INFLAMMATION AND PSORIASIS AND ASTHMA

<130> GNE-0364WO

<140>
<141> 2011-10-24

<150> 61/627,493
<151> 2011-10-12

<150> 61/626,838
<151> 2011-10-03

<150> 61/445,780
<151> 2010-10-25

<160> 31

<170> PatentIn version 3.5

<210> 1
<211> 20
<212> DNA
<213> Artificial Sequence

<220>
<221> source
<223> /note="Description of Artificial Sequence: Synthetic
      primer"

<400> 1
gcgcatcctc atggagcaca                                                    20


<210> 2
<211> 20
<212> DNA
<213> Artificial Sequence

<220>
<221> source
<223> /note="Description of Artificial Sequence: Synthetic
      primer"

<400> 2
ggtcagccag gagcttcttg                                                    20


<210> 3
<211> 25
<212> DNA
<213> Artificial Sequence

<220>
<221> source
<223> /note="Description of Artificial Sequence: Synthetic
      probe"

<400> 3

cacaagctga aggcagacaa ggccc                                          25

<210> 4
<211> 20
<212> DNA
<213> Artificial Sequence

<220>
<221> source
<223> /note="Description of Artificial Sequence: Synthetic
      primer"

<400> 4
gcggattctc atggaacaca                                                20

<210> 5
<211> 19
<212> DNA
<213> Artificial Sequence

<220>
<221> source
<223> /note="Description of Artificial Sequence: Synthetic
      primer"

<400> 5
aattccttct gccctgcat                                                 19

<210> 6
<211> 20
<212> DNA
<213> Artificial Sequence

<220>
<221> source
<223> /note="Description of Artificial Sequence: Synthetic
      primer"

<400> 6
acacttttg cgcctcacag                                                 20

<210> 7
<211> 26
<212> DNA
<213> Artificial Sequence

<220>
<221> source
<223> /note="Description of Artificial Sequence: Synthetic
      probe"

<400> 7
tagaggcctc ctacctgcaa gaggac                                         26

<210> 8
<211> 22
<212> DNA
<213> Artificial Sequence

<220>
<221> source
<223> /note="Description of Artificial Sequence: Synthetic
      primer"

<400> 8
ttctgtccaa actgaggcat ca                                                22


<210> 9
<211> 20
<212> DNA
<213> Artificial Sequence

<220>
<221> source
<223> /note="Description of Artificial Sequence: Synthetic
      primer"

<400> 9
agggtcaacc acaaagtggc                                                   20


<210> 10
<211> 24
<212> DNA
<213> Artificial Sequence

<220>
<221> source
<223> /note="Description of Artificial Sequence: Synthetic
      probe"

<400> 10
cacaggcggt ggcgttttac cttc                                              24


<210> 11
<211> 21
<212> DNA
<213> Artificial Sequence

<220>
<221> source
<223> /note="Description of Artificial Sequence: Synthetic
      primer"

<400> 11
gaatttgagt ctgcccagtt c                                                 21


<210> 12
<211> 19
<212> DNA
<213> Artificial Sequence

<220>
<221> source
<223> /note="Description of Artificial Sequence: Synthetic
      primer"

<400> 12

aagacgggca tgttttctg                                                    19

<210> 13
<211> 27
<212> DNA
<213> Artificial Sequence

<220>
<221> source
<223> /note="Description of Artificial Sequence: Synthetic
      probe"

<400> 13
ccaactggta catcagcacc tctcaag                                           27

<210> 14
<211> 19
<212> DNA
<213> Artificial Sequence

<220>
<221> source
<223> /note="Description of Artificial Sequence: Synthetic
      primer"

<400> 14
ttggaggcag acacccacc                                                    19

<210> 15
<211> 20
<212> DNA
<213> Artificial Sequence

<220>
<221> source
<223> /note="Description of Artificial Sequence: Synthetic
      primer"

<400> 15
gatagcggtc ctcatccgtg                                                   20

<210> 16
<211> 26
<212> DNA
<213> Artificial Sequence

<220>
<221> source
<223> /note="Description of Artificial Sequence: Synthetic
      probe"

<400> 16
ccatctcacc ctggagatac cgtgtg                                            26

<210> 17
<211> 20
<212> DNA
<213> Artificial Sequence

<220>
<221> source
<223> /note="Description of Artificial Sequence: Synthetic
      primer"

<400> 17
cctgccccaa tccctttatt                                                    20


<210> 18
<211> 21
<212> DNA
<213> Artificial Sequence

<220>
<221> source
<223> /note="Description of Artificial Sequence: Synthetic
      primer"

<400> 18
ccccaattct ctttttgagc c                                                  21


<210> 19
<211> 25
<212> DNA
<213> Artificial Sequence

<220>
<221> source
<223> /note="Description of Artificial Sequence: Synthetic
      probe"

<400> 19
ccccctcctt cagacaccct caacc                                              25


<210> 20
<211> 19
<212> DNA
<213> Artificial Sequence

<220>
<221> source
<223> /note="Description of Artificial Sequence: Synthetic
      primer"

<400> 20
ctggaagctg acactcacg                                                     19


<210> 21
<211> 20
<212> DNA
<213> Artificial Sequence

<220>
<221> source
<223> /note="Description of Artificial Sequence: Synthetic
      primer"

<400> 21

ggtagcggtt ctcatctgtg 20

<210> 22
<211> 26
<212> DNA
<213> Artificial Sequence

<220>
<221> source
<223> /note="Description of Artificial Sequence: Synthetic
      probe"

<400> 22
ccatctcacc atggagatat cgcatc 26

<210> 23
<211> 20
<212> DNA
<213> Artificial Sequence

<220>
<221> source
<223> /note="Description of Artificial Sequence: Synthetic
      primer"

<400> 23
tggtacatca gcacctcaca 20

<210> 24
<211> 23
<212> DNA
<213> Artificial Sequence

<220>
<221> source
<223> /note="Description of Artificial Sequence: Synthetic
      primer"

<400> 24
ttatgtcctg accactgttg ttt 23

<210> 25
<211> 24
<212> DNA
<213> Artificial Sequence

<220>
<221> source
<223> /note="Description of Artificial Sequence: Synthetic
      probe"

<400> 25
agagcacaag cctgtcttcc tggg 24

<210> 26
<211> 21
<212> DNA
<213> Artificial Sequence

<220>
<221> source
<223> /note="Description of Artificial Sequence: Synthetic
      primer"

<400> 26
gaccaggctg tcgctacatc a                                                21


<210> 27
<211> 21
<212> DNA
<213> Artificial Sequence

<220>
<221> source
<223> /note="Description of Artificial Sequence: Synthetic
      primer"

<400> 27
cccgtagggc gattacagtc a                                                21


<210> 28
<211> 23
<212> DNA
<213> Artificial Sequence

<220>
<221> source
<223> /note="Description of Artificial Sequence: Synthetic
      probe"

<400> 28
tgaacctctg ctccccacgg gag                                              23


<210> 29
<211> 197
<212> PRT
<213> Homo sapiens

<400> 29
Met Thr Leu Leu Pro Gly Leu Leu Phe Leu Thr Trp Leu His Thr Cys
1               5                   10                  15

Leu Ala His His Asp Pro Ser Leu Arg Gly His Pro His Ser His Gly
            20                  25                  30

Thr Pro His Cys Tyr Ser Ala Glu Glu Leu Pro Leu Gly Gln Ala Pro
        35                  40                  45

Pro His Leu Leu Ala Arg Gly Ala Lys Trp Gly Gln Ala Leu Pro Val
    50                  55                  60

Ala Leu Val Ser Ser Leu Glu Ala Ala Ser His Arg Gly Arg His Glu
65                  70                  75                  80

Arg Pro Ser Ala Thr Thr Gln Cys Pro Val Leu Arg Pro Glu Glu Val
                85                  90                  95

Leu Glu Ala Asp Thr His Gln Arg Ser Ile Ser Pro Trp Arg Tyr Arg
            100                 105                 110

Val Asp Thr Asp Glu Asp Arg Tyr Pro Gln Lys Leu Ala Phe Ala Glu
        115                 120                 125

Cys Leu Cys Arg Gly Cys Ile Asp Ala Arg Thr Gly Arg Glu Thr Ala
    130                 135                 140

Ala Leu Asn Ser Val Arg Leu Leu Gln Ser Leu Leu Val Leu Arg Arg
145                 150                 155                 160

Arg Pro Cys Ser Arg Asp Gly Ser Gly Leu Pro Thr Pro Gly Ala Phe
                165                 170                 175

Ala Phe His Thr Glu Phe Ile His Val Pro Val Gly Cys Thr Cys Val
            180                 185                 190

Leu Pro Arg Ser Val
        195

<210> 30
<211> 866
<212> PRT
<213> Homo sapiens

<400> 30
Met Gly Ala Ala Arg Ser Pro Pro Ser Ala Val Pro Gly Pro Leu Leu
1               5                   10                  15

Gly Leu Leu Leu Leu Leu Leu Gly Val Leu Ala Pro Gly Gly Ala Ser
            20                  25                  30

Leu Arg Leu Leu Asp His Arg Ala Leu Val Cys Ser Gln Pro Gly Leu
        35                  40                  45

Asn Cys Thr Val Lys Asn Ser Thr Cys Leu Asp Asp Ser Trp Ile His
    50                  55                  60

Pro Arg Asn Leu Thr Pro Ser Ser Pro Lys Asp Leu Gln Ile Gln Leu
65                  70                  75                  80

His Phe Ala His Thr Gln Gln Gly Asp Leu Phe Pro Val Ala His Ile
                85                  90                  95

Glu Trp Thr Leu Gln Thr Asp Ala Ser Ile Leu Tyr Leu Glu Gly Ala
            100                 105                 110

Glu Leu Ser Val Leu Gln Leu Asn Thr Asn Glu Arg Leu Cys Val Arg
        115                 120                 125

Phe Glu Phe Leu Ser Lys Leu Arg His His His Arg Arg Trp Arg Phe
    130                 135                 140

Thr Phe Ser His Phe Val Val Asp Pro Asp Gln Glu Tyr Glu Val Thr
145                 150                 155                 160

Val His His Leu Pro Lys Pro Ile Pro Asp Gly Asp Pro Asn His Gln
                165                 170                 175

Ser Lys Asn Phe Leu Val Pro Asp Cys Glu His Ala Arg Met Lys Val
            180                 185                 190

Thr Thr Pro Cys Met Ser Ser Gly Ser Leu Trp Asp Pro Asn Ile Thr
        195                 200                 205

Val Glu Thr Leu Glu Ala His Gln Leu Arg Val Ser Phe Thr Leu Trp
    210                 215                 220

Asn Glu Ser Thr His Tyr Gln Ile Leu Leu Thr Ser Phe Pro His Met
225                 230                 235                 240

Glu Asn His Ser Cys Phe Glu His Met His His Ile Pro Ala Pro Arg
                245                 250                 255

Pro Glu Glu Phe His Gln Arg Ser Asn Val Thr Leu Thr Leu Arg Asn
            260                 265                 270

Leu Lys Gly Cys Cys Arg His Gln Val Gln Ile Gln Pro Phe Phe Ser
        275                 280                 285

Ser Cys Leu Asn Asp Cys Leu Arg His Ser Ala Thr Val Ser Cys Pro
    290                 295                 300

Glu Met Pro Asp Thr Pro Glu Pro Ile Pro Asp Tyr Met Pro Leu Trp
305                 310                 315                 320

Val Tyr Trp Phe Ile Thr Gly Ile Ser Ile Leu Leu Val Gly Ser Val
                325                 330                 335

Ile Leu Leu Ile Val Cys Met Thr Trp Arg Leu Ala Gly Pro Gly Ser
            340                 345                 350

Glu Lys Tyr Ser Asp Asp Thr Lys Tyr Thr Asp Gly Leu Pro Ala Ala
        355                 360                 365

Asp Leu Ile Pro Pro Pro Leu Lys Pro Arg Lys Val Trp Ile Ile Tyr
    370                 375                 380

Ser Ala Asp His Pro Leu Tyr Val Asp Val Val Leu Lys Phe Ala Gln
385                 390                 395                 400

Phe Leu Leu Thr Ala Cys Gly Thr Glu Val Ala Leu Asp Leu Leu Glu
                405                 410                 415

Glu Gln Ala Ile Ser Glu Ala Gly Val Met Thr Trp Val Gly Arg Gln
            420                 425                 430

Lys Gln Glu Met Val Glu Ser Asn Ser Lys Ile Ile Val Leu Cys Ser
        435                 440                 445

Arg Gly Thr Arg Ala Lys Trp Gln Ala Leu Leu Gly Arg Gly Ala Pro
    450                 455                 460

Val Arg Leu Arg Cys Asp His Gly Lys Pro Val Gly Asp Leu Phe Thr
465                 470                 475                 480

Ala Ala Met Asn Met Ile Leu Pro Asp Phe Lys Arg Pro Ala Cys Phe
                485                 490                 495

Gly Thr Tyr Val Val Cys Tyr Phe Ser Glu Val Ser Cys Asp Gly Asp
            500                 505                 510

Val Pro Asp Leu Phe Gly Ala Ala Pro Arg Tyr Pro Leu Met Asp Arg
        515                 520                 525

Phe Glu Glu Val Tyr Phe Arg Ile Gln Asp Leu Glu Met Phe Gln Pro
    530                 535                 540

Gly Arg Met His Arg Val Gly Glu Leu Ser Gly Asp Asn Tyr Leu Arg
545                 550                 555                 560

Ser Pro Gly Gly Arg Gln Leu Arg Ala Ala Leu Asp Arg Phe Arg Asp
                565                 570                 575

Trp Gln Val Arg Cys Pro Asp Trp Phe Glu Cys Glu Asn Leu Tyr Ser
            580                 585                 590

Ala Asp Asp Gln Asp Ala Pro Ser Leu Asp Glu Glu Val Phe Glu Glu
        595                 600                 605

Pro Leu Leu Pro Pro Gly Thr Gly Ile Val Lys Arg Ala Pro Leu Val
    610                 615                 620

Arg Glu Pro Gly Ser Gln Ala Cys Leu Ala Ile Asp Pro Leu Val Gly
625                 630                 635                 640

Glu Glu Gly Gly Ala Ala Val Ala Lys Leu Glu Pro His Leu Gln Pro
                645                 650                 655

Arg Gly Gln Pro Ala Pro Gln Pro Leu His Thr Leu Val Leu Ala Ala
            660                 665                 670

Glu Glu Gly Ala Leu Val Ala Ala Val Glu Pro Gly Pro Leu Ala Asp
        675                 680                 685

Gly Ala Ala Val Arg Leu Ala Leu Ala Gly Glu Gly Glu Ala Cys Pro
    690                 695                 700

Leu Leu Gly Ser Pro Gly Ala Gly Arg Asn Ser Val Leu Phe Leu Pro
705                 710                 715                 720

Val Asp Pro Glu Asp Ser Pro Leu Gly Ser Ser Thr Pro Met Ala Ser
                725                 730                 735

Pro Asp Leu Leu Pro Glu Asp Val Arg Glu His Leu Glu Gly Leu Met
            740                 745                 750

Leu Ser Leu Phe Glu Gln Ser Leu Ser Cys Gln Ala Gln Gly Gly Cys
        755                 760                 765

Ser Arg Pro Ala Met Val Leu Thr Asp Pro His Thr Pro Tyr Glu Glu
    770                 775                 780

Glu Gln Arg Gln Ser Val Gln Ser Asp Gln Gly Tyr Ile Ser Arg Ser
785                 790                 795                 800

Ser Pro Gln Pro Pro Glu Gly Leu Thr Glu Met Glu Glu Glu Glu Glu
                805                 810                 815

Glu Glu Gln Asp Pro Gly Lys Pro Ala Leu Pro Leu Ser Pro Glu Asp
            820                 825                 830

Leu Glu Ser Leu Arg Ser Leu Gln Arg Gln Leu Leu Phe Arg Gln Leu
        835                 840                 845

Gln Lys Asn Ser Gly Trp Asp Thr Met Gly Ser Glu Ser Glu Gly Pro

850                 855                 860

Ser Ala
865

<210> 31
<211> 667
<212> PRT
<213> Homo sapiens

<400> 31
Met Gly Ser Ser Arg Leu Ala Ala Leu Leu Leu Pro Leu Leu Leu Ile
1               5                   10                  15

Val Ile Asp Leu Ser Asp Ser Ala Gly Ile Gly Phe Arg His Leu Pro
            20                  25                  30

His Trp Asn Thr Arg Cys Pro Leu Ala Ser His Thr Asp Asp Ser Phe
        35                  40                  45

Thr Gly Ser Ser Ala Tyr Ile Pro Cys Arg Thr Trp Trp Ala Leu Phe
    50                  55                  60

Ser Thr Lys Pro Trp Cys Val Arg Val Trp His Cys Ser Arg Cys Leu
65                  70                  75                  80

Cys Gln His Leu Leu Ser Gly Gly Ser Gly Leu Gln Arg Gly Leu Phe
                85                  90                  95

His Leu Leu Val Gln Lys Ser Lys Lys Ser Ser Thr Phe Lys Phe Tyr
            100                 105                 110

Arg Arg His Lys Met Pro Ala Pro Ala Gln Arg Lys Leu Leu Pro Arg
        115                 120                 125

Arg His Leu Ser Glu Lys Ser His His Ile Ser Ile Pro Ser Pro Asp
    130                 135                 140

Ile Ser His Lys Gly Leu Arg Ser Lys Arg Thr Gln Pro Ser Asp Pro
145                 150                 155                 160

Glu Thr Trp Glu Ser Leu Pro Arg Leu Asp Ser Gln Arg His Gly Gly
                165                 170                 175

Pro Glu Phe Ser Phe Asp Leu Leu Pro Glu Ala Arg Ala Ile Arg Val
            180                 185                 190

Thr Ile Ser Ser Gly Pro Glu Val Ser Val Arg Leu Cys His Gln Trp
        195                 200                 205

Ala Leu Glu Cys Glu Glu Leu Ser Ser Pro Tyr Asp Val Gln Lys Ile
    210                 215                 220

Val Ser Gly Gly His Thr Val Glu Leu Pro Tyr Glu Phe Leu Leu Pro
225                 230                 235                 240

Cys Leu Cys Ile Glu Ala Ser Tyr Leu Gln Glu Asp Thr Val Arg Arg
                245                 250                 255

Lys Lys Cys Pro Phe Gln Ser Trp Pro Glu Ala Tyr Gly Ser Asp Phe
            260                 265                 270

Trp Lys Ser Val His Phe Thr Asp Tyr Ser Gln His Thr Gln Met Val
        275                 280                 285

Met Ala Leu Thr Leu Arg Cys Pro Leu Lys Leu Glu Ala Ala Leu Cys
    290                 295                 300

Gln Arg His Asp Trp His Thr Leu Cys Lys Asp Leu Pro Asn Ala Thr
305                 310                 315                 320

Ala Arg Glu Ser Asp Gly Trp Tyr Val Leu Glu Lys Val Asp Leu His
                325                 330                 335

Pro Gln Leu Cys Phe Lys Phe Ser Phe Gly Asn Ser Ser His Val Glu
            340                 345                 350

Cys Pro His Gln Thr Gly Ser Leu Thr Ser Trp Asn Val Ser Met Asp
        355                 360                 365

Thr Gln Ala Gln Gln Leu Ile Leu His Phe Ser Ser Arg Met His Ala
    370                 375                 380

Thr Phe Ser Ala Ala Trp Ser Leu Pro Gly Leu Gly Gln Asp Thr Leu
385                 390                 395                 400

Val Pro Pro Val Tyr Thr Val Ser Gln Ala Arg Gly Ser Ser Pro Val
                405                 410                 415

Ser Leu Asp Leu Ile Ile Pro Phe Leu Arg Pro Gly Cys Cys Val Leu
            420                 425                 430

Val Trp Arg Ser Asp Val Gln Phe Ala Trp Lys His Leu Leu Cys Pro
        435                 440                 445

Asp Val Ser Tyr Arg His Leu Gly Leu Leu Ile Leu Ala Leu Leu Ala

    450                 455                 460

Leu Leu Thr Leu Leu Gly Val Val Leu Ala Leu Thr Cys Arg Arg Pro
465                 470                 475                 480

Gln Ser Gly Pro Gly Pro Ala Arg Pro Val Leu Leu Leu His Ala Ala
                485                 490                 495

Asp Ser Glu Ala Gln Arg Arg Leu Val Gly Ala Leu Ala Glu Leu Leu
            500                 505                 510

Arg Ala Ala Leu Gly Gly Gly Arg Asp Val Ile Val Asp Leu Trp Glu
        515                 520                 525

Gly Arg His Val Ala Arg Val Gly Pro Leu Pro Trp Leu Trp Ala Ala
    530                 535                 540

Arg Thr Arg Val Ala Arg Glu Gln Gly Thr Val Leu Leu Leu Trp Ser
545                 550                 555                 560

Gly Ala Asp Leu Arg Pro Val Ser Gly Pro Asp Pro Arg Ala Ala Pro
                565                 570                 575

Leu Leu Ala Leu Leu His Ala Ala Pro Arg Pro Leu Leu Leu Leu Ala
            580                 585                 590

Tyr Phe Ser Arg Leu Cys Ala Lys Gly Asp Ile Pro Pro Pro Leu Arg
        595                 600                 605

Ala Leu Pro Arg Tyr Arg Leu Leu Arg Asp Leu Pro Arg Leu Leu Arg
    610                 615                 620

Ala Leu Asp Ala Arg Pro Phe Ala Glu Ala Thr Ser Trp Gly Arg Leu
625                 630                 635                 640

Gly Ala Arg Gln Arg Arg Gln Ser Arg Leu Glu Leu Cys Ser Arg Leu
                645                 650                 655

Glu Arg Glu Ala Ala Arg Leu Ala Asp Leu Gly
            660                 665